(19) **Europäisches Patentamt / European Patent Office / Office européen des brevets**

(11) **EP 3 775 884 B1**

(12) **EUROPEAN PATENT SPECIFICATION**

(45) Date of publication and mention of the grant of the patent:
**08.05.2024 Bulletin 2024/19**

(21) Application number: **19715084.0**

(22) Date of filing: **01.04.2019**

(51) International Patent Classification (IPC):
**G01N 33/50** (2006.01)    **G01N 33/68** (2006.01)

(52) Cooperative Patent Classification (CPC):
**G01N 33/6896; G01N 33/5091**

(86) International application number:
**PCT/EP2019/058206**

(87) International publication number:
**WO 2019/192969 (10.10.2019 Gazette 2019/41)**

(54) **METHOD FOR THE DIAGNOSIS OF AMYLOID-ASSOCIATED DISEASES**

VERFAHREN ZUR DIAGNOSE VON AMYLOIDEN KRANKHEITEN

MÉTHODE POUR LE DIAGNOSE DE MALADIES LIÉES AUX AMYLOÏDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **03.04.2018 GB 201805466**

(43) Date of publication of application:
**17.02.2021 Bulletin 2021/07**

(73) Proprietor: **Amyloidia Sweden AB**
**172 37 Sundbyberg (SE)**

(72) Inventors:
- **VUKOJEVIC, Vladana**
  **17237 Sundbyberg (SE)**
- **GRÄSLUND, Astrid**
  **19144 Sollentuna (SE)**
- **JARVET, Jüri**
  **17445 Sundbyberg (SE)**
- **TIIMAN, Ann**
  **17162 Solna (SE)**
- **RIGLER, Rudolf**
  **18260 Djurshol (SE)**
- **TERENIUS, Lars**
  **75312 Uppsala (SE)**

- **WÄRMLÄNDER, Sebastian**
  **11847 Stockholm (SE)**

(74) Representative: **Weickmann & Weickmann PartmbB**
**Postfach 860 820**
**81635 München (DE)**

(56) References cited:
**WO-A2-2008/030973    US-A1- 2016 077 110**

- **ANN TIIMAN ET AL: "Heterogeneity and Turnover of Intermediates during Amyloid-[beta] (A[beta]) Peptide Aggregation Studied by Fluorescence Correlation Spectroscopy", BIOCHEMISTRY, vol. 54, no. 49, 3 December 2015 (2015-12-03), pages 7203-7211, XP055584465, ISSN: 0006-2960, DOI: 10.1021/acs.biochem.5b00976 cited in the application**
- **PITSCHKE M ET AL: "DETECTION OF SINGLE AMYLOID BETA-PROTEIN AGGREGATES IN THE CEREBROSPINAL FLUID OF ALZHEIMER'S PATIENTS BY FLUORESCENCE CORRELATION SPECTROSCOPY", NATURE MEDICINE, NATURE PUB. CO, NEW YORK, vol. 4, no. 7, 1 July 1998 (1998-07-01), pages 832-834, XP001073409, ISSN: 1078-8956, DOI: 10.1038/NM0798-832**

EP 3 775 884 B1

**Description**

The invention relates generally to methods for the diagnosis and/or prognosis of amyloid-associated diseases, and methods for identifying an agent for the treatment of amyloid-associated diseases, as well as related uses.

Amyloid-associated diseases are characterised by the presence and accumulation of amyloid fibrils and aggregates in a patient. Well known examples of amyloid-associated diseases are Alzheimer's disease, Huntington's disease and Parkinson's disease. These diseases often lead to a deterioration of a patient's health and mental well-being, sometimes over a long period of time, and often result in death. The pathogenesis and symptoms of the amyloid-associated diseases are often very traumatic for the patient, and his/her family. Alzheimer's disease is the most common cause of dementia worldwide (Prince et al., World Alzheimer Report 2016. London, UK: 2016 and Alzheimer's Disease Facts and Figures. Alzheimer's Association. 2017).

Key to treating and managing the symptoms of amyloid-associated diseases is an early diagnosis, as well as a clear prognosis of disease progression. However, since amyloid-associated diseases often develop slowly and there is a lack of known early biomarkers of the disease, a patient usually has a well-established disease before receiving any diagnosis and/or prognosis. In some cases, methods to identify the disease using biomarkers can only be conducted post-mortem, which provide no benefit to the patient. This drastically delays the patient being able to receive timely and appropriate treatment, which is detrimental to health. Accordingly, there is a need for the development of methods for the early diagnosis and prognosis of amyloid-associated diseases.

US 6,498,017 discloses a method for detection of diseases associated with protein depositions. An important characteristic of the method is the use of probes which typically are of the same type of compound as the target to be detected. The probes are suitably obtained by subjecting pathological protein deposits by physical and/or chemical means (e.g. ultrasonication, detergents). The probes in conjunction with disease associated deposits serving as nuclei provides for a polymerisation process thereby amplifying the measured signal.

WO 2016/04090 A1 presents a methodology for determining the presence of a misfolded Aβ protein in a sample. Indispensable to the method is the integration of a step amplifying the Aβ protein prior to analysis.

Tiiman et al. (Biochemistry 2015, 54, 7203-7211) relates to the kinetics of Aβ aggregation and more specifically the time dependent growth of structured Aβ aggregates. The kinetic studies are performed on model solutions comprising recombinant lyophilized Aβ42.

Guan et al. (ACS Chem. Neurosci. 2015, 6, 1503-1508) and WO 2017/004560 disclose a novel fluorescent market (ARCAM-1) having valuable spectroscopic characteristics especially when associating to protein aggregates of Aβ. When ARCAM-1 associates to Aβ aggregates a significant increase in fluorescence emission is achieved compared to un-bound ARCAM-1. The spectroscopic properties of ARCAM-1 are evaluated using a model system incorporating synthesized Aβ42.

Wennmalm et al. (Anal. Chem. 2015, 87, 11700-11705) presents a system for detection of amyloid beat oligomers with high sensitivity by the provision of FRET and FCS (Fluorescence Correlation Spectroscopy). Synthetic fluorescent labelled Aβ40 and Aβ42 are used as model substances.

Against this background, the inventors have developed a new method allowing for the early diagnosis and prognosis of amyloid-associated diseases.

The inventors' development involves the determination of the presence and/or amount and/or concentration and/or size of individual amyloid aggregates in the patient, and is based on the surprising finding that individual amyloid aggregates that occur in patients with amyloid-associated diseases can be identified in a patient's bodily fluid such as blood or cerebrospinal fluid (CSF). The inventors have found that these individual amyloid aggregates can be identified well before the disease is well-established and detectable using other known methods, and that the detection of the individual amyloid aggregates provides a highly effective diagnosis and/or prognosis of the disease. A further advantage of the inventors' technology is that individual amyloid aggregates can be detected at low levels; since low levels of individual amyloid aggregates are sometimes present at an early stage of an amyloid-associated disease, this aids in determining an early diagnosis and/or prognosis.

The invention is defined in the appended claims.

Accordingly, a first aspect of the invention provides a method for the diagnosis and/or prognosis of an amyloid-associated disease in a subject, the amyloid-associated disease being associated with individual amyloid aggregates, wherein the method comprises the steps of:

- adding a detection agent to a sample of bodily fluid from the subject, the detection agent binding to and/or associating with and/or reacting with individual amyloid aggregates; and
- determining the presence and/or amount and/or concentration and/or size of individual amyloid aggregates in the sample

wherein determining the presence and/or amount and/or concentration and/or size of individual amyloid aggregates in the sample comprises an analysis based on time-resolved detection comprising fluorescence correlation spectroscopy (FCS) and fluorescence intensity fluctuation analysis (FIFA), and wherein the method does not comprise amplification of individual amyloid aggregates.

Preferably, the method further comprises the step of providing a diagnosis and/or prognosis of the amyloid-associated disease on the basis of the determination. Most preferably, the step of providing a diagnosis and/or prognosis of the amyloid-associated disease on the basis of the determination follows the step of determining the presence and/or amount and/or concentration and/or size of individual amyloid aggregates in the sample.

Accordingly, the invention provides a highly effective early and non-invasive method for the diagnosis and prognosis of amyloid-associated diseases in living patients, which is also applicable to following the effects of the treatment of amyloid-associated diseases. The methods are further useful for identifying and developing agents to treat amyloid-related diseases. Particularly useful for these applications is that the methods described herein can be used to routinely identify and characterise the levels of amyloid aggregates in the same patient repeatedly over time. The invention also provides kits corresponding to the methods described herein.

An additional advantage of the method of the invention is that as it is conducted on bodily fluid it is non-, or minimally, invasive, so less unpleasant for the patient. Additionally, as the method is able to detect early signs of the disease, it can be conducted on living patients and not just post-mortem. This also allows the method to be repeated multiple times, in order to monitor the progress of the disease in a living patient.

The term "amyloid-associated disease" would be known to one skilled in medicine. An amyloid-associated disease is sometimes referred to as amyloidosis, and encompasses diseases in which amyloids accumulate in a subject. Amyloids are accumulations of a number of copies of particular peptides or particular proteins that stick together to form aggregates, which in turn combine to form fibrils. Hence, fibrils may be larger structures than aggregates. In the aggregate and/or fibril form, the peptides or proteins no longer have their physiological function. When the amyloid aggregates and fibrils accumulate in the tissues of the subject, they often disrupt the function of those tissues which can lead to the pathology of the amyloid-associated disease.

By "determining the presence and/or amount and/or concentration and/or size of individual amyloid aggregates", we include that individual aggregates are determined. The determination does not involve determining the total amount or concentration of aggregates, but instead the presence and/or amount and/or concentration and/or size of individual amyloid aggregates within the total present. Accordingly, the claimed method differs from current methods, if and where available, in which only the total amount and/or large groups of amyloid fibrils are detected. As discussed herein, the "individual amyloid aggregates" are early biomarkers for amyloid-associated diseases, which allows for an early diagnosis and/or prognosis.

By "individual amyloid aggregates" we include accumulations of peptides or proteins, which are, or form, structures associated with the amyloid-associated disease. The term "individual amyloid aggregates" can be referred to as structured amyloid aggregates, which include amyloid aggregates that have a defined secondary structure, such as amyloid aggregates that comprise β-pleated sheets. The term "amyloid aggregate" is used to refer to any peptide or protein that can form structures associated with an amyloid-associated disease. Whilst that term is sometimes used in the art to refer to amyloid β peptide (which form structures associated with Alzheimer's disease), for the purposes of the present application, it is not limited to that peptide.

A "peptide" is generally considered to be a molecule comprising two or more amino acids, but not more than about 50 amino acids; for example: about three to about 50 amino acids; about four to about 50 amino acids; about five to about 50 amino acids; about six to about 50 amino acids; about seven to about 50 amino acids; about eight to about 50 amino acids; about nine to about 50 amino acids; about ten to about 50 amino acids; about 20 to about 50 amino acids; about 30 to about 50 amino acids; or about 40 to about 50 amino acids.

A "protein" is generally considered to be a molecule comprising about 50 or more amino acids; for example: about 60 or more; about 70 or more; about 80 or more; about 90 or more; about 100 or more; about 150 or more; about 200 or more; about 250 or more; about 300 or more; about 350 or more; about 400 or more; about 450 or more; or about 500 or more amino acids.

In one aspect, the individual amyloid aggregates do not comprise amorphous amyloid aggregates. By "amorphous amyloid aggregates", we include amyloid aggregates that do not have a well-defined secondary structure, such as amyloid aggregates that do not have a β-pleated sheet structure.

The invention generally relates to the detection of individual amyloid aggregates, and the characterisation of the individual amyloid aggregates. That detection and characterisation is described as "determining the presence and/or amount and/or concentration and/or size of individual amyloid aggregates in the sample", and by that we include "identifying and/or calculating and/or detecting" the presence and/or amount and/or concentration and/or size of individual amyloid aggregates in the sample.

By "determining the presence of individual amyloid aggregates in the sample", we include determining whether individual amyloid aggregates are present in the sample or not. As discussed further below, where the sample is a bodily fluid obtained from a patient and if individual amyloid aggregates are present in the bodily fluid of the patient, then that patient is diagnosed with an amyloid-associated disease.

By "determining the <u>amount</u> of individual amyloid aggregates in the sample", we include determining the total number of the individual amyloid aggregates in the sample. As discussed further below, where the sample is a bodily fluid obtained from a patient, the higher the amount of individual amyloid aggregates in the bodily fluid of the patient, the worse the diagnosis and/or prognosis of the amyloid-associated disease.

By "determining the <u>size</u> of individual amyloid aggregates in the sample", we include determining the number of peptides in the individual amyloid aggregates, and/or determining the dimensions of the individual amyloid aggregates in the sample, and/or determining the measurements of the individual amyloid aggregates in the sample; and/or determining the molecular weight of the individual amyloid aggregates in the sample. As discussed further below, where the sample is a bodily fluid obtained from a patient, the larger the size of individual amyloid aggregates in the bodily fluid of the patient, the worse the diagnosis and/or prognosis of the amyloid-associated disease.

By "determining the <u>concentration</u> of individual amyloid aggregates in the sample", we include identifying the relative amount of the individual amyloid aggregates in the sample. As discussed further below, where the sample is a bodily obtained fluid from a patient, the higher the concentration of individual amyloid aggregates in the bodily fluid of the patient, the worse the diagnosis and/or prognosis of the amyloid-associated disease.

In a first aspect, the method comprises the steps in the order of:

- providing a sample of bodily fluid from the subject;
- determining the presence and/or amount and/or concentration and/or size of individual amyloid aggregates in the sample; and,
- providing a diagnosis and/or prognosis of the amyloid-associated disease on the basis of the determination.

An additional aspect relates to a method for the diagnosis and/or prognosis of an amyloid-associated disease in a subject, wherein the method comprises the steps of:

- providing a sample selected from the group consisting of blood plasma and/or blood serum from the subject; and
- determining the presence and/or amount and/or concentration and/or size of individual amyloid aggregates in the sample by an analysis based on fluorescence spectroscopy, suitably fluorescence correlation spectroscopy.

The terms "diagnosis" and "prognosis" would be known to one skilled in medicine.

By "diagnosis" we include that the method can be used to identify whether the subject has an amyloid-associated disease. By "prognosis" we include that the method can be used to identify how quickly the amyloid-associated disease is likely to progress in the subject, and/or how severe the symptoms and/or signs of the amyloid-associated disease are likely to become in the subject, and/or if the subject is likely to die from the amyloid-associated disease, and/or how soon the subject is likely to die from the amyloid-associated disease, and/or an assessment of the effectiveness of a treatment for the amyloid-associated disease that has already been administered to, or practised on, the subject, and/or the likely effectiveness of a treatment for the amyloid-associated disease that might be administered to, or practised on, the subject in future. To put another way, by prognosis we include following the effectiveness of a treatment for an amyloid-associated disease that has previously been administered to, or practised on, the subject.

Treatments for amyloid-associated diseases, if and where available, would be known to one skilled in medicine.

By "treatment", we include a treatment that is intended to cure the amyloid-associated disease, and/or a treatment that is intended to reduce the number and/or severity of the symptoms and/or signs of the amyloid-associated disease, and/or a palliative treatment, and/or an experimental treatment, and/or a physical therapy. Specific treatments for amyloid-associated diseases are discussed herein.

In one embodiment, the bodily fluid is one or more from the group consisting of: blood and/or cerebrospinal fluid (CSF) and/or saliva and/or urine and/or synovial fluid and/or faeces. In a preferable embodiment, the bodily fluid comprises blood. In an alternative, the bodily fluid comprises saliva. In a further alternative, the bodily fluid comprises urine. In a further preferable embodiment the bodily fluid is selected from whole blood and/or blood plasma and/or blood serum. In a still further more preferred embodiment the bodily fluid is selected from blood serum. In an alternative preferred embodiment bodily fluid comprises blood cells, specifically erythrocytes, leukocytes and thrombocytes. According to a further embodiment the blood cells may be lysated.

A particularly surprising development of the current invention is that it is possible to diagnose, or reach a prognosis for, an amyloid-associated disease using blood, urine, synovial fluid, faeces and/or saliva bodily fluid samples. Previously, studies have shown that using known methods it was not possible to diagnose the amyloid-associated disease Alzheimer's disease using a blood sample - in particular, as described herein (Oh et al., Neuromolecular medicine. 2008;10(3):195-207 and Toledo et al., Alzheimer's research & therapy. 2013;5(2):8). However, the problems identified in those previous methods are overcome in the present invention by detecting individual amyloid aggregates, as they have now been shown to be effective early biomarkers for amyloid-associated diseases. For urine, saliva, synovial fluid, faeces and CSF samples, it had not previously been suggested that detecting individual amyloid aggregates in those bodily fluids could be used in the diagnosis or prognosis of amyloid-associated diseases, such as Alzheimer's disease.

A particular advantage of the bodily fluids of urine, saliva, faeces and blood is that they can be provided from the subject using minimally invasive methods, with the provision of the urine, faeces and saliva samples not requiring the assistance of a medical professional.

It would be known to one skilled in medicine how a bodily fluid sample can be provided, for the purposes of the present invention. For example, a blood bodily fluid sample could be obtained by a medical professional, by removing blood from a vein using a needle.

In one embodiment, the blood sample is one or more from the group consisting of: whole blood; blood serum; blood plasma; blood cells: erythrocytes, leukocytes and/or thrombocytes; blood cell lysates.

Blood collected from a subject comprises inter alia blood cells suspended in blood plasma. The major types of blood cells are erythrocytes (red blood cells), leukocytes (white blood cells) and thrombocytes (platelets). According to an embodiment it may be expedient to separate the blood cells from blood and thereby obtain the blood plasma. Furthermore, the clotting factors may be removed from the blood plasma thereby receiving the blood serum. Whole blood can be fractionated by centrifugation. By centrifugation the whole blood is fractioned into blood plasma and erythrocytes and in between these two fractions a thin layer of leukocytes and platelets. While whole blood can be used directly as the sample to be analysed it might be preferable in certain cases to remove the blood cells and used the blood plasma as the sample for the analysis. Further, it may also be advisable to remove any clotting factor from the blood plasma and use the blood serum (as the sample) for analysis.

By "cell lysates", we include erythrocyte, leukocytes and/or thrombocyte (*i.e.* platelet) cell lysates.

Particular components of blood (such as erythrocytes, leukocytes and/or thrombocytes) can be isolated using methods known in art, such as by blood fractionation which involves centrifuging the whole blood sample.

In some circumstances, FAD enzymes and lipopigments and NADH enzymes and lipopigments may lead to a reduction in the sensitivity of the method, as they autofluoresce. Accordingly, there might be some advantages in removing these molecules from the bodily fluid sample before using the method.

In one embodiment, the bodily fluid sample does not comprise one or more from the group consisting of: flavin adenine dinucleotide (FAD) enzymes and/or FAD lipopigments; and/or pyridinic (NADH) enzymes and/or NADH lipopigments.

In a particular embodiment, the blood sample does not comprise one or more from the group consisting of: flavin adenine dinucleotide (FAD) enzymes and/or FAD lipopigments; and/or pyridinic (NADH) enzymes and/or NADH lipopigments.

In a particular aspect, the CSF sample does not comprise one or more from the group comprising or consisting of: flavin adenine dinucleotide (FAD) enzymes and/or FAD lipopigments; and/or pyridinic (NADH) enzymes and/or NADH lipopigments.

How to remove flavin adenine dinucleotide (FAD) enzymes, FAD lipopigments, pyridinic (NADH) enzymes, and/or NADH lipopigments from the sample would be known to one skilled in chemistry. For example, FAD is completely hydrolyzed to FMN in 5% trichloroacetic within 20 hours at 37"C, as described in Burch HB, Bessey OA, Lowry OH. *Fluorometric Measurements of Riboflavin and its Natural Derivatives in Small Quantities of Blood Serum and Cells. J Biol Chem.* 1948 175(1):457-470.

In one embodiment, the individual amyloid aggregate is selected from the group comprising: an amyloid oligomer; an amyloid nano-aggregate; and/or an amyloid senile aggregate; and/or an amyloid fibril; and/or an amyloid protofibril, wherein the senile amyloid aggregate preferably comprises more than 1,000,000 proteins or peptides. Preferably, the individual amyloid aggregate is an amyloid nano-aggregate. These amyloid aggregates are formed by the cross-reacting of peptides and/or proteins susceptible to forming amyloid aggregates in the subject.

The amyloid nano-aggregate can be referred to as an amyloid nano-plaque or a structured amyloid nano-plaque, such as those that comprise a β-pleated sheet secondary structure. The term "amyloid nano-aggregate" describes that comprise higher numbers of peptides than dimers and trimers (and which can be described as structured supramolecular assemblies), but which have not yet formed amyloid fibrils or senile amyloid aggregates (which can be known as senile plaques). These distinctions would be understood by one skilled in biology or chemistry.

Amyloid oligomers and amyloid protofibrils are soluble peptide assemblies observed during the growth of amyloid fibrils. They contain a significant amount of β-sheet structure so are able to bind ThT, so can give rise to ThT fluorescence. Whilst amyloid oligomers and amyloid protofibrils comprise structurally heterogeneous supramolecular assemblies, it is typically regarded that oligomers are smaller and precede protofibril formation. Additionally, oligomers are regarded to assume more often globular rather than elongated and ring-like shapes that is characteristic of protofibrils. The degree of structural order in protofibrils is intermediate between that found in oligomers and in fibrils. The molecular weight of oligomers and protofibrils is in the range from 40-3000 kDa (Nichols et al. Biochemistry. 2015 54 (13): 2193-2204; Byron & Vestergaard Curr. Opin. Struct. Biol. 2015 35: 76-86.).

Amyloid protofibrils are elongated, single-chain like supramolecular assemblies of elongated or ring-like shape that grow and consolidate into amyloid fibrils. Protofibrils can have diameters ranging from 2-10 nm and are about 100-200 nm long. Amyloid fibrils are elongated, multiple-chain like assemblies of molecules with high length-to-diameter ratio, and a diameter ranging from 10-100 nm. Fibrils give rise to senile plaques, which are heterogeneous structures consisting of intricately interwoven mature fibrils (Nichols MR, Colvin BA, Hood EA, Paranjape GS, Osborn DC, Terrill-Usery SE. Biophysical comparison of soluble amyloid-β(1-42) protofibrils, oligomers, and protofilaments. Biochemistry. 2015 54 (13): 2193-2204. doi: 10.1021/bi500957g; Byron O, Vestergaard B. Protein-protein interactions: a supra-structural phenomenon demanding trans-disciplinary biophysical approaches. Curr. Opin. Struct. Biol. 2015 35: 76-86; Relini A. Amyloid like protofibrils with different physical properties. Bio-nanoimaging: Protein Misfolding and Aggregation, Vladimir Uversky and Yuri Lyubchenko Ed. 2013 Technology & Engineering.)

In one embodiment, the individual amyloid aggregates comprise one or more beta (β)-sheets (such as a β-pleated sheet). As would be known to one skilled in biology or chemistry, a beta (β)-sheet is a secondary protein structure, which is generally a twisted stretch of amino acids. The association of two or more beta (β)-sheets is often a mediator of peptide accumulation, such as to form individual amyloid aggregates relevant to amyloid-associated diseases.

In one embodiment, the individual amyloid aggregates can be bound by 2-(4-(Dimethylamino)phenyl)-3,6-dimethylbenzo[d]thiazol-3-ium chloride (Thioflavin T (ThT)) (Groenning, M. (2010) Binding mode of Thioflavin T and other molecular probes in the context of amyloid fibrils-current status. Journal of Chemical Biology 3, 1-18). ThT is a molecule that binds beta (β)-sheet structures, and is available from Sigma-Aldrich.

In one embodiment, the individual amyloid aggregate comprises about 20 or more peptides or proteins; for example: about 30 or more; about 40 or more; about 50 or more; about 60 or more; about 70 or more; about 80 or more; about 90 or more; about 100 or more, preferably about 40 or more peptides or proteins. In a further aspect, the individual amyloid aggregate comprises about 200 or more; about 300 or more; about 400 or more; about 500 or more; about 600 or more; about 700 or more; about 800 or more; about 900 or more; about 1,000 or more; about 2,000 or more; about 3,000 or more; about 4,000 or more; about 5,000 or more; about 6,000 or more; about 7,000 or more; about 8,000 or more; about 9,000 or more; about 10,000 or more; about 20,000 or more; about 30,000 or more; about 40,000 or more; about 50,000 or more; about 60,000 or more; about 70,000 or more; about 80,000 or more; about 90,000 or more; about 100,000 or more; about 200,000 or more; about 300,000 or more; about 400,000 or more; about 500,000 or more; or about 1,000,000 or more peptides or proteins.

More preferably the amyloid nano-aggregate can comprise about 20 proteins or peptides to 100,000 proteins or peptides, such as: about 30 proteins or peptides to 100,000 proteins or peptides; about 40 proteins or peptides to 100,000 proteins or peptides; about 50 proteins or peptides to 100,000 proteins or peptides; about 60 proteins or peptides to 100,000 proteins or peptides; about 70 proteins or peptides to 100,000 proteins or peptides; about 80 proteins or peptides to 100,000 proteins or peptides; about 90 proteins or peptides to 100,000 proteins or peptides; about 100 proteins or peptides to 100,000 proteins or peptides; about 200 proteins or peptides to 100,000 proteins or peptides; about 300 proteins or peptides to 100,000 proteins or peptides; about 400 proteins or peptides to 100,000 proteins or peptides; about 500 proteins or peptides to 100,000 proteins or peptides; about 600 proteins or peptides to 100,000 proteins or peptides; about 700 proteins or peptides to 100,000 proteins or peptides; about 800 proteins or peptides to 100,000 proteins or peptides; about 900 proteins or peptides to 100,000 proteins or peptides; about 1,000 proteins or peptides to 100,000 proteins or peptides; about 2,000 proteins or peptides to 100,000 proteins or peptides; about 3,000 proteins or peptides to 100,000 proteins or peptides; about 4,000 proteins or peptides to 100,000 proteins or peptides; about 5,000 proteins or peptides to 100,000 proteins or peptides; about 6,000 proteins or peptides to 100,000 proteins or peptides; about 7,000 proteins or peptides to 100,000 proteins or peptides; about 8,000 proteins or peptides to 100,000 proteins or peptides; about 9,000 proteins or peptides to 100,000 proteins or peptides; about 10,000 proteins or peptides to 100,000 proteins or peptides; about 40 proteins or peptides to about 1,000 proteins or peptides; about 1,000 proteins or peptides to about 2,000 proteins or peptides; about 1,000 proteins or peptides to about 3,000 proteins or peptides; about 1,000 proteins or peptides to about 4,000 proteins or peptides; about 1,000 proteins or peptides to about 5,000 proteins or peptides; about 1,000 proteins or peptides to about 6,000 proteins or peptides; about 1,000 proteins or peptides to about 7,000 proteins or peptides; about 1,000 proteins or peptides to about 8,000 proteins or peptides; about 1,000 proteins or peptides to about 8,000 proteins or peptides; or about 1,000 proteins or peptides to about 10,000 proteins or peptides.

As will be appreciated, amyloid fibrils can comprise more than 100,000 peptides or proteins to 1,000,000 peptides or proteins, such as: about 200,000 peptides or proteins to 1,000,000 peptides or proteins; about 300,000 peptides or proteins to 1,000,000 peptides or proteins; about 400,000 peptides or proteins to 1,000,000 peptides or proteins; or about 500,000 peptides or proteins to 1,000,000 peptides or proteins.

As will be appreciated, senile amyloid aggregates can comprise more than 1,000,000 peptides or proteins.

In one embodiment, the method further comprises the step of adding a detection agent to the sample.

Preferably, the step of "adding a detection agent to the sample" precedes the step of "determining the presence and/or amount and/or concentration and/or size of individual amyloid aggregates in the sample".

In one embodiment, the detection agent binds to and/or associates with and/or reacts with individual amyloid aggregates. Preferably, the detection agent binds to and/or associates with and/or reacts with individual amyloid aggregates; leading the detection agent to become detectable and/or measurable, or to become more easily detectable and/or more easily measurable and/or to produce a higher measurable output.

By "the detection agent binds to individual amyloid aggregates", we include that the detection agent forms a molecular bond (such as covalent bond) with the individual amyloid aggregates.

By "the detection agent associates with individual amyloid aggregates", we include that the detection agent forms a non-covalent interaction (such as an electrostatic interaction, and/or a pi ($\pi$)- interaction, and/or a van der Waals interaction, and/or a hydrophobic interaction) with the individual amyloid aggregates.

By "the detection agent reacts with individual amyloid aggregates", we include that the detection agent does not necessarily need to be bound to or associated with the individual amyloid aggregates and/or in proximity to the individual amyloid aggregates in order to function.

By "leading the detection agent to become detectable", we include that when the detection agent is bound to and/or associated with and/or reacting in the presence of individual amyloid aggregates, it is possible to identify or detect the presence of the detection agent. To put another way, if the sample comprised no individual amyloid aggregates it would not be possible to identify or detect the presence of the detection agent, using the method described herein.

By "leading the detection agent to become measurable", we include that when the detection agent is bound to and/or associated with and/or reacting in the presence of individual amyloid aggregates, it is possible to quantify the amount of detection agent and/or assess the relative amount or relative level of the detection agent. The amount or level of the detection agent can be used to quantify the amount and/or concentration and/or size of the individual amyloid aggregates.

In one embodiment, the determination comprises the detection and/or measurement of the detection agent. For the avoidance of doubt, this determination can occur as part of the step of: "determining the presence and/or amount and/or concentration and/or size of individual amyloid aggregates in the sample".

By "detection of the detection agent", we include that the presence of the detection agent is detected or identified. By "measurement of the detection agent", we include quantifying the amount of detection agent and/or assessing the relative amount or relative level of the detection agent. The amount or level of the detection agent can be used to quantify the amount and/or concentration and/or size of the individual amyloid aggregates.

In one embodiment, the detection agent binds to and/or associates with and/or reacts with individual amyloid aggregates that comprise one or more beta $\beta$-sheets.

In one embodiment, the detection agent comprises a fluorescent moiety and/or a bioluminescent moiety. One skilled in biology and/or chemistry would understand what is encompassed by the terms "fluorescent moiety" and "bioluminescent moiety".

In one embodiment, the detection agent comprises a fluorescent moiety and produces or releases fluorescence when it binds to and/or associates with and/or reacts with individual amyloid aggregates.

In a preferred embodiment, a detection agent comprising a fluorescent agent is added, and the measurement and/or detection of fluorescence from the detection agent is used to determine the presence and/or amount and/or concentration and/or size of individual amyloid aggregates in the sample.

In a further alternative embodiment, the detection agent comprises a bioluminescence moiety and produces or releases luminescence when it binds to and/or associates with and/or reacts with individual amyloid aggregates.

In a preferred embodiment, the detection agent is one or more from the group consisting of: Thioflavin T (ThT) and/or ARCAM1 and/or pentameric formyl thiophene acetic acid (pFTAA) and/or 4-(dicyanovinyl)- julolidine (DCVJ) and/or 1-amino-8-naphtalene sulphonate (ANS) and/or bis-ANS and/or 2-[N-bis-(3-dimethylaminopropyl)-amino]-4-[2,3-dihydro-3-methyl-(benzo-1,3-thiazol-2-yl)-methylidene]-1-phenyl-quinolinium (PicoGreen).

ARCAM1 is an aryl cyano amide, which binds to beta ($\beta$)-sheets (Guan et al., 2015, ACS Chemical Neuroscience, 6: 1503-1508).

In a further aspect, not encompassed by the wording of the claims, the detection agent is one or more from the group comprising or consisting of: a variant or derivative or modified Thioflavin T (ThT) and/or ARCAM1 and/or pentameric formyl thiophene acetic acid (pFTAA) and/or 4-(dicyanovinyl)- julolidine (DCVJ) and/or 1-amino-8-naphtalene sulphonate (ANS) and/or bis-ANS and/or 2-[N-bis-(3-dimethylaminopropyl)-amino]-4-[2,3-dihydro-3-methyl-(benzo-1,3-thiazol-2-yl)-methylidene]-1-phenyl-quinolinium (PicoGreen).

In one embodiment, the amyloid-associated disease is associated with the individual amyloid aggregates, preferably individual amyloid aggregates comprising one or more beta ($\beta$)-sheets.

By "the amyloid-associated disease is associated with the individual amyloid aggregates", we include that the amyloid-associated disease is caused or worsened by the presence and/or accumulation of the individual amyloid aggregates.

In one embodiment, the amyloid-associated disease is one or more selected from the group comprising: Alzheimer's disease; and/or Parkinson's disease; and/or Huntington's disease; and/or amyotrophic lateral sclerosis (ALS); and/or type 2 diabetes; and/or systemic amyloidosis; and/or a prion disease (such as Creutzfeldt-Jakob disease (CJD));

and/or amyloid light-chain amyloidosis; and/or multiple myeloma; and/or inflammation; and/or kidney failure; and/or Leukocyte Chemotactic Factor 2 (LECT2) amyloidosis; and/or transthyretin-related hereditary amyloidosis with polyneuropathy (also known as Familial Amyloid Polyneuropathy (FAP) or Skelleftesjukan); and/or haemodialysis-associated amyloidosis; and/or Cerebral amyloid angiopathy; and/or familial visceral amyloidosis; and/or primary cutaneous amyloidosis; and/or cerebral amyloid angiopathy; and/or prolactinoma; and/or familial corneal amyloidosis; and/or senile amyloid of the atria of heart; and/or medullary carcinoma of the thyroid; and/or pharmaceutical insulin-derived amyloidosis. The aforementioned diseases would be known to one skilled in medicine.

In a preferred embodiment, the amyloid-associated disease is Alzheimer's disease.

In an alternative aspect, the amyloid-associated disease is Parkinson's disease.

In a further alternative aspect, the amyloid-associated disease is Huntington's disease.

In a further alternative aspect, the amyloid-associated disease is amyotrophic lateral sclerosis (ALS).

Alzheimer's disease is a chronic neurodegenerative disease, which slowly develops over time and for which there is usually of life expectancy of three to nine years following diagnosis. However, the diagnosis of Alzheimer's is often very delayed due to a lack of reliable earlier markers of the disease. Currently, diagnosis relies on a patient exhibiting behavioural symptoms which are often quite subtle and difficult to observe, such as short term memory of loss. Based on the current methods, it is also quite difficult to provide a prognosis for how quickly the patient is likely to deteriorate. Therefore, there is a particular need for an effective method to diagnose Alzheimer's disease early, and to be able to provide a reliable prognosis. The current invention solves this problem by detecting the individual amyloid aggregates, which the inventors have demonstrated provide an effective early diagnostic marker, and a reliable marker by which to assess prognosis.

Similarly, Parkinson's disease Huntington's disease and ALS are chronic diseases that are diagnosed using behavioural symptoms, and which lack effective early diagnostic and prognostic markers. As with all the amyloid-associated diseases described herein, there is a particular need for effective methods to diagnose Parkinson's disease, Huntington's disease and ALS early, and to be able to provide a reliable prognosis. The current disclosure solves this problem by detecting the individual amyloid aggregates, which the inventors have demonstrated provide an effective early diagnostic marker, and a reliable marker by which to assess prognosis.

In one embodiment, the Alzheimer's disease is selected from the group comprising: Subjective Cognitive Impairment; Mild Cognitive impairment; Alzheimer's disease; and Alzheimer's disease with vascular dementia. These types of Alzheimer's disease would be known to one skilled in medicine.

Subjective Cognitive Impairment (SCI), also known as subjective memory disorder, relates to a subject having a worsening of their cognitive abilities, including memory impairment, but where that worsening cannot be verified by standard tests. In the absence of objective cognitive dysfunction or depression, SCI is sometimes regarded to be a common transitional stage in normal aging.

Mild Cognitive Impairment (MCI), also known as incipient dementia or isolated memory impairment, is a brain function syndrome involving the onset and evolution of cognitive impairments beyond those expected based on the age of the subject, but which are not significant enough to interfere with their daily activities. MCI may occur as a transitional stage between normal aging and dementia. The cause of MCI is unknown and there is therefore no prevention and no treatment. In the US, MCI has been designated the designated as G31.84 in the American ICD-10-CM medical diagnosis code.

Alzheimer's disease (AD) is a progressive neurodegenerative disease that is characterised by irreversible destruction of nerve cells and neural connections in the cerebral cortex of the brain and by a significant loss of brain mass. As a result, dementia symptoms occur that worsen over time. Eventually, the disease becomes severe enough to render the subject incapable of performing daily tasks and unable of living without constant care. While much is known about underlying biochemical changes, the cause of AD is still not known and there is presently no cure for AD. As discussed herein, there are also no reliable biomarkers for early AD diagnosis and no reliable disease progression biomarkers. Therapeutic agents commonly used for AD treatment slow disease progression and alleviate some symptoms and/or signs, but don't cure the patient.

Alzheimer's disease with vascular dementia also known as mixed dementia is an often encountered comorbidity that includes both AD and vascular dementia, *i.e.* cognitive decay caused by reduced blood supply to the brain. In Alzheimer's disease with vascular dementia symptoms of dementia, such as memory and language problems, loss of the ability to focus and pay attention, difficulties with reasoning and judgement, trouble with visual perception *etc.* are potentiated due to dual causes.

In one aspect, the individual amyloid aggregates comprise one or more peptide or protein selected from the list comprising: amyloid beta (β) peptide (β peptide) (such as: beta (β)40 and/or beta (β)42 and/or beta (β)43); and/or amyloid light chain; and/or serum amyloid A protein (SAA); and/or Leukocyte Chemotactic Factor 2 (LECT2); and/or transthyretin; and/or beta (β)2 microglobulin; and/or amylin; and/or prion protein; and/or cystatin C (CST3); and/or apolipoprotein A1 (APOA1); and/or fibrinogen alpha chain (FGA); and/or lysozyme (LYZ); and/or oncostatin-M specific receptor subunit beta (OSMR); and/or integral membrane protein 2B (ITM2B); and/or prolactin; and/or keratoepithelin;

and/or atrial natriuretic factor; and/or calcitonin; and/or Huntingtin; and/or alpha ($\alpha$)-Synuclein; and/or insulin.

In one embodiment, the amyloid-associated disease is Alzheimer's disease, and the individual amyloid aggregates comprise an amyloid beta ($\beta$) peptide, for example $\beta$40 and/or $\beta$42 and/or $\beta$43. In an aspect, the amyloid-associated disease is Alzheimer's disease, and the individual amyloid aggregates comprise $\beta$40 and $\beta$42. In an alternative aspect, the amyloid-associated disease is Alzheimer's disease, and the individual amyloid aggregates comprise $\beta$40. In a further alternative aspect, the amyloid-associated disease is Alzheimer's disease, and the individual amyloid aggregates comprise $\beta$42. In an additional alternative aspect, the amyloid-associated disease is Alzheimer's disease, and the individual amyloid aggregates comprise $\beta$40 and/or $\beta$42 alone or in co-aggregates with other peptides and/or proteins.

In various aspects:

- the amyloid-associated disease is amyloid light chain amyloidosis and the individual amyloid aggregates comprise amyloid light chain; and/or
- the amyloid-associated disease is multiple myeloma and the individual amyloid aggregates comprise amyloid light chain; and/or
- the amyloid-associated disease is inflammation and the individual amyloid aggregates comprise serum amyloid A protein (SAA); and/or
- the amyloid-associated disease is Leukocyte Chemotactic Factor 2 (LECT2) amyloidosis and the individual amyloid aggregates comprise Leukocyte Chemotactic Factor 2 (LECT2); and/or
- the amyloid-associated disease is kidney failure and the individual amyloid aggregates comprise Leukocyte Chemotactic Factor 2 (LECT2); and/or
- the amyloid-associated disease is transthyretin-related hereditary amyloidosis with polyneuropathy (also known as Familial Amyloid Polyneuropathy (FAP) or Skelleftesjukan) and the individual amyloid aggregates comprise transthyretin; and/or
- the amyloid-associated disease is Familial Amyloid Polyneuropathy (FAP) and the individual amyloid aggregates comprise transthyretin; and/or
- the amyloid-associated disease is haemodialysis-associated amyloidosis and the individual amyloid aggregates comprise beta ($\beta$)2 microglobulin; and/or
- the amyloid-associated disease is type 2 diabetes and the individual amyloid aggregates comprise amylin; and/or
- the amyloid-associated disease is prion diseases and the individual amyloid aggregates comprise prion protein; and/or
- the amyloid-associated disease is cerebral amyloid angiopathy and the individual amyloid aggregates comprise cystatin C (CST3); and/or
- the amyloid-associated disease is familial visceral amyloidosis and the individual amyloid aggregates comprise apolipoprotein A1 (APOA1) and/or fibrinogen alpha chain (FGA) and/or lysozyme (LYZ); and/or
- the amyloid-associated disease is primary cutaneous amyloidosis and the individual amyloid aggregates comprise oncostatin-M specific receptor subunit beta (OSMR); and/or
- the amyloid-associated disease is cerebral amyloid angiopathy and the individual amyloid aggregates comprise integral membrane protein 2B (ITM2B); and/or
- the amyloid-associated disease is prolactinoma and the individual amyloid aggregates comprise prolactin; and/or
- the amyloid-associated disease is familial corneal amyloidosis and the individual amyloid aggregates comprise keratoepithelin; and/or
- the amyloid-associated disease is senile amyloid of atria of the heart and the individual amyloid aggregates comprise atrial natriuretic factor; and/or
- the amyloid-associated disease is medullary carcinoma of the thyroid and the individual amyloid aggregates comprise calcitonin; and/or
- the amyloid-associated disease is Huntington's disease and the individual amyloid aggregates comprise Huntingtin; and/or
- the amyloid-associated disease is Parkinson's disease and the individual amyloid aggregates comprise alpha ($\alpha$)-Synuclein; and/or
- the amyloid-associated disease is pharmaceutical insulin-derived amyloidosis and the individual amyloid aggregates comprise insulin; and/or
- the amyloid-associated disease is amyotrophic lateral sclerosis (ALS) and the individual amyloid aggregates comprise copper-zinc superoxide dismutase 1 (SOD1); and/or
- the amyloid-associated disease is systemic amyloidosis and the individual amyloid aggregates comprise monoclonal immunoglobulin (Ig) light (L) chains or L-chain fragments.

It will be appreciated that whilst an amyloid aggregate might primarily comprise a single type of peptide or protein (for example, when the amyloid-associated disease is amyloid light chain amyloidosis, the individual amyloid aggregates may primarily comprise amyloid light chain), in some circumstances the amyloid aggregate may also comprise additional types of peptide and/or protein.

In one aspect, not encompassed by the wording of the claims, the (in particular, wherein the amyloid-associated disease is Alzheimer's disease) method further comprises the steps of:

- determining the presence and/or amount and/or concentration and/or size of Tau protein in the sample; and,
- providing a diagnosis and/or prognosis of the amyloid-associated disease on the basis of the determination.

Tau is a marker of neuro degeneration, can be used in the diagnosis and prognosis of Alzheimer's disease, and can be detected in bodily fluids, such as cerebrospinal fluid (CSF), serum, saliva and urine (Hei-Nga Chan, Di Xu, See-Lok Ho, Man Shing Wong ORCID logo* and Hung-Wing Li, Ultra-sensitive detection of protein biomarkers for diagnosis of Alzheimer's disease. Chem. Sci., 2017, 8, 4012-4018). Combining the identification of biomarkers generally can improve methods of diagnosis and prognosis, as could be the case with combining the methods described herein relating to individual amyloid aggregates, and methods relating to Tau.

In one aspect, the Tau protein is phosphorylated Tau protein.

In one aspect in which the presence and/or amount and/or concentration and/or size of Tau protein is determined, the method further comprises the step of adding a further detection agent that binds to and/or associates with and/or reacts in the presence of Tau, such as pentameric formyl thiophene acetic acid (pFTAA).

Methods of detecting of Tau using pFTAA were described by Brelstaff and colleagues (Brelstaff et al. Front Neurosci. 2015 May 29;9:184). Additional guidance on detecting Tau protein can be found in Chan et al., 2017 (Hei-Nga Chan, Di Xu, See-Lok Ho , Man Shing Wong ORCID logo* and Hung-Wing Li, Ultra-sensitive detection of protein biomarkers for diagnosis of Alzheimer's disease. Chem. Sci., 2017, 8, 4012-4018).

In one embodiment, determining the presence and/or amount and/or concentration and/or size of individual amyloid aggregates is at an individual amyloid aggregate resolution.

By "individual amyloid aggregate resolution", we include that the method detects and/or measures single amyloid aggregates in the sample (i.e. the method does not simultaneously detect and/or measure a number of individual amyloid aggregates in the sample), and/or the method detects and/or measures each individual amyloid aggregate in the sample. Alternatively, "individual amyloid aggregate resolution" may be referred to as a "single particle resolution" or "single molecule resolution".

In one embodiment, determining the presence and/or amount and/or concentration and/or size of individual amyloid aggregates comprises time-resolved detection. Put another way, determining the presence and/or amount and/or concentration and/or size of individual amyloid aggregates is conducted using time-resolved detection. "Time-resolved detection" methods would be well known to those skilled in chemistry, and are described in detail in the Examples herein. As discussed in the Examples, time-resolved detection is particularly effective for use with the methods described herein.

In one embodiment the method encompasses an analysis means based on fluorescence correlation spectroscopy, where the number of observed molecules/particles/aggregates in an observation volume element is sufficiently low to enable individual molecules/particles/aggregates substantially to contribute to the measured signal. The number of molecules/particles/aggregates in the volume element should preferably be below about 1000, below about 500, below about 100, such as below about 50, below about 25. The number of observed molecules/particles/aggregates in a volume element is inter alia a function of the size (volume) of the observation volume element and the concentration of the aggregates in the solution. As the number of molecules/particles/aggregates in the observation volume element increases, the contribution of individual aggregates to a measured signal decreases. At a high number of aggregates in the volume element, the correlation of an individual aggregate to a measured signal is low and eventually goes towards zero. If, however, the number of molecules/particles/aggregates is low, the passage of individual molecules/particles/aggregates through the observation volume element is being observed. In this case, the measured signal may therefore be referred to as a single event occurrence (seo).

It is also advantageous that the aggregates have a high fluorescent yield, which implies that it is preferred to have a detection agent that is capable of associating with individual amyloid aggregates of interest to yield an inclusion compound with high fluorescent yield whilst the detection compound is non/weakly fluorescent when on its own. A detection agent with a high fluorescent yield is capable of providing a high number of photons when reverting to a lower energy level. It is further beneficial for the fluorescence correlation spectroscopy-based analysis to include a detector having single-photon sensitivity.

According to an embodiment there is no need for amplification of any amyloidal compound such as amyloid peptide (protein), amyloid monomer, amyloid multimer, amyloid aggregate, amyloid macro aggregate, before the analysis. There is specifically no need for further amplification of amyloidal entities if an analysis technology is implemented providing single molecule (particle) resolution. Hence, there is no need for the implementation of an amyloid amplification step in the method of the present invention comprising fluorescent spectroscopy analysis, such as single molecule resolution fluorescent spectroscopy.

According to yet a further embodiment the method does not necessitate the use of non-target amyloid molecules/peptides/proteins/aggregates (amyloid entities) or the inclusion of a step comprising mixing target amyloid molecules/peptides/proteins/aggregates and non-target amyloid molecules/aggregates. Non-target amyloid entities include any amyloid entities not stemming from the body fluid to be analysed or manipulated amyloid entities from the body fluid to be analysed. Examples of non-target amyloid entities include recombinantly formed amyloid entities or amyloid entities derived from pathological depositions (which may originate from the amyloid entities found in the body fluid to be analysed). The mixture of non-target and target amyloid entities induces agglomeration (polymerisation) amplifying the amount of aggregated amyloid.

Hence, an embodiment relates to a method for the diagnosis and/or prognosis of an amyloid-associated disease in a subject, wherein the method comprises the steps of:

- adding a detection agent to a sample of bodily fluid from the subject, the detection agent binding to and/or associating with and/or reacting with individual amyloid aggregates; and
- determining the presence and/or amount and/or concentration and/or size of individual amyloid aggregates in the sample;

and where the method does not necessitate the use of non-target amyloid entities (non-target amyloid molecules and/or peptides and/or proteins and/or aggregates).

In time-resolved detection, the step of "determining the presence and/or amount and/or concentration and/or size of individual amyloid aggregates" is not undertaken on the whole sample simultaneously. Instead, a fraction of the sample is measured at a time with numerous measurements of fractions of the sample being taken over a unit of time. In some embodiments, the fraction of the sample being measured is the "observation volume element", as described below. When using time-resolved detection only individual amyloid aggregates that are present within the fraction of the sample are measured and/or detected. Accordingly, the presence and/or amount and/or concentration and/or size of individual amyloid aggregates is determined based on the frequency of sample fractions in which individual amyloid aggregates are detected and/or measured, and/or based on the characteristics of those individual amyloid aggregates. As would be appreciated, "time-resolved detection" could be defined as photon counting with sub-microsecond resolution.

Accordingly, in one embodiment the step of "determining the presence and/or amount and/or concentration and/or size of individual amyloid aggregates in the sample" comprises determining the frequency (such as the frequency of occurrence) of individual amyloid aggregates in the sample (such as in fractions of a sample), where the method is conducted using time-resolved detection.

In this embodiment, the higher the frequency of the individual amyloid aggregates, the higher the amount and/or concentration of the individual amyloid aggregates. The concepts of measuring frequency are explained herein (in particular in the Examples), and are applicable to each of the aspects of the invention described herein.

In a further aspect, determining the presence and/or amount and/or concentration and/or size of individual amyloid aggregates in the sample comprises determining the frequency of individual amyloid aggregates in a unit of time.

In some aspects, the frequency of individual amyloid aggregates is referred to as the frequency of single event occurrences ($f_{SEO}$). The relevance of single event occurrence ($f_{SEO}$) to the diagnosis and/or prognosis of the amyloid-associated disease is exemplified in Table 1. A single event occurrence equates to the detection of an individual amyloid aggregate. In one aspect, the frequency of single event occurrence ($f_{SEO}$) can be proportional to the concentration of individual amyloid aggregates. In a related embodiment, the frequency of single event occurrences ($f_{SEO}$) is calculated as the average number of single event occurrences per unit of time.

In one aspect, the unit of time is about 0.05 microseconds ($\mu$s) or more; for example, about 0.1 $\mu$s or more; about 0.15 $\mu$s or more; about 0.2 $\mu$s or more; about 0.25 $\mu$s or more; about 0.3 $\mu$s or more; about 0.35 $\mu$s or more; about 0.4 $\mu$s or more; about 0.45 $\mu$s or more; about 0.5 $\mu$s or more; about 0.6 $\mu$s or more; about 0.7 $\mu$s or more; about 0.8 $\mu$s or more; about 0.9 $\mu$s or more; about 1 $\mu$s or more; about 1.5 $\mu$s or more; about 2 $\mu$s or more; about 2.5 $\mu$s or more; about 3 $\mu$s or more; about 4 $\mu$s or more; about 5 $\mu$s or more; about 6 $\mu$s or more; about 7 $\mu$s or more; about 8 $\mu$s or more; about 9 $\mu$s or more; about 10 $\mu$s or more; about 20 $\mu$s or more; about 30 $\mu$s or more; about 40 $\mu$s or more; about 50 $\mu$s or more; about 60 $\mu$s or more; about 70 $\mu$s or more; about 80 $\mu$s or more; about 90 $\mu$s or more; about 0.1 millisecond or more; about 0.2 millisecond or more; about 0.3 millisecond or more; about 0.4 millisecond or more; about 0.5 millisecond or more; about 0.6 millisecond or more; about 0.7 millisecond or more; about 0.8 millisecond or more; about 0.9 millisecond or more; about 1 millisecond or more; about 2 milliseconds or more; about 3 milliseconds or more; about 4 milliseconds or more; about 5 milliseconds or more; about 6 milliseconds or more; about 7 milliseconds or more; about 8 milliseconds or more; about 9 milliseconds or more; about 10 milliseconds or more; about 20 milliseconds or more; about 30 milliseconds or more; about 40 milliseconds or more; about 50 milliseconds or more; about 60 milliseconds or more; about 70 milliseconds or more; about 80 milliseconds or more; about 90 milliseconds or more; about 0.1 second or more; about 0.2 second or more; about 0.3 second or more; about 0.4 second or more; about 0.5 second or more;

about 0.6 second or more; about 0.7 second or more; about 0.8 second or more; about 0.9 second or more; about 1 second or more; about 2 seconds or more; about 3 seconds or more; about 4 second or more; about 5 seconds or more; about 6 seconds or more; about 7 seconds or more; about 8 seconds or more; about 9 seconds or more; about 10 seconds or more; about 20 seconds or more; about 30 seconds or more; about 40 seconds or more; about 50 seconds or more; about 60 seconds or more; about 120 seconds or more; about 180 seconds or more; about 240 seconds or more; about 300 seconds or more; about 360 seconds or more; about 420 seconds or more; about 480 seconds or more; about 540 seconds or more; about 600 seconds or more; about 1200 seconds or more; about 1800 seconds or more; about 2400 seconds or more; about 3000 seconds or more; or about 3600 seconds or more, preferably about 0.2 $\mu$s or more or about 3,000 seconds or more.

In one aspect, a diagnosis and/or prognosis of the amyloid-associated disease is provided if the frequency of individual amyloid aggregates (such as the frequency of the single event occurrence ($f_{SEO}$)) is about 0.1 or more; for example: about 0.2 or more; about 0.3 or more; about 0.4 or more; about 0.5 or more; about 0.6 or more; about 0.7 or more; about 0.8 or more; about 0.9 or more; about 1 or more; about 1.1 or more; about 1.2 or more; about 1.3 or more; about 1.4 or more; about 1.5 or more; about 1.6 or more; about 1.7 or more; about 1.8 or more; about 1.9 or more; about 2 or more; about 2.2 or more; about 2.4 or more; about 2.6 or more; about 2.8 or more; about 3 or more; about 3.2 or more; about 3.4 or more; about 3.6 or more; about 3.8 or more; about 4 or more; about 4.2 or more; about 4.4 or more; about 4.6 or more; about 4.8 or more; about 5 or more; about 5.5 or more; about 6 or more; about 6.5 or more; about 7 or more; about 7.5 or more; about 8 or more; about 8.5 or more; about 9 or more; about 9.5 or more; or about 10 or more, preferably about 1.5 or more.

In a further aspect, a diagnosis and/or prognosis of the amyloid-associated disease is provided if the frequency of individual amyloid aggregates (such as the frequency of the single event occurrence ($f_{SEO}$)) is about $0.1\times10^{-3}$ s$^{-1}$ or more; for example: about $0.2\times10^{-3}$ s$^{-1}$ or more; about $0.3\times10^{-3}$ s$^{-1}$ or more; about $0.4\times10^{-3}$ s$^{-1}$ or more; about $0.5\times10^{-3}$ s$^{-1}$ or more; about $0.6\times10^{-3}$ s$^{-1}$ or more; about $0.7\times10^{-3}$ s$^{-1}$ or more; about $0.8\times10^{-3}$ s$^{-1}$ or more; about $0.9\times10^{-3}$ s$^{-1}$ or more; about $1\times10^{-3}$ s$^{-1}$ or more; about $1.1\times10^{-3}$ s$^{-1}$ or more; about $1.2\times10^{-3}$ s$^{-1}$ or more; about $1.3\times10^{-3}$ s$^{-1}$ or more; about $1.4\times10^{-3}$ s$^{-1}$ or more; about $1.5\times10^{-3}$ s$^{-1}$ or more; about $1.6\times10^{-3}$ s$^{-1}$ or more; about $1.7\times10^{-3}$ s$^{-1}$ or more; about $1.8\times10^{-3}$ s$^{-1}$ or more; about $1.9\times10^{-3}$ s$^{-1}$ or more; about $2\times10^{-3}$ s$^{-1}$ or more; about $2.2\times10^{-3}$ s$^{-1}$ or more; about $2.4\times10^{-3}$ s$^{-1}$ or more; about $2.6\times10^{-3}$ s$^{-1}$ or more; about $2.8\times10^{-3}$ s$^{-1}$ or more; about $3\times10^{-3}$ s$^{-1}$ or more; about $3.2\times10^{-3}$ s$^{-1}$ or more; about $3.4\times10^{-3}$ s$^{-1}$ or more; about $3.6\times10^{-3}$ s$^{-1}$ or more; about $3.8\times10^{-3}$ s$^{-1}$ or more; about $4\times10^{-3}$ s$^{-1}$ or more; about $4.2\times10^{-3}$ s$^{-1}$ or more; about $4.4\times10^{-3}$ s$^{-1}$ or more; about $4.6\times10^{-3}$ s$^{-1}$ or more; about $4.8\times10'^{3}$ s$^{-1}$ or more; about $5\times10^{-3}$ s$^{-1}$ or more; about $5.5\times10^{-3}$ s$^{-1}$ or more; about $6\times10^{-3}$ s$^{-1}$ or more; about $6.5\times10^{-3}$ s$^{-1}$ or more; about $7\times10^{-3}$ s$^{-1}$ or more; about $7.5\times10^{-3}$ s$^{-1}$ or more; about $8\times10^{-3}$ s$^{-1}$ or more; about $8.5\times10^{-3}$ s$^{-1}$ or more; about $9\times10^{-3}$ s$^{-1}$ or more; about $9.5\times10^{-3}$ s$^{-1}$ or more; or about $10\times10^{-3}$ s$^{-1}$ or more, preferably about $1.5\times10^{-3}$ s$^{-1}$ or more.

In a preferred aspect, determining the presence and/or amount and/or concentration and/or size of individual amyloid aggregates in the sample comprises determining the frequency of individual amyloid aggregates in a unit of time of about 50 minutes or more, and a diagnosis and/or prognosis of the amyloid-associated disease is provided if the frequency of single event occurrence ($f_{SEO}$) is about 1.5 (such as $1.5\times10^{-3}$ s$^{-1}$) or more.

In one aspect, the characteristics of a single event occurrence ($f_{SEO}$) and the decay time, *i.e.* the diffusion time ($\tau_D$) can be proportional to the amount and size of individual amyloid aggregates, respectively. By characteristics of a single event occurrence ($f_{SEO}$), we include the signal (or intensity) of the single event occurrence. By characteristics of the decay time we include the intensity of the diffusion time ($\tau_D$).

From the distribution of diffusion times, a profile of molecules/particles/aggregates sizes can be determined for each individual. The distribution of sizes may reflect on the disease state and provide information about disease prognosis.

In one aspect, a diagnosis and/or prognosis of the amyloid-associated disease is provided if the decay time of the single event occurrence ($f_{SEO}$) or the diffusion time ($\tau_D$) is increased.

Observing single event occurrences (such as the passage of individual structured soluble amyloid aggregates one at a time) allows an analysis of the time that it takes for the amyloid aggregate to pass through the observation volume element (such as to analyse the width of the individual fluorescence intensity bursts). The larger the structured soluble amyloid aggregates, the slower their movement, and the wider is the burst. To determine the average time that it takes for a structured soluble amyloid aggregate to pass through the observation volume element, temporal autocorrelation is used to analyse the signal and derive a temporal autocorrelation curve (tACC). The characteristic decay time of the tACC is short for small structured soluble amyloid aggregates, and it is long for large structured soluble amyloid aggregates.

Accordingly, in one embodiment the step of "determining the presence and/or amount and/or concentration and/or size of individual amyloid aggregates in the sample" comprises providing a temporal autocorrelation curve (tACC), in particular wherein the method comprises determining the frequency of individual amyloid aggregates. A further explanation of how to provide (or derive) a tACC is provided in the Examples.

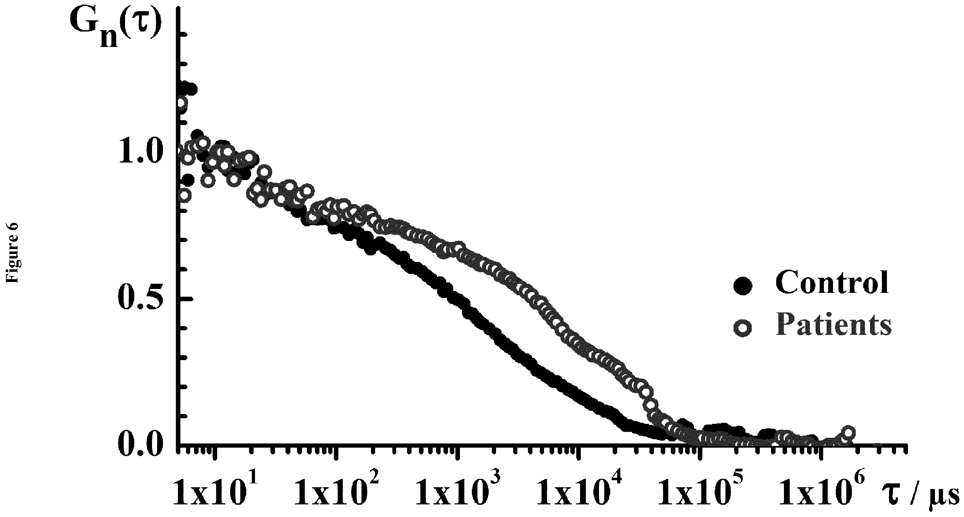

Data analysis presented in Figure 6 shows a clear difference in the characteristic decay time between the tACCs recorded for the two groups, with a markedly longer decay time for the patient group. Evaluation of the normalized tACCs shows that the analysed system is complex and does not contain particles that are uniform in size, but rather contains a mixture of particles of different sizes. The average diffusion time for the amyloid aggregates (which are bound to ThT) in the control group is about 5 ms, and the average diffusion time for particles in the patient group is about 15 ms. This indicates that the molecular weight difference in the amyloid aggregates is about 27 times between the two groups. Accordingly, the patient group can carry larger ThT-active structured aggregates in the serum in addition to the larger number of nano-plaques.

In one aspect, if the decay time (in particular, the decay time of the tACC) is longer in the sample when compared to one or more control samples (such one or more samples from one or more control subjects, as described below), a diagnosis and/or prognosis of the amyloid-associated disease is provided.

In one aspect, if the molecular weight of the amyloid aggregates in the sample is about 2 or more times higher when compared to one or more control samples (such one or more samples from one or more control subjects, as described below), a diagnosis and/or prognosis of the amyloid-associated disease is provided; for example: about three or more times higher; about four or more times higher; about five or more times higher; about six or more times higher; about seven or more times higher; about eight or more times higher; about nine or more times higher; about 10 or more times higher; about 11 or more times higher; about 12 or more times higher; about 13 or more times higher; about 14 or more times higher; about 15 or more times higher; about 16 or more times higher; about 17 or more times higher; about 18 or more times higher; about 19 or more times higher; about 20 or more times higher; about 21 or more times higher; about 22 or more times higher; about 23 or more times higher; about 24 or more times higher; about 25 or more times higher; about 26 or more times higher; about 27 or more times higher; about 28 or more times higher; about 29 or more times higher; or about 30 or more times higher, preferably about 27 or more times higher.

In one aspect, if the decay time (*i.e.* the diffusion time ($\tau_D$)) (in particular, the decay time of the tACC) is about 6 ms or more a diagnosis and/or prognosis of the amyloid-associated disease is provided; for example: about 7 ms or more; about 8 ms or more; about 9 ms or more; about 10 ms or more; about 11 ms or more; about 12 ms or more; about 13 ms or more; about 14 ms or more; about 15 ms or more; about 16 ms or more; about 17 ms or more; about 18 ms or more; about 19 ms or more; or about 20 ms or more, preferably about 15 ms or more.

In an alternative aspect, determining the presence and/or amount and/or concentration and/or size of individual amyloid aggregates comprises Total Internal Fluorescence Microscopy (TIRF); and/or image correlation spectroscopy; and/or enzyme-linked immunosorbent assay (ELISA); and/or massively parallel Fluorescence Correlation Spectroscopy (mpFCS). Put another way, determining the presence and/or amount and/or concentration and/or size of individual amyloid aggregates is conducted using Total Internal Fluorescence Microscopy (TIRF); and/or image correlation spectroscopy; and/or enzyme-linked immunosorbent assay (ELISA); and/or massively parallel Fluorescence Correlation Spectroscopy (mpFCS). Preferably, determining the presence and/or amount and/or concentration and/or size of individual amyloid aggregates comprises Total Internal Reflexion Fluorescence Microscopy (TIRF) and image correlation spectroscopy.

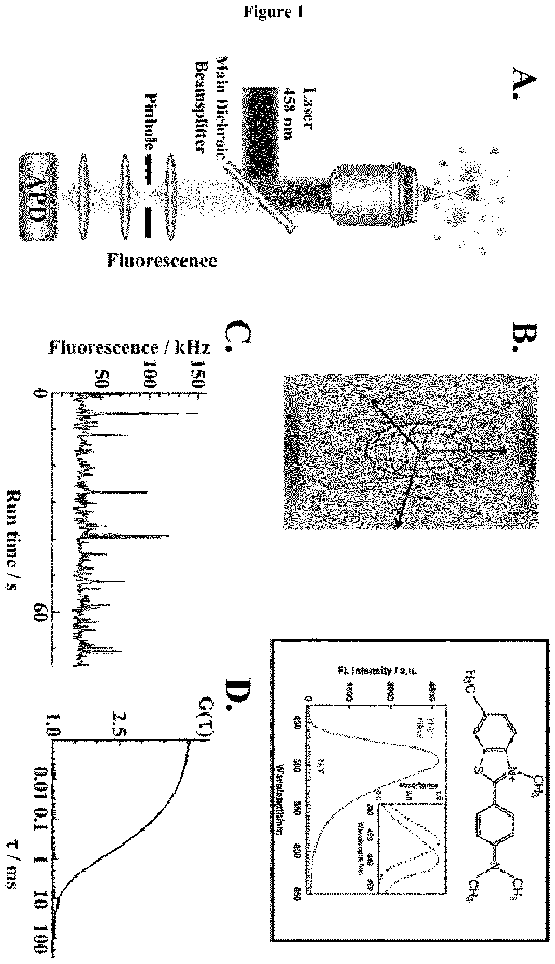

Fluorescence correlation spectroscopy is a suitable methodology providing single particle/aggregate/molecule resolution. In order to satisfactory achieve single molecule resolution it is important that the observation volume element detected by a suitable detector is small. The smaller the observation volume element, the fewer the number of particles that are present/observed at a given moment. Hence, the signal-to-background ratio improves with decreasing the observation volume element size. One option to achieve a small observation volume element is to have a specific arrangement of optical elements in the optical pathway between the sample and the detector, such as the conjugate focal plane arrangement in a confocal microscope. The volume of a thus obtained confocal observation volume element is smaller than about $10^{-11}$ litres and typically from about $10^{-13}$ down to about $10^{-16}$ litres. Figures 1-14 exemplify data obtained using a confocal optical arrangement.

Even smaller observation volume elements can be achieved by implementation of Total Internal Reflection (TIR). One alternative to provide TIR is to focus light on the back-focal plane of an optical element generating exiting, typically collimated, light angular to the (main) optical axis of the optical element. Dependent on the location of the focussing of the incident light at the back focal plane of the optical element the angle with respect to the optical axis of the optical element of the exiting light will vary. The optical element is suitably an objective. The objective for providing TIR of the exiting light has preferably a high numerical aperture, suitably above about 0.9, above about 1.0, above about 1.1, above about 1.2. The numerical aperture may be as high as around 2.0.

A further possibility to provide a very small observation volume element is the implementation of Stimulated Emission Depletion (STED) optical arrangement. Here, a light of specific wavelength and a distinct intensity profile (with an intensity minimum in the centre) is used to reduce the volume from which fluorescence is being emitted (Mueller et al. Biophys. J. 2011 101(7): 1651-1660 doi: 10.1016/j.bpj.2011.09.006.).

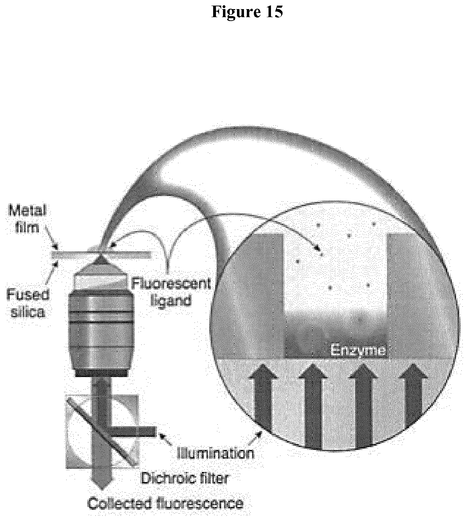

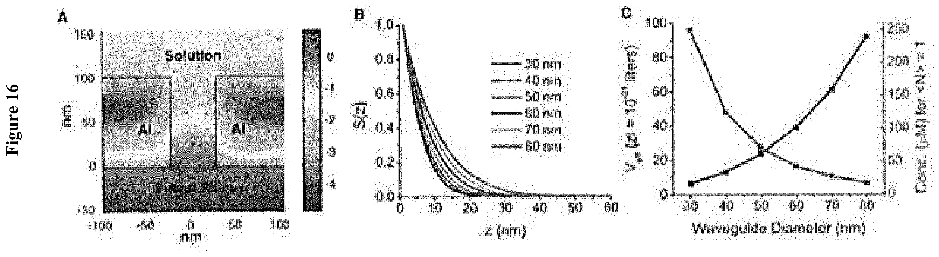

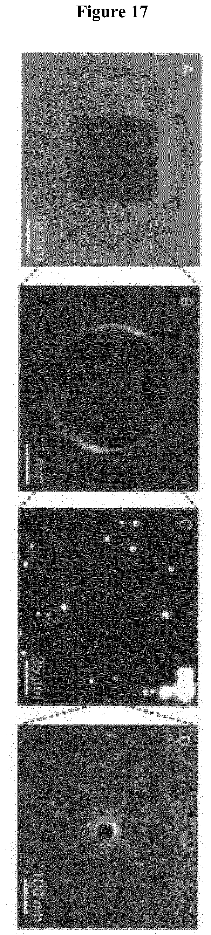

Yet another possibility to provide a very small observation volume element is the implementation of a transparent substrate on which a thin layer of a material is deposited that is capable of essentially blocking electromagnetic radiation from the UV-VIS region of the spectrum (Figures 15 and 16). The thin layer with electromagnetic blocking capability has a plurality of openings down to the transparent substrate, effectively forming wells (Figure 17). The openings dimension is such that only an evanescent field stemming from the impinging electromagnetic radiation can progress. The phenomena of generating an evanescent filed without total internal reflection is appearing when the dimension of the well, such as its diameter, is smaller than the wavelength of the incident light. Typically, the openings have a circular shape equating to a circularly shaped well bottom restricted by the transparent substrate with a diameter below the wave-length of the impinging light. Depending on the area of the well bottom, a volume element down to the zepto-litre ($10^{-21}$ litre) can be achieved. Diameters of the well bottom between about 30 to 80 nm generate volume elements from about $10 \times 10^{-21}$ litres up to about $100 \times 10^{-21}$ litres (Figure 17 b, c). These substrates with a thin electro-magnetic radiation-blocking layer comprising a plurality wells, preferably circularly formed, may be referred to as zepto-litre wave-guides. The electro-magnetic radiation-blocking material can be any suitable material capable of essentially blocking the electromagnetic radiation suitable for use in fluorescence spectroscopy. Suitable the electro-magnetic radiation-blocking material is a metal which is suited for vapour deposition processes. The electro-magnetic radiation-blocking layer may comprise several layers of different metals. Sometimes a second metal layer is situated between the transparent substrate and the main (primary) metal layer for the purpose of better securing the metal layer(s) to the transparent substrate. Examples of metals include those metals which significantly reduces the transmissivity at any given wavelength. Suitable metals are aluminium, chromium, gold. Thickness of the layer of a specific metal is a function of the metal and the wavelength. An example of a dual metal layer is chromium-gold layer where chromium is situated between the gold and the transparent substrate. The transparent substrate is suitably a silicon dioxide containing material such as fused silica. The zepto-litre waveguide typically has a plurality of wells in the nano-size. A zepto-litre waveguide may have several million nano-wells. Each nano-well may be (optically) aligned with an array of single photon avalanche photodiodes such that each discrete single photon counting pixels is aligned to each nano-well of the waveguide. Hence, the implementation of a zepto-litre waveguide provides an unprecedented signal to noise ratio in the realm of fluorescence correlation spectroscopy and the ability of massive parallelization.

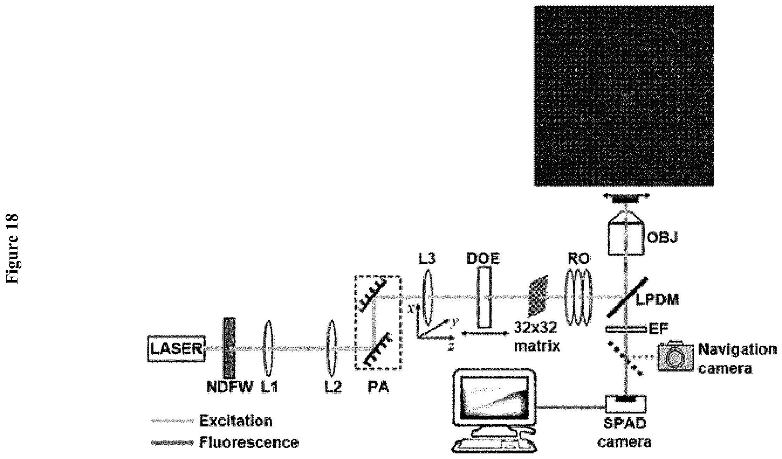

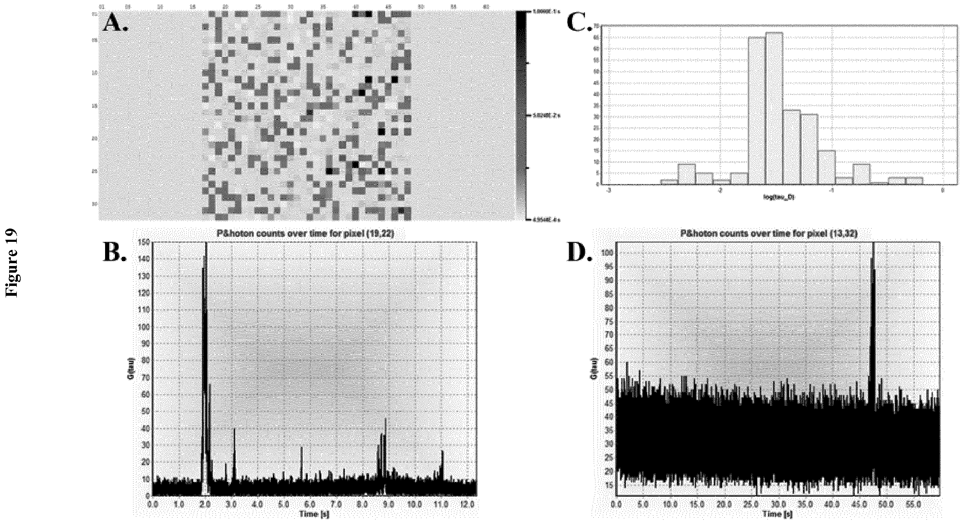

Fluorescence correlation spectroscopy using confocal volume elements can be parallelized by the implementation of an optical element capable of splitting up the incident beam of light into an array of light beams (plurality of light beams). A suitable optical element for splitting a beam is a diffractive optical element (DOE). Figure 18 presents an optical arrangement in an instrument for massively parallel Fluorescence Correlation Spectroscopy (mpFCS) comprising a diffractive optical element (DOE). The DOE provides an array (here: $32 \times 32$) of light beams providing, after passage through a high numerical aperture objective, 1024 confocal volume elements in the sample. The DOE can be configured to provide any suitable number of secondary beams. With a suitable DOE one can easily provide up to about 10,000 individual beams, up to about 100,000, and even up to 1 million individual beams. Moreover, by using a multiple confocal arrangement, the measurement time can be significantly shortened, e.g. from 3000 s (Figures 1-14) to 60 s (Figures 18 and 19) and the distribution of diffusion times (Figure 19C) can be efficiently derived. This, in turn, enables us to determine for each individual and with great statistical confidence the molecules/particle/aggregate size distribution profile. This is invaluable for determining how far the disease has progressed and/or to predict how fast it will progress.

The methods of Total Internal Reflection Fluorescence (TIRF) microscopy; and/or image correlation spectroscopy; and/or enzyme-linked immunosorbent assay (ELISA) and/or massively parallel Fluorescence Correlation Spectroscopy (mpFCS) would be known to one skilled in physics/physical chemistry.

In one embodiment, the time-resolved detection comprises Fluorescence Correlation Spectroscopy (FCS), which is using temporal autocorrelation analysis to analyse the data. Put another way, the time-resolved detection is conducted using Fluorescence Correlation Spectroscopy (FCS). "Fluorescence Correlation Spectroscopy (FCS)" is a method used to detect fluorescence which would be known to those skilled in chemistry, and is described in detail in the Examples herein. As discussed in the Examples, Fluorescence Correlation Spectroscopy (FCS) is particularly effective for use with the methods of the invention.

In one aspect, the FCS is a multiplex FCS. A multiplex FCS allows for the measurement of more samples by allowing more than one sample to be measured simultaneously, and so is advantageous as it increases the throughput of the method.

In one embodiment, fluorescence detected by Fluorescence Correlation Spectroscopy (FCS) represents the presence and/or amount and/or concentration and/or size of the individual amyloid aggregates. In a preferred embodiment, the method further comprises the step of adding a detection agent comprising a fluorescent moiety to the sample, and the fluorescence emitted by the fluorescent moiety is detected by Fluorescence Correlation Spectroscopy (FCS) and represents the presence and/or amount and/or concentration and/or size of the individual amyloid aggregates.

In one embodiment, the frequency of individual amyloid aggregates is calculated using fluorescence intensity fluctuation analysis (FIFA). FIFA can be calculated as the number of peaks (*i.e.* individual amyloid aggregates) per unit of time, wherein the peak has an intensity which is larger than at least two or five standard deviations of the control sample such as "a control level of fluorescence in the sample", as discussed below (preferably, at least five times larger than the standard deviation of the control sample).

In one embodiment, a diagnosis and/or prognosis of the amyloid-associated disease is provided if the presence and/or amount and/or concentration of the individual amyloid aggregates in the sample is higher than the presence and/or amount and/or concentration of the individual amyloid aggregates in a control sample; and/or

the size of the individual amyloid aggregates in the sample is larger than the size of the individual amyloid aggregates in a control sample.

A control sample suitable for use as part of the invention would be known to one skilled in biology or medicine, which includes the controls outlined herein. In some embodiments, the control sample is one or more selected from the list comprising or consisting of: a control sample from one or more control subjects; an *in vitro* control sample; a negative control sample; and a control level of fluorescence in the sample.

By "control subject", we include a subject that does not have an amyloid-associated disease. In the methods, the control subjects may be matched by age and/or sex. Preferably, the control subjects are matched by age and/or sex when compared to the subject. By "matched by age and/or sex", we include that the control subjects are selected so that they are all of the same or similar age and/or sex. How to select appropriate control subjects for the method of the invention would be known to one skilled in medicine.

By *"in vitro* control sample" we include that the control sample is prepared in a laboratory so that it has a known presence and/or amount and/or concentration and/or size of individual amyloid aggregates. How to produce such an *in vitro* control sample would be known to one skilled in chemistry, for example the production of peptide and protein monomers from which the individual amyloid aggregates could be formed may be undertaken using solid phase synthesis.

By "a negative control sample", we include that the control sample is removed from the bodily fluid sample, and the methods described herein are undertaken on the control sample but without the detection agent being added. For example, the bodily fluid sample is split into two (or more) volumes, with the first volume being the "sample of bodily fluid" and the having the detection agent added to it, and with the second volume being the "negative control sample" not having the detection agent added to it. This control sample therefore provides a background reading for the sample, under the experimental conditions of the method.

As discussed herein, a particularly preferred embodiment of the invention is determining the presence and/or amount and/or concentration and/or size of individual amyloid aggregates in the sample using time-resolved detection and fluorescence. Using this method, a control (or background) level of fluorescence can be determined in the same sample of bodily fluid which is subject of the methods described herein, and this is discussed further below.

In one aspect, the presence and/or amount and/or concentration of the individual amyloid aggregates in the sample is about 20% or more higher than the presence and/or amount and/or concentration and/or size of individual amyloid aggregates in a control sample; for example: about 25% or more higher; about 30% or more higher; about 35% or more higher; about 40% or more higher; about 45% or more higher; about 50% or more higher; about 55% or more higher; about 60% or more higher; about 65% or more higher; about 70% or more higher; about 75% or more higher; about 80% or more higher; about 85% or more higher; about 90% or more higher; about 95% or more higher, preferably about 50% or more higher; and/or

the size of the individual amyloid aggregates in the sample is about 20% or more larger than the size of individual amyloid aggregates in a control sample; for example: about 25% or more larger; about 30% or more larger; about 35% or more larger; about 40% or more larger; about 45% or more larger; about 50% or more larger; about 55% or more larger; about 60% or more larger; about 65% or more larger; about 70% or more larger; about 75% or more larger; about 80% or more larger; about 85% or more larger; about 90% or more larger; about 95% or more larger, preferably about 50% or more larger.

When we refer to the individual amyloid aggregates in the sample being "larger" than the size of the individual amyloid aggregates in a control sample, we include that the individual amyloid aggregates in the sample have more peptides *per* aggregate than the aggregates found in the control sample, and/or that they have a higher molecular weight.

In one aspect, the size of the individual amyloid aggregates in the sample is about 10 times or more of the molecular weight of the individual amyloid aggregates in the control sample; for example: about 11 times or more of the molecular weight; about 12 times or more of the molecular weight; about 13 times or more of the molecular weight; about 14 times or more of the molecular weight; about 15 times or more of the molecular weight; about 16 times or more of the molecular weight; about 17 times or more of the molecular weight; about 18 times or more of the molecular weight; about 19 times or more of the molecular weight; about 20 times or more of the molecular weight; about 21 times or more of the molecular weight; about 22 times or more of the molecular weight; about 23 times or more of the molecular weight; about 24 times or more of the molecular weight; about 25 times or more of the molecular weight; about 26 times or more of the molecular weight; about 27 times or more of the molecular weight; about 28 times or more of the molecular weight; about 29 times or more of the molecular weight; about 30 times or more of the molecular weight; about 35 times or more of the molecular weight; about 40 times or more of the molecular weight; about 45 times or more of the molecular weight; or about 50 times or more of the molecular weight the individual amyloid aggregates in the control sample, preferably about 27 times or more of the molecular weight the individual amyloid aggregates in the control sample.

In one aspect the presence and/or amount and/or concentration of the individual amyloid aggregates in the sample is about two times or more higher than in a control sample; for example, about three times or more higher; about four times or more higher; about five times or more higher; about six times or more higher; about seven times or more higher; about eight times or more higher; about nine times or more higher; about ten times or more higher than in a control sample, preferably about five times or more higher than in a control sample; and/or the size of the individual amyloid aggregates in the sample is about two times or more larger than in a control sample; for example, about three times or more larger; about four times or more larger; about five times or more larger; about six times or more larger; about seven times or more larger; about eight times or more larger; about nine times or more larger; about ten times or more larger than in a control sample, preferably about five times or more larger than in a control sample

In one aspect, the presence and/or amount and/or concentration of the individual amyloid aggregates in the sample is about two standard deviations or more higher than in a control sample; for example, about three standard deviations or more higher; about four standard deviations or more higher; about five standard deviations or more higher; about six standard deviations or more higher; about seven standard deviations or more higher; about eight standard deviations or more higher; about nine standard deviations or more higher; about ten standard deviations or more higher than in a control sample, preferably about five standard deviations or more higher than in a control sample; and/or the size of the individual amyloid aggregates in the sample is about two standard deviations or more larger than in a control sample; for example, about three standard deviations or more larger; about four standard deviations or more larger; about five standard deviations or more larger; about six standard deviations or more larger; about seven standard deviations or more larger; about eight standard deviations or more larger; about nine standard deviations or more larger; about ten standard deviations or more larger than in a control sample, preferably about five standard deviations or more larger than in a control sample.

In one aspect, the higher the amount and/or concentration of the individual amyloid aggregates in the sample, the more serious the diagnosis and/or the more serious the prognosis.

In one aspect, the larger the size of the individual amyloid aggregates in the sample, the more serious the diagnosis and/or the more serious the prognosis.

By "serious" in the context of the diagnosis and/or prognosis, we include that the likelihood that the onset and/or progression of the amyloid-associated disease will proceed quickly is high, and/or that the subject might soon exhibit one or more symptoms of an amyloid-associated disease, and/or that the subject will exhibit severe symptoms of the amyloid-associated disease, and/or that the subject might die soon.

In one aspect, the size of the individual amyloid aggregates is the mean size.

One skilled in biology and/or statistics would understand, and would be able to calculate, the mean size of the individual amyloid aggregates. By "mean size", we include that the mean is the average mean.

As would be appreciated by one skilled in the art, the type of bodily fluid sample used in the method of the invention might dictate, to some extent, the size of the individual amyloid aggregates that can be detected. For example, for urine as a bodily fluid sample the maximum size of the individual amyloid aggregates detectable might be defined by size limitations caused by glomerular filtration.

In the methods, the control sample may comprise a bodily fluid sample from one or more control subjects that do not have an amyloid-associated disease; for example: about two or more control subjects; about three or more control subjects; about four or more control subjects; about five or more control subjects; about six or more control subjects; about seven or more control subjects; about eight or more control subjects; about nine or more control subjects; about ten or more control subjects; about 11 or more control subjects; about 12 or more control subjects; about 13 or more control subjects; about 14 or more control subjects; about 15 or more control subjects; about 16 or more control subjects; about 17 or more control subjects; about 18 or more control subjects; about 19 or more control subjects; about 20 or more control subjects; about 25 or more control subjects; about 30 or more control subjects; about 35 or more control subjects; about 40 or more control subjects; about 45 or more control subjects; about 50 or more control subjects; about 60 or more control subjects; about 70 or more control subjects; about 80 or more control subjects; about 90 or more control subjects; about 100 or more control subjects, preferably bodily fluid samples from about 30 or more control subjects.

In one aspect, where there are two or more control subjects the control sample is the mean (preferably the average mean) of the presence and/or amount and/or concentration and/or size of individual amyloid aggregates in the two or more control subjects.

In one embodiment, where the measurement and/or detection of fluorescence is used to determine the presence and/or amount and/or concentration and/or size of individual amyloid aggregates in the sample, the control sample is "a control level of fluorescence in the sample" (for example, the "overall signal") and the fluorescence (i.e. the fluorescence signal) comprises a level of fluorescence higher than the control level of fluorescence in the sample. Put another way, the level of fluorescence being higher than the control level of fluorescence represents the presence and/or amount and/or concentration and/or size of the individual amyloid aggregates.

In some aspects, the "level of fluorescence" is the peak of fluorescence (i.e. the measured value of the fluorescence that is larger than five times the standard deviation of the signal), such as a peak of fluorescence which is detectable above the control level of fluorescence in the sample.

In one aspect, the method comprises the step of detecting and/or measuring the control level of fluorescence in the sample, such as detecting and/or measuring the fluorescence fluctuations. In one aspect, the step of "detecting and/or measuring the control level of fluorescence in the sample" precedes the step of "adding a detection agent to the sample".

In one aspect, the fluorescence comprises a level of fluorescence (such as a peak in fluorescence) about two times or more higher than a control level of fluorescence; for example, about three times or more; about four times or more; about five times or more; about six times or more; about seven times or more; about eight times or more; about nine times or more; about ten times or more higher than a control level of fluorescence (for example, the "overall signal"), preferably about five times or more higher than a control level of fluorescence (for example, the "overall signal").

In one aspect, the fluorescence comprises a level of fluorescence (such as a peak in fluorescence) about two standard deviations or more higher than a control level of fluorescence; for example, about three standard deviations or more; about four standard deviations or more; about five standard deviations or more; about six standard deviations or more; about seven standard deviations or more; about eight standard deviations or more; about nine standard deviations or more; about ten standard deviations or more higher than a control level of fluorescence (for example, the "overall signal"), preferably about five standard deviations or more higher than a control level of fluorescence (for example, the "overall signal").

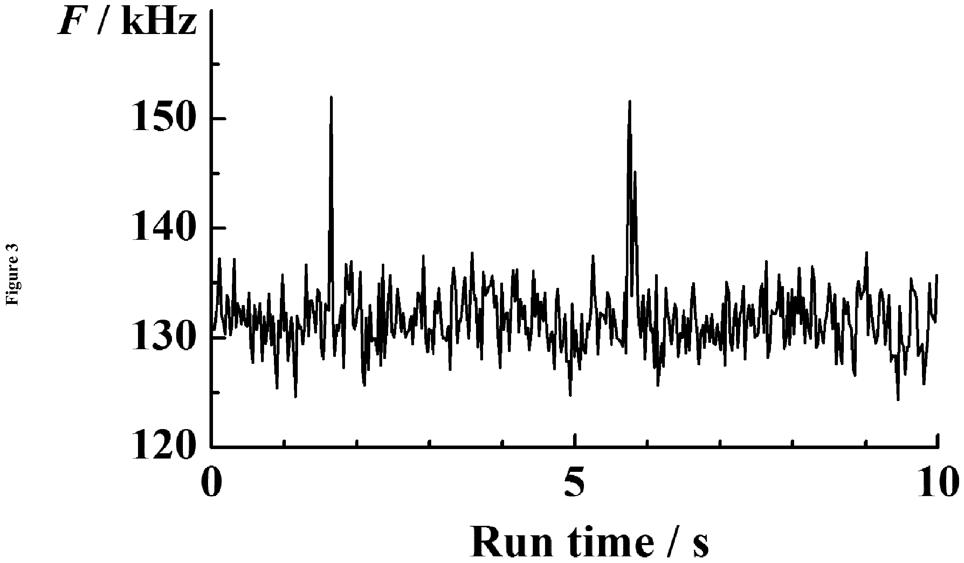

As discussed herein, the control level of fluorescence is the background level of fluorescence in the sample, which can be referred to as the variance of the background level of fluorescence or baseline signal (it can also be referred to as the overall signal). As would be understood by one skilled in biology, a sample of bodily fluid might have a certain level of auto fluorescence, which may differ between bodily fluid samples. An example of this is provided in Figure 3 of the application. The background level of fluorescence is the uneven line at approximately 132.5 kHz, with the peaks of fluorescence being above this at approximately 150 kHz. In this particular example, those peaks of fluorescence are representative of individual amyloid aggregates, and their signal is about higher than five times the standard deviation of the background level of fluorescence. It is sometimes necessary to take that background level of auto fluorescence into consideration when detecting and/or measuring fluorescence from a detection agent, with fluorescence detected and/or measured above that background level of fluorescence representing the presence and/or amount and/or concentration and/or size of individual amyloid aggregates in the sample.

In one aspect in which time-resolved detection is used, the control level of fluorescence in the sample is the baseline signal or variance of the background level of fluorescence of the time resolved detection. In this particular aspect, the baseline signal or variance of the background level of fluorescence can be time-resolved detection measurement(s) in a fraction(s) of the sample of bodily fluid which do not comprise any individual amyloid aggregates or the overall signal of the sample. The overall signal is the fluorescence measurements that are taken during the course of the time-resolved detection. Accordingly, the baseline signal or variance of the background level of fluorescence can be provided throughout the unit of time of the time-resolved detection.

In one aspect, each peak of fluorescence (such as a single event occurrence ($f_{SEO}$)) above a baseline signal or variance of the background level of fluorescence is indicative of an individual amyloid aggregate (preferably, the peak of fluorescence is at least five standard deviations higher than the overall signal). Put another way, each peak of fluorescence (such as a single event occurrence ($f_{SEO}$)) above a baseline signal or variance of the background level of fluorescence represents a frequency of one, and two peaks represents a frequency of two, and so on. In a particularly preferred aspect, the peak of fluorescence (such as the single event occurrence ($f_{SEO}$)) is a sharp and brief increase of the level of fluorescence above the baseline signal or variance of the background level of fluorescence (for example, the overall signal), which is indicative of an individual amyloid aggregate.

In one aspect, the control level of fluorescence (such as the baseline signal or variance of the background level of fluorescence) is the mean level of fluorescence measured throughout the unit of time of the time-resolved detection (such as Fluorescence Correlation Spectroscopy (FCS)), preferably the average mean.

In a preferred aspect, the fluorescence comprises a peak of fluorescence about two times or more higher than a baseline signal or variance of the background level of fluorescence; for example, about three times or more; about four times or more; about five times or more; about six times or more; about seven times or more; about eight times or more; about nine times or more; about ten times or more higher than a variance of the background level of fluorescence (for example, the overall signal), preferably about five times or more higher than a baseline signal or variance of the background level of fluorescence (for example, the overall signal).

In a preferred aspect, the fluorescence comprises a peak of fluorescence about two standard deviations or more higher than a baseline signal or variance of the background level of fluorescence; for example, about three standard deviations or more; about four standard deviations or more; about five standard deviations or more; about six standard deviations or more; about seven standard deviations or more; about eight standard deviations or more; about nine standard deviations or more; about ten standard deviations or more higher than a variance of the background level of fluorescence (for example, the overall signal), preferably about five standard deviations or more higher than a baseline signal or variance of the background level of fluorescence (for example, the overall signal).

In one aspect, the sample comprises a volume of about 30 microlitres ($\mu$1) to about 300 microlitres ($\mu$l).

In one aspect, the sample comprises a volume of about 500 microlitres (μl) or less; for example, about 490 microlitres (μl) or less; about 480 microlitres (μl) or less; about 470 microlitres (μl) or less; about 460 microlitres (μl) or less; about 450 microlitres (μl) or less; about 440 microlitres (μl) or less; about 430 microlitres (μl) or less; about 420 microlitres (μl) or less; about 410 microlitres (μl) or less; about 400 microlitres (μl) or less; about 390 microlitres (μl) or less; about 380 microlitres (μl) or less; about 370 microlitres (μl) or less; about 360 microlitres (μl) or less; about 350 microlitres (μl) or less; about 340 microlitres (μl) or less; about 330 microlitres (μl) or less; about 320 microlitres (μl) or less; about 310 microlitres (μl) or less; about 300 microlitres (μl) or less; about 290 microlitres (μl) or less; about 280 microlitres (μl) or less; about 270 microlitres (μl) or less; about 260 microlitres (μl) or less; about 250 microlitres (μl) or less; about 240 microlitres (μl) or less; about 230 microlitres (μl) or less; about 220 microlitres (μl) or less; about 210 microlitres (μl) or less; about 200 microlitres (μl) or less; about 190 microlitres (μl) or less; about 180 microlitres (μl) or less; about 170 microlitres (μl) or less; about 160 microlitres (μl) or less; about 150 microlitres (μl) or less; about 140 microlitres (μl) or less; about 130 microlitres (μl) or less; about 120 microlitres (μl) or less; about 110 microlitres (μl) or less; about 100 microlitres (μl) or less; about 90 microlitres (μl) or less; about 80 microlitres (μl) or less; about 70 microlitres (μl) or less; about 60 microlitres (μl) or less; about 50 microlitres (μl) or less; about 40 microlitres (μl) or less; about 30 microlitres (μl) or less; about 20 microlitres (μl) or less; or about 10 microlitres (μl) or less. In a preferred aspect, the sample comprises a volume of 150 microlitres (μl) or less. In an alternative preferred embodiment, the sample comprises a volume of 200 microlitres (μl) or less.

As the method is undertaken using time-resolved detection by Fluorescence Correlation Spectroscopy (FCS), the sample may be processed into fractions, such as observation volume elements (OVE), as explained in the Examples.

In one aspect, the fractions (such as observation volume element) comprises a volume of about 1 microlitre (μl) or less; for example, about 0.1 microlitre (μl) or less; about 0.01 microlitre (μl) or less; about 0.001 microlitre (μl) or less; about 0.0001 microlitre (μl) or less; about 0.00001 microlitre (μl) or less; about 0.000001 microlitre (μl) or less; about 0.0000001 microlitre (μl) or less; about 0.00000001 microlitre (μl) or less; about 1 femtolitre (fl) or less; about 0.1 femtolitre (fl) or less; about 0.2 femtolitre (fl) or less; about 0.3 femtolitre (fl) or less; about 0.4 femtolitre (fl) or less; about 0.5 femtolitre (fl) or less; about 0.6 femtolitre (fl) or less; about 0.7 femtolitre (fl) or less; about 0.8 femtolitre (fl) or less; about 0.9 femtolitre (fl) or less; about 0.01 femtolitre (fl) or less; about 0.001 femtolitre (fl) or less; or about 0.0001 femtolitre (fl) or less, preferably about 0.2 femtolitre (fl) or less. According to an embodiment the observation volume can be from about $10^{-20}$ litre down to about $10^{-22}$ litres, such as in in the zepto-litre range.

In one aspect, the method comprises the use of a flow cell to circulate the sample while determining the presence and/or amount and/or concentration of individual amyloid aggregates. A flow cell is a device which would be known to one skilled in microscopy. A flow cell can be used to circulate the sample through the device while doing time-resolved detection. In this way the frequency of encountering nano-plaques can be increased - instead of waiting for aggregates to freely diffuse, one is propelling their movement by the flow. This can be advantageous as it may expedite the measurements, thus shortening the analysis time. Exemplary flow cells suitable for the described method can be obtained from Warner Instruments - US (www.warneronline.com), such as the are RC-30HV and RC-30WA chambers.

In one aspect, a diagnosis and/or prognosis of the amyloid-associated disease is provided if the presence and/or amount of the individual amyloid aggregates is one individual amyloid aggregate or more; for example: about two individual amyloid aggregates or more; about three individual amyloid aggregates or more; about four individual amyloid aggregates or more; about five individual amyloid aggregates or more; about six individual amyloid aggregates or more; about seven individual amyloid aggregates or more; about eight individual amyloid aggregates or more; about nine individual amyloid aggregates or more; about 10 individual amyloid aggregates or more; about 15 individual amyloid aggregates or more; about 20 individual amyloid aggregates or more; about 25 individual amyloid aggregates or more; about 30 individual amyloid aggregates or more; about 50 individual amyloid aggregates or more; about 100 individual amyloid aggregates or more; about 200 individual amyloid aggregates or more; about 300 individual amyloid aggregates or more; about 400 individual amyloid aggregates or more; about 500 individual amyloid aggregates or more; about 1,000 individual amyloid aggregates or more; about 2,000 individual amyloid aggregates or more; about 3,000 individual amyloid aggregates or more; about 4,000 individual amyloid aggregates or more; about 5,000 individual amyloid aggregates or more; or about 10,000 individual amyloid aggregates or more.

In one aspect, the method is repeated at intervals, such as two times or more; for example, three times or more; four times or more; five times or more; six times or more; seven times or more; eight times or more; nine times or more; 10 times or more; 11 times or more; 12 times or more; 13 times or more; 14 times or more; 15 times or more; 16 times or more; 17 times or more; 18 times or more; 19 times or more; 20 times or more; 25 times or more; 30 times or more; 35 times or more; 40 times or more; 45 times or more; or times or more; or 50 times or more.

Repeating the method over time allows for the diagnosis and/or prognosis to be followed over time, which allows for the diagnosis and/or prognosis to be reviewed and revised. Accordingly, when the method is repeated two or more times, for the second or subsequent repeat the step of providing a diagnosis and/or prognosis of the amyloid-associated disease on the basis of the determination can be the step revising the diagnosis and/or the prognosis of the amyloid-associated disease on the basis of the determination.

In one aspect, the period between the method of the first aspect of the invention being repeated is one day or more; for example, about two days or more; about three days or more; about four days or more; about five days or more; about six days or more; about one week or more; about two weeks or more; about three weeks or more; about four weeks or more; about one month or more; about five weeks or more; about six weeks or more; about seven weeks or more; about eight weeks or more; about two months or more; about three months or more; about four months or more; about five months or more; about six months or more; about seven months or more; about eight months or more; about nine months or more; about 10 months or more; about 11 months or more; about 1 year or more; about two years or more; about 3 years or more; about 4 years or more; about 5 years or more; about 6 years or more; about 7 years or more; about 8 years or more; about nine years or more; or about ten years or more, preferably about one month or more.

In one aspect, the subject and/or the control subject is one or more from the group comprising: a rodent (for example, a mouse, and/or a rat, and/or a hamster, and/or a guinea pig, and/or a gerbil, and/or a rabbit); and/or a canine (for example, a dog); and/or a feline (for example, a cat); a primate (for example, a human; and/or a monkey; and/or an ape); and/or an equine (for example, a horse); and/or a bovine (for example, a cow); and/or a porcine (for example, a pig); and/or a non-human mammal. Preferably the subject is human, preferably both the subject and control subject are human.

In an embodiment, the subject and/or the control subject is alive. In a preferred embodiment, the subject is alive.

In one embodiment not part of the invention, the method further comprises the step of selecting and/or providing a treatment for the amyloid-associated disease to the subject.

By "selecting and/or providing a treatment", we include that based on the diagnosis and/or prognosis of the amyloid-associated disease an appropriate treatment for that disease can be identified. Based on the diagnosis and/or prognosis of the amyloid-associated disease, one skilled in medicine would be able to select and/or provide an appropriate treatment. Preferably, the step of "selecting and/or providing a treatment for the amyloid-associated disease to the subject" follows the step of "providing a diagnosis and/or prognosis of the amyloid-associated disease on the basis of the determination".

In one embodiment, the treatment is one that reduces the number and/or severity of the symptoms and/or signs of the amyloid-associated disease, and/or treats the cause of the amyloid-associated disease; and/or improves neuromodulator processes; and/or improves neurotransmission processes; and/or reduces the presence and/or amount and/or concentration and/or size of individual amyloid aggregates.

As disclosed herein, by treatment we include a palliative treatment, and/or an experimental treatment, and/or a physical therapy.

In one aspect, the treatment is one or more selected from the group comprising: Levodopa (L-DOPA); acetylcholinesterase inhibitors (AChEIs, such as Donepezil, Galantamine and Rivastigmine); N-methyl-D-aspartate (NMDA) inhibitors (such as Memantine); secretase 1 (BACE1) inhibitors; monoclonal antibodies (including monoclonal antibodies analogues); and/or Tafamidis. One skilled in medicine would be familiar with the treatments discussed herein, and would know when they should be selected and/or provided.

AChEIs inhibit the acetylcholinesterase enzyme from breaking down acetylcholine, thereby increasing both the level and duration of action of the neurotransmitter acetylcholine. The NMDA receptor antagonist Memantine, regulates the activity of glutamate, an important neurotransmitter in the brain involved in learning and memory, by partially blocking the NMDA receptors to which glutamate released from damaged cells is being released. Tafamidis (trade name Vyndaqel) stabilizes the correctly folded tetrameric form of transthyretin (TTR), thereby slowing down the rate at which TTR aggregates are being formed and accumulate in the diseased individual. Monoclonal antibodies are capable of crossing the blood-brain barrier and selectively target soluble amyloid β aggregates (in particular, insoluble fibrils in the brain), which reduces the amyloid β aggregate load. It was shown in preclinical studies, that a monoclonal antibody analogue could clear Aβ from plaque-bearing transgenic mouse brains (Sevigny et al. Nature 2016 537:50-56 doi:10.1038/nature19323).

In a preferred aspect, the amyloid-associated disease is Parkinson's disease and the treatment comprises L-DOPA.

In an alternative preferred aspect, the amyloid-associated disease is Familial Amyloid Polyneuropathy (FAP) and the treatment comprises Tafamidis.

In a further alternative preferred aspect, the amyloid-associated disease is Alzheimer's disease and the treatment comprises secretase 1 (BACE1) inhibitors and/or monoclonal antibodies (including monoclonal antibodies analogues).

In one aspect, the secretase 1 (BACE1) inhibitors exclude verubecestat.

In one embodiment not part of the invention, the treatment comprises administering a treatment for the amyloid-associated disease to the subject. In a preferred embodiment, the step of "administering a treatment for the amyloid-associated disease to the subject" follows the step of "selecting and/or providing a treatment for the amyloid-associated disease to the subject".

A second aspect of the invention provides a method for identifying an agent for the treatment of an amyloid-associated disease, wherein the method comprises the steps of:

- determining the presence and/or amount and/or concentration and/or size of individual amyloid aggregates in a first sample of bodily fluid that has been obtained from a test subject;
- determining the presence and/or amount and/or concentration and/or size of individual amyloid aggregates in a second sample of bodily fluid that has been obtained from the a test subject after having administered the agent to the subject;
- comparing the determination of the presence and/or amount and/or concentration and/or size of individual amyloid aggregates in the first sample with the determination of presence and/or amount and/or concentration and/or size of individual amyloid aggregates in the second sample; and,
- identifying the agent as being for the treatment of the amyloid-associated disease based on the comparison; wherein the amyloid-associated disease is associated with individual amyloid aggregates, the method further comprises the addition of a detection agent to the sample, the detection agent binding to and/or associating with and/or reacting with individual amyloid aggregates, the determination of the presence and/or amount and/or concentration and/or size of individual amyloid aggregates in the sample comprises an analysis based on time-resolved detection comprising fluorescence correlation spectroscopy (FCS) and fluorescence intensity fluctuation analysis (FIFA), and wherein the method does not comprise amplification of individual amyloid aggregates.

By "identifying an agent for the treatment of an amyloid-associated disease", we include determining whether a treatment that has not previously been identified is useful for treating an amyloid-associated disease, and/or is useful for reducing the number and/or severity of the symptoms and/or signs of the amyloid-associated disease, and/or is useful for improving neuromodulator processes; and/or is useful for improving neurotransmission processes, and/or is useful for reducing the presence and/or amount and/or concentration and/or size of individual amyloid aggregates. Alternatively, by "identifying an agent for the treatment of an amyloid-associated disease" we include determining the effectiveness of a treatment for the amyloid-associated disease on the subject. To put another way, we include following the effectiveness of a treatment for an amyloid-associated disease in a subject.

The second aspect of the invention relates to an *ex vivo* method of identifying an agent for the treatment of an amyloid-associated disease.

It will be appreciated that the features defined above in relation to the first aspect of the invention apply to the method of the second aspect of the invention. For example, the features discussed in relation to "providing a diagnosis and/or prognosis of the amyloid-associated disease on the basis of the determination" in the first aspect of the invention can be used for "identifying the agent as being for the treatment of the amyloid-associated disease based on the comparison".

In another aspect, not encompassed by the wording of the claims, the method comprises the steps in the order of:

- providing a first sample of bodily fluid from a test subject;
- determining the presence and/or amount and/or concentration and/or size of individual amyloid aggregates in the first sample;
- administering an agent to the subject;
- providing a second sample of bodily fluid from the test subject;
- determining the presence and/or amount and/or concentration and/or size of individual amyloid aggregates in the second sample;
- comparing the determination of the presence and/or amount and/or concentration and/or size of individual amyloid aggregates in the first sample with the determination of presence and/or amount and/or concentration and/or size of individual amyloid aggregates in the second sample; and,
- identifying the agent as being for the treatment of the amyloid-associated disease based on the comparison.

In one aspect, an agent is identified as being for the treatment of the amyloid-associated disease if the presence and/or amount and/or concentration of the individual amyloid aggregates in the second sample is lower than the presence and/or amount and/or concentration of the individual amyloid aggregates in the first sample; and/or the size of the individual amyloid aggregates in the second sample is smaller than the size of the individual amyloid aggregates in the first sample.

When we refer to the individual amyloid aggregates in the second sample being "smaller" than the size of the individual amyloid aggregates in a first sample, we include that the individual amyloid aggregates in the second sample have fewer peptides *per* aggregate than the aggregates found in the first sample, or have a lower molecular weight.

Without wishing to be bound by any theory, the inventors believe that if the test subject has fewer and/or the smaller individual amyloid aggregates after administration of the agent, then that agent is likely to have a role in treating the amyloid-associated disease. The bigger the reduction in number and/or size of the individual amyloid aggregates the more effective the agent is likely to be in treating the amyloid-associated disease.

In one aspect, the presence and/or amount and/or concentration of the individual amyloid aggregates in the second sample is about 20% or more lower than the presence and/or amount and/or concentration and/or size of individual amyloid aggregates in a first sample; for example: about 25% or more lower; about 30% or more lower; about 35% or more lower; about 40% or more lower; about 45% or more lower; about 50% or more lower; about 55% or more lower; about 60% or more lower; about 65% or more lower; about 70% or more lower; about 75% or more lower; about 80% or more lower; about 85% or more lower; about 90% or more lower; about 95% or more lower, preferably about 50% or more lower; and/or

the size of the individual amyloid aggregates in the second sample is about 20% or more smaller than the size of individual amyloid aggregates in a first sample; for example: about 25% or more smaller; about 30% or more smaller; about 35% or more smaller; about 40% or more smaller; about 45% or more smaller; about 50% or more smaller; about 55% or more smaller; about 60% or more smaller; about 65% or more smaller; about 70% or more smaller; about 75% or more smaller; about 80% or more smaller; about 85% or more smaller; about 90% or more smaller; about 95% or more smaller, preferably about 50% or more smaller.

In one aspect, the size of the individual amyloid aggregates in the first sample is about 10 times or more of the molecular weight of the individual amyloid aggregates in the second sample; for example: about 11 times or more of the molecular weight; about 12 times or more of the molecular weight; about 13 times or more of the molecular weight; about 14 times or more of the molecular weight; about 15 times or more of the molecular weight; about 16 times or more of the molecular weight; about 17 times or more of the molecular weight; about 18 times or more of the molecular weight; about 19 times or more of the molecular weight; about 20 times or more of the molecular weight; about 21 times or more of the molecular weight; about 22 times or more of the molecular weight; about 23 times or more of the molecular weight; about 24 times or more of the molecular weight; about 25 times or more of the molecular weight; about 26 times or more of the molecular weight; about 27 times or more of the molecular weight; about 28 times or more of the molecular weight; about 29 times or more of the molecular weight; about 30 times or more of the molecular weight; about 35 times or more of the molecular weight; about 40 times or more of the molecular weight; about 45 times or more of the molecular weight; or about 50 times or more of the molecular weight the individual amyloid aggregates in the second sample, preferably about 27 times or more of the molecular weight the individual amyloid aggregates in the second sample.

In one aspect, the presence and/or amount and/or concentration of the individual amyloid aggregates in the first sample is about two times or more higher than in the second sample; for example, about three times or more higher; about four times or more higher; about five times or more higher; about six times or more higher; about seven times or more higher; about eight times or more higher; about nine times or more higher; about ten times or more higher than in a first sample, preferably about five times or more higher than in the second sample; and/or the size of the individual amyloid aggregates in the first sample is about two times or more larger than in the second sample; for example, about three times or more larger; about four times or more larger; about five times or more larger; about six times or more larger; about seven times or more larger; about eight times or more larger; about nine times or more larger; about ten times or more larger than in a first sample, preferably about five times or more larger than in the second sample

In one aspect, the presence and/or amount and/or concentration of the individual amyloid aggregates in the first sample is about two standard deviations or more higher than in the second sample; for example, about three standard deviations or more higher; about four standard deviations or more higher; about five standard deviations or more higher; about six standard deviations or more higher; about seven standard deviations or more higher; about eight standard deviations or more higher; about nine standard deviations or more higher; about ten standard deviations or more higher than in a first sample, preferably about five standard deviations or more higher than in the second sample; and/or the size of the individual amyloid aggregates in the first sample is about two standard deviations or more larger than in the second sample; for example, about three standard deviations or more larger; about four standard deviations or more larger; about five standard deviations or more larger; about six standard deviations or more larger; about seven standard deviations or more larger; about eight standard deviations or more larger; about nine standard deviations or more larger; about ten standard deviations or more larger than in a first sample, preferably about five standard deviations or more larger than in the second sample.

Similarly as discussed in relation to the first aspect, in one aspect "determining the presence and/or amount and/or concentration and/or size of individual amyloid aggregates in the first sample" and/or "determining the presence and/or amount and/or concentration and/or size of individual amyloid aggregates in the second sample" comprises determining the frequency of individual amyloid aggregates (such as single event occurrence ($f_{SEO}$)) in a unit of time. In a preferred aspect, the frequency of individual amyloid aggregates in the first sample and the second sample is compared, and the agent is identified as being for the treatment of the amyloid-associated disease if the frequency of individual amyloid aggregates in the second sample is lower than the frequency of individual amyloid aggregates in the first sample.

In one aspect, not encompassed by the wording of the claims, the following steps are repeated at intervals:

- providing a first sample of bodily fluid from a test subject; and
- determining the presence and/or amount and/or concentration and/or size of individual amyloid aggregates in the first sample,

optionally, wherein the steps are repeated two times or more; for example: three times or more; four times or more; five times or more; six times or more; seven times or more; eight times or more; nine times or more; 10 times or more; 11 times or more; 12 times or more; 13 times or more; 14 times or more; 15 times or more; 16 times or more; 17 times or more; 18 times or more; 19 times or more; 20 times or more; 25 times or more; 30 times or more; 35 times or more; 40 times or more; 45 times or more; or times or more; or 50 times or more.

In one aspect, where the steps of:

- providing a first sample of bodily fluid from a test subject; and
- determining the presence and/or amount and/or concentration and/or size of individual amyloid aggregates in the first sample,

have been repeated at intervals, the first sample in the comparison step of the second aspect of the invention is the mean (preferably the average mean) of the presence and/or amount and/or concentration and/or size of individual amyloid aggregates in the two or more first samples.

In one aspect, not encompassed by the wording of the claims, where the steps of:

- providing a first sample of bodily fluid from a test subject; and
- determining the presence and/or amount and/or concentration and/or size of individual amyloid aggregates in the first sample,

have been repeated at intervals, the period between the steps being repeated is one hour or more; for example, about two hours or more; about three hours or more; about four hours or more; about five hours or more; about six hours or more; about seven hours or more; about eight hours or more; about nine hours or more; about 10 hours or more; about 11 hours or more; about 12 hours or more; about 13 hours or more; about 14 hours or more; about 15 hours or more; about 16 hours or more; about 17 hours or more; about 18 hours or more; about 19 hours or more; about 20 hours or more; about 21 hours or more; about 22 hours or more; about 23 hours or more; about one day or more; about two days or more; about three days or more; about four days or more; about five days or more; about six days or more; about one week or more; about two weeks or more; about three weeks or more; about four weeks or more; about one month or more; about two months or more; about three months or more; about four months or more; about five months or more; about six months or more; about seven months of more; about eight months of more; about nine months or more; about 10 months or more; about 11 months or more; or about 12 months or more.

In one aspect, not encompassed by the wording of the claims, the following steps are repeated at intervals:

- providing a second sample of bodily fluid from the test subject;
- determining the presence and/or amount and/or concentration and/or size of individual amyloid aggregates in the second sample; and
- comparing the determination of the presence and/or amount and/or concentration and/or size of individual amyloid aggregates in the first sample with the determination of presence and/or amount and/or concentration and/or size of individual amyloid aggregates in the second sample;

optionally, wherein the steps are repeated two times or more; for example: three times or more; four times or more; five times or more; six times or more; seven times or more; eight times or more; nine times or more; 10 times or more; 11 times or more; 12 times or more; 13 times or more; 14 times or more; 15 times or more; 16 times or more; 17 times or more; 18 times or more; 19 times or more; 20 times or more; 25 times or more; 30 times or more; 35 times or more; 40 times or more; 45 times or more; or times or more; or 50 times or more.

In one aspect, not encompassed by the wording of the claims, where the steps of:

- providing a second sample of bodily fluid from the test subject;
- determining the presence and/or amount and/or concentration and/or size of individual amyloid aggregates in the second sample; and
- comparing the determination of the presence and/or amount and/or concentration and/or size of individual amyloid aggregates in the first sample with the determination of presence and/or amount and/or concentration and/or size of individual amyloid aggregates in the second sample;

have been repeated at intervals, the second sample in the comparison step of the second aspect of the invention is the mean (preferably the average mean) of the presence and/or amount and/or concentration and/or size of individual amyloid aggregates in the two or more second samples.

In one aspect, not encompassed by the wording of the claims, where the steps of:

- providing a second sample of bodily fluid from the test subject;
- determining the presence and/or amount and/or concentration and/or size of individual amyloid aggregates in the second sample; and
- comparing the determination of the presence and/or amount and/or concentration and/or size of individual amyloid aggregates in the first sample with the determination of presence and/or amount and/or concentration and/or size of individual amyloid aggregates in the second sample;

have been repeated at intervals, the period between the steps being repeated is one hour or more; for example, about two hours or more; about three hours or more; about four hours or more; about five hours or more; about six hours or more; about seven hours or more; about eight hours or more; about nine hours or more; about 10 hours or more; about 11 hours or more; about 12 hours or more; about 13 hours or more; about 14 hours or more; about 15 hours or more; about 16 hours or more; about 17 hours or more; about 18 hours or more; about 19 hours or more; about 20 hours or more; about 21 hours or more; about 22 hours or more; about 23 hours or more; about one day or more; about two days or more; about three days or more; about four days or more; about five days or more; about six days or more; about one week or more; about two weeks or more; about three weeks or more; about four weeks or more; about one month or more; about two months or more; about three months or more; about four months or more; about five months or more; about six months or more; about seven months of more; about eight months of more; about nine months or more; about 10 months or more; about 11 months or more; or about 12 months or more.

In a preferred aspect, not encompassed by the wording of the claims, where the steps of:

- providing a second sample of bodily fluid from the test subject;
- determining the presence and/or amount and/or concentration and/or size of individual amyloid aggregates in the second sample; and
- comparing the determination of the presence and/or amount and/or concentration and/or size of individual amyloid aggregates in the first sample with the determination of presence and/or amount and/or concentration and/or size of individual amyloid aggregates in the second sample;

have been repeated at intervals, the first provision of second sample from the test subject immediately follows the step of administering the agent to the test subject.

In one aspect, not encompassed by the wording of the claims, the test subject is one or more from the group comprising: a rodent (for example, a mouse, and/or a rat, and/or a hamster, and/or a guinea pig, and/or a gerbil, and/or a rabbit); and/or a canine (for example, a dog); and/or a feline (for example, a cat); a primate (for example, a human; and/or a monkey; and/or an ape); and/or an equine (for example, a horse); and/or a bovine (for example, a cow); and/or a porcine (for example, a pig); and/or a fish (for example, a zebrafish); and/or an insect (for example, a *Drosophila melanogaster*); and/or a amphibian (for example, *Xenopus laevis*); and/or a reptile; and/or a nematode (for example, *Caenorhabditis elegans*).

In one aspect, the test subject is a non-human, for example a non-human mammal. In a preferred embodiment, the non-human mammal is one or more from the group comprising: a rodent (for example, a mouse, and/or a rat, and/or a hamster, and/or a guinea pig, and/or a gerbil, and/or a rabbit); and/or a canine (for example, a dog); and/or a feline (for example, a cat); a non-human primate (for example, a monkey; and/or an ape); and/or an equine (for example, a horse); and/or a bovine (for example, a cow); and/or a porcine (for example, a pig).

A third aspect of the invention provides a method for identifying an agent for the treatment of an amyloid-associated disease, wherein the method comprises the steps of:

- determining the presence and/or amount and/or concentration and/or size of individual amyloid aggregates in a first sample that has been taken from a test fluid;
- determining the presence and/or amount and/or concentration and/or size of individual amyloid aggregates in a second sample that has been taken from the a test fluid after having administered the agent to the fluid;
- comparing the determination of the presence and/or amount and/or concentration and/or size of individual amyloid aggregates in the first sample with the determination of presence and/or amount and/or concentration and/or size of individual amyloid aggregates in the second sample; and,
- identifying the agent as being for the treatment of the amyloid-associated disease based on the comparison; wherein the amyloid-associated disease is associated with individual amyloid aggregates, the method further comprises the addition of a detection agent to the sample, the detection agent binding to and/or associating with and/or reacting with individual amyloid aggregates, the determination of the presence and/or amount and/or concentration and/or size of individual amyloid aggregates in the sample comprises an analysis based on fluorescence correlation spectroscopy (FCS) and fluorescence intensity fluctuation analysis (FIFA), and wherein the method does not comprise amplification of individual amyloid aggregate.

The second aspect of the invention relates to an *in vitro* and/or *ex vivo* method of identifying an agent for the treatment of an amyloid-associated disease.

It will be appreciated that the features defined above in relation to the first aspect and/or second aspect of the invention apply to the method of the third aspect of the invention. For example, the features discussed in relation to "providing a diagnosis and/or prognosis of the amyloid-associated disease on the basis of the determination" in the first aspect of the invention can be used for "identifying the agent as being for the treatment of the amyloid-associated disease based on the comparison". Similarly, the features discussed in relation to "determining the presence and/or amount and/or concentration and/or size of individual amyloid aggregates in the first sample" and/or "determining the presence and/or amount and/or concentration and/or size of individual amyloid aggregates in the second sample" in the second aspect of the invention can also be used for the third aspect of the invention.

In a preferred third aspect, the method comprises the steps in the order of:

- providing a first sample from a test fluid;
- determining the presence and/or amount and/or concentration and/or size of individual amyloid aggregates in the first sample;
- administering an agent to the test fluid;
- providing a second sample from the test fluid;
- determining the presence and/or amount and/or concentration and/or size of individual amyloid aggregates in the second sample;
- comparing the determination of the presence and/or amount and/or concentration and/or size of individual amyloid aggregates in the first sample with the determination of presence and/or amount and/or concentration and/or size of individual amyloid aggregates in the second sample; and,
- identifying the agent as being for the treatment of the amyloid-associated disease based on the comparison.

Without wishing to be bound by any theory, the inventors believe that if the test fluid has fewer and/or the smaller individual amyloid aggregates after administration of the agent, then that agent is likely to have a role in treating the amyloid-associated disease. The bigger the reduction in number and/or size of the individual amyloid aggregates the more effective the agent is likely to be in treating the amyloid-associated disease.

In one aspect, not encompassed by the wording of the claims, the following steps are repeated at intervals:

- providing a first sample from a test fluid; and
- determining the presence and/or amount and/or concentration and/or size of individual amyloid aggregates in the first sample;

optionally, wherein the steps are repeated two times or more; for example: three times or more; four times or more; five times or more; six times or more; seven times or more; eight times or more; nine times or more; 10 times or more; 11 times or more; 12 times or more; 13 times or more; 14 times or more; 15 times or more; 16 times or more; 17 times or more; 18 times or more; 19 times or more; 20 times or more; 25 times or more; 30 times or more; 35 times or more; 40 times or more; 45 times or more; or times or more; or 50 times or more.

In one aspect, not encompassed by the wording of the claims, where the steps of:

- providing a first sample from a test fluid; and
- determining the presence and/or amount and/or concentration and/or size of individual amyloid aggregates in the first sample;

have been repeated at intervals, the first sample in the comparison step of the third aspect of the invention is the mean (preferably the average mean) of the presence and/or amount and/or concentration and/or size of individual amyloid aggregates in the two or more first samples.

In one aspect, not encompassed by the wording of the claims, where the steps of:

- providing a first sample from a test fluid; and
- determining the presence and/or amount and/or concentration and/or size of individual amyloid aggregates in the first sample;

have been repeated at intervals, the period between the steps being repeated is one hour or more; for example, about two hours or more; about three hours or more; about four hours or more; about five hours or more; about six hours or more; about seven hours or more; about eight hours or more; about nine hours or more; about 10 hours or more; about 11 hours or more; about 12 hours or more; about 13 hours or more; about 14 hours or more; about 15 hours or more; about 16 hours or more; about 17 hours or more; about 18 hours or more; about 19 hours or more; about 20 hours or more; about 21 hours or more; about 22 hours or more; about 23 hours or more; about one day or more; about two days or more; about three days or more; about four days or more; about five days or more; about six days or more; about one week or more; about two weeks or more; about three weeks or more; about four weeks or more; about one month or more; about two months or more; about three months or more; about four months or more; about five months or more; about six months or more; about seven months of more; about eight months of more; about nine months or more; about 10 months or more; about 11 months or more; or about 12 months or more.

In one aspect, not encompassed by the wording of the claims, the following steps are repeated at intervals:

- providing a second sample from the test fluid;
- determining the presence and/or amount and/or concentration and/or size of individual amyloid aggregates in the second sample; and
- comparing the determination of the presence and/or amount and/or concentration and/or size of individual amyloid aggregates in the first sample with the determination of presence and/or amount and/or concentration and/or size of individual amyloid aggregates in the second sample;

optionally, wherein the steps are repeated two times or more; for example: three times or more; four times or more; five times or more; six times or more; seven times or more; eight times or more; nine times or more; 10 times or more; 11 times or more; 12 times or more; 13 times or more; 14 times or more; 15 times or more; 16 times or more; 17 times or more; 18 times or more; 19 times or more; 20 times or more; 25 times or more; 30 times or more; 35 times or more; 40 times or more; 45 times or more; or times or more; or 50 times or more.

In one aspect, not encompassed by the wording of the claims, where the steps of:

- providing a second sample from the test fluid;
- determining the presence and/or amount and/or concentration and/or size of individual amyloid aggregates in the second sample; and
- comparing the determination of the presence and/or amount and/or concentration and/or size of individual amyloid aggregates in the first sample with the determination of presence and/or amount and/or concentration and/or size of individual amyloid aggregates in the second sample;

have been repeated at intervals, the second sample in the comparison step of the second aspect of the invention is the mean (preferably the average mean) of the presence and/or amount and/or concentration and/or size of individual amyloid aggregates in the two or more samples.

In one aspect, not encompassed by the wording of the claims, where the steps of:

- providing a second sample from the test fluid;
- determining the presence and/or amount and/or concentration and/or size of individual amyloid aggregates in the second sample; and
- comparing the determination of the presence and/or amount and/or concentration amyloid of individual amyloid aggregates in the first sample with the determination of presence and/or amount and/or concentration and/or size of individual amyloid aggregates in the second sample;

have been repeated at intervals, the period between the steps being repeated is one hour or more; for example, about two hours or more; about three hours or more; about four hours or more; about five hours or more; about six hours or more; about seven hours or more; about eight hours or more; about nine hours or more; about 10 hours or more; about 11 hours or more; about 12 hours or more; about 13 hours or more; about 14 hours or more; about 15 hours or more; about 16 hours or more; about 17 hours or more; about 18 hours or more; about 19 hours or more; about 20 hours or more; about 21 hours or more; about 22 hours or more; about 23 hours or more; about one day or more; about two days or more; about three days or more; about four days or more; about five days or more; about six days or more; about one week or more; about two weeks or more; about three weeks or more; about four weeks or more; about one month or more; about two months or more; about three months or more; about four months or more; about five months or more; about six months or more; about seven months of more; about eight months of more; about nine months or more; about 10 months or more; about 11 months or more; or about 12 months or more.

In a preferred aspect, not encompassed by the wording of the claims, where the steps of:

- providing a second sample from the test fluid;
- determining the presence and/or amount and/or concentration and/or size of individual amyloid aggregates in the second sample; and
- comparing the determination of the presence and/or amount and/or concentration and/or size of individual amyloid aggregates in the first sample with the determination of presence and/or amount and/or concentration and/or size of individual amyloid aggregates in the second sample;

have been repeated at intervals, the first provision of the second sample from the test fluid immediately follows the step of administering the agent to the test fluid.

In one embodiment, the method is performed *in vitro* and/or *ex vivo.*

In one embodiment, the test fluid is a bodily fluid.

In a preferred aspect, the test fluid is one or more bodily fluid from the group comprising or consisting of: blood and/or cerebrospinal fluid (CSF) and/or saliva and/or synovial fluid and/or faeces and/or urine, preferably blood.

In an alternative aspect, the test fluid is an *in vitro* test fluid.

By *"in vitro* test fluid", we include that the test fluid was prepared in a laboratory, and that the *in vitro* test fluid has a known presence and/or amount and/or concentration and/or size of individual amyloid aggregates.

Another aspect, not encompassed by the wording of the claims, is a kit of parts for the method of the first aspect and/or the second aspect and/or the third aspect, for example using a method of the invention as defined herein. The kit of parts comprises one or more from the list comprising or consisting of:

- a detection agent as described herein, preferably Thioflavin T (ThT);
- a means for time-resolved detection, such as a microscope for Fluorescence Correlation Spectroscopy (FCS); and/or
- a control sample.

The kit of parts comprises instructions. The instructions can be descriptive, instructional, marketing or other material that relates to the uses, and/or methods, and/or compositions described herein.

The informational material of the kits can be provided in a variety of formats, include printed text, computer readable material, video recording, or audio recording, or information that provides a link or address to substantive material, e.g., on the internet.

The kit of parts comprises instructions for the diagnosis and/or prognosis of an amyloid-associated disease in a subject and/or for identifying an agent for the treatment of an amyloid-associated disease.

The present invention will now be described with reference to one or more figures and examples.

The listing or discussion of an apparently prior-published document in this specification should not necessarily be taken as an acknowledgement that the document is part of the state of the art or is common general knowledge.

**Figure legends**

**Figure 1. Schematic presentation of the instrumental setup for Fluorescence Correlation Spectroscopy (FCS). A.** Schematic drawing of the optical arrangement in an inverted confocal microscope. Incident laser light (dark grey) is reflected by the main dichroic beam splitter and sharply focused by the objective into the sample, generating a double-conus-like illumination volume. Molecules in the illumination volume that can absorb the incident laser light become excited, but unbound ThT molecules (light grey dots) and small amyloidogenic oligomers and peptide/protein monomers (dark grey dots) do not emit fluorescence, whereas the ThT-reactive structured amyloidogenic oligomers do (stars). The elastically scattered incident light (dark grey) and the spectrally distinct fluorescence (light grey) are collected back by the objective, and separated by the main dichroic beam splitter that reflects the elastically scattered light and allows the fluorescence light, which is of longer wavelength than the incident/elastically scattered laser light, to pass through the pinhole to the detector. **B.** Magnified image of the double-cones-like illumination volume generated in the sample by focusing the incident laser light with a microscope objective (dark grey) and the idealized observation volume element (OVE) in the form of a prolate ellipsoid from which fluorescence is being detected (light grey). **C.** Photons emitted by fluorescent molecules/particles passing through the OVE are detected by an avalanche photodiode (APD) detector, which responds with an electrical pulse to each detected photon. The number of electrical pulses originating from photons detected during a specific time interval, so-called binning time, corresponds to the measured light intensity at a given point of time. Exemplified here is a fluorescence intensity fluctuations time series recorded in a diluted suspension of quantum dots in blood serum using the same optical setting as described above. **D.** The electrical signal is transferred to a digital signal correlation unit and the corresponding normalized autocorrelation function G($\tau$) is calculated on-line to yield an experimentally derived temporal autocorrelation curve (tACC). The tACC shown here is derived from the time trace shown in C. *Inset:* Thioflavin T (ThT) chemical formula (top). *Bottom:* Fluorescence emission spectrum of ThT in water (dotted black line) and in an aqueous solution containing structured amyloidogenic oligomers of insulin (solid grey line). Normalized ground state absorption spectra of ThT in water (dotted black line) and in an aqueous solution of insulin amyloid fibrils (dashed grey line) are shown in the small inset. Image reproduced from Singh *et al.* 2015 with permission by the Royal Society of Chemistry.

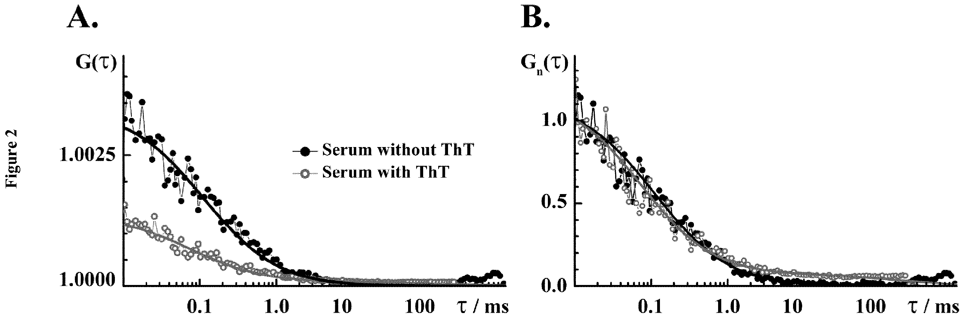

**Figure 2. FCS analysis of serum autofluorescence and serum ThT fluorescence. A.** Experimental ACC recorded in the blood serum without ThT (black dots) and with ThT (grey circles), and the corresponding best fit theoretical AF (solid lines). For measurements in the serum without ThT, the simplest theoretical model that could fit the experimental data was AF for free 3D diffusion without triplet. The parameter values derived by fitting are: $N$ = 300 and $\tau_D$ = 55. The structure parameter was fixed at the value determined in calibration experiments, $S_p$ = 5. For measurements in the serum with ThT, the simplest theoretical model that could fit the experimental data was AF for free 3D diffusion of two components without triplet. The parameter values derived by fitting are: $N$ = 750, $\tau_{D1}$ = 40 $\mu$s, $\tau_{D2}$ = 100 ms, $y_1$ = 0.94 and $y_2$ = 0.06. The structure parameter was fixed at the value determined in calibration experiments, $S_p$ = 5. **B.** Temporal autocorrelation curves shown in A. normalized to the same amplitude, G($\tau$) = 1 at $\tau$ = 10 $\mu$s.

**Figure 3. Time-resolved fluorescence intensity fluctuations in serum ThT recorded over time.** The clearly visible peaks in fluorescence intensity reflect the rare passage of bright ThT-reactive structured amyloidogenic oligomers, *i.e.* nano-plaques.

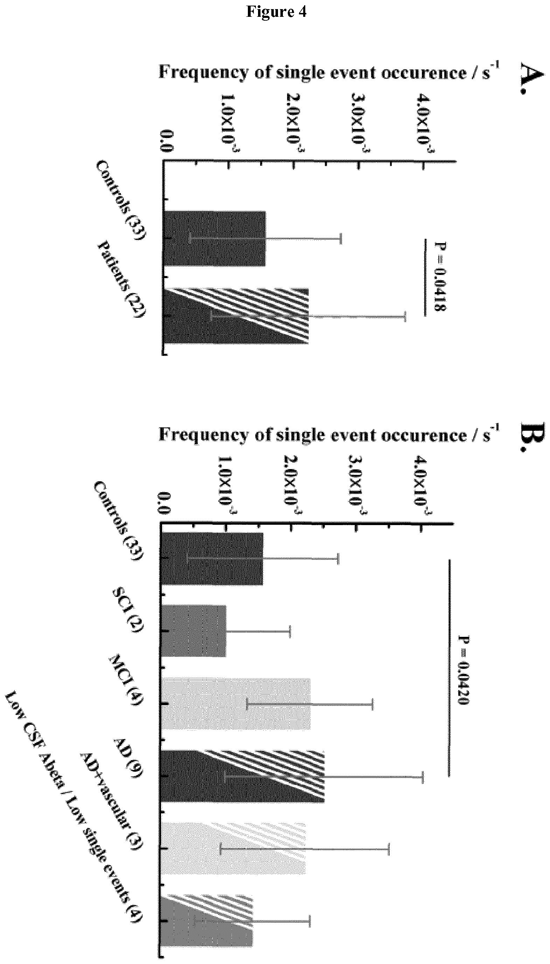

**Figure 4. The frequency of ThT-reactive structured amyloidogenic oligomeric aggregates occurrence in the patient and in the control group. A.** Mean frequency of single event occurrence in the patient group (dark striped grey) and the control group (dark grey). Error bars indicate standard deviations ($\pm$ SD). **B.** Mean frequency of single event occurrence in different diagnostic groups.

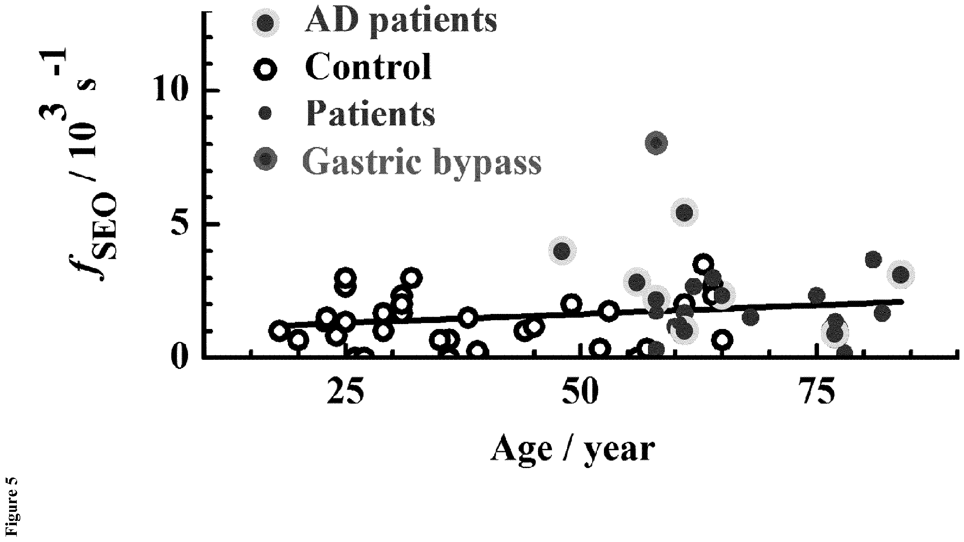

**Figure 5. The concentration of ThT-reactive structured amyloidogenic nano-plaques in blood serum increases with aging.** The frequency of single event occurrence as a function of age in apparently healthy controls (black circles) and patients (grey dots). Data from AD patients are light grey-lined. The dark grey-lined point originates from a patient that had gastric bypass. The slope of the linear regression equation is (1.6 $\pm$ 1.0)$\times 10^{-5}$ s$^{-1}$ year$^{-1}$ and the intercept (9.4 $\pm$ 0.6)$\times 10^{-4}$ s$^{-1}$. The adjusted R-squared value, $R_{adj}^2 = 0.032$ , indicates that this correlation is very weak.

**Figure 6. The translational diffusion time, *i.e.* the size of ThT-reactive structured amyloidogenic oligomeric aggregates in the patient and in the control group.** Temporal autocorrelation curves (tACCs) for the control (black dots) and the patient (grey circles) groups showing that the characteristic decay time is longer for the patient group, indicating that the structured aggregates observed in this group are larger.

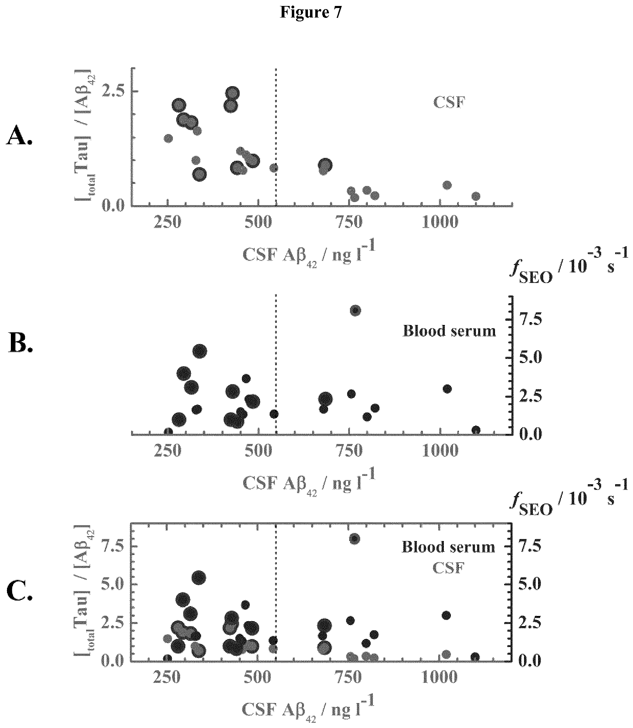

**Figure 7. Correlation between clinical CSF parameters and the frequency of single event occurrence in blood serum. A.** The ratio of the total Tau level over A$\beta_{42}$ level in the CSF as a function of the A$\beta_{42}$ level in the CSF. Light grey dots with a dark grey rim designate data from individuals diagnosed with AD. In all graphs, the dashed line indicates the generally accepted limit for an AD diagnosis based on A$\beta_{42}$ level in CSF, [A$\beta_{42}$] < 550 ng l$^{-1}$. **B.** The frequency of single event occurrence in the blood serum as a function of the A$\beta_{42}$ level in the CSF. Large dots designate data from individuals diagnosed with AD, whereas small dots indicate data from individuals in the patient cohort that were not diagnosed with AD. **C.** Comparison between CSF and blood serum measurements. Light grey axes and symbols relate to measurements in the CSF, showing the ratio of the total Tau level over A$\beta_{42}$ level in the CSF as a function of the A$\beta_{42}$ level in the CSF. Dark grey ordinate and symbols relate to measurements in the blood serum. Dark grey symbols present the frequency of single event occurrence in the blood serum as a function of the A$\beta_{42}$ level in the CSF. Data from individuals diagnosed with AD based on a multimodal clinical assessment are represented by the large dots. The light grey-lined point indicates the value from a patient with gastric bypass.

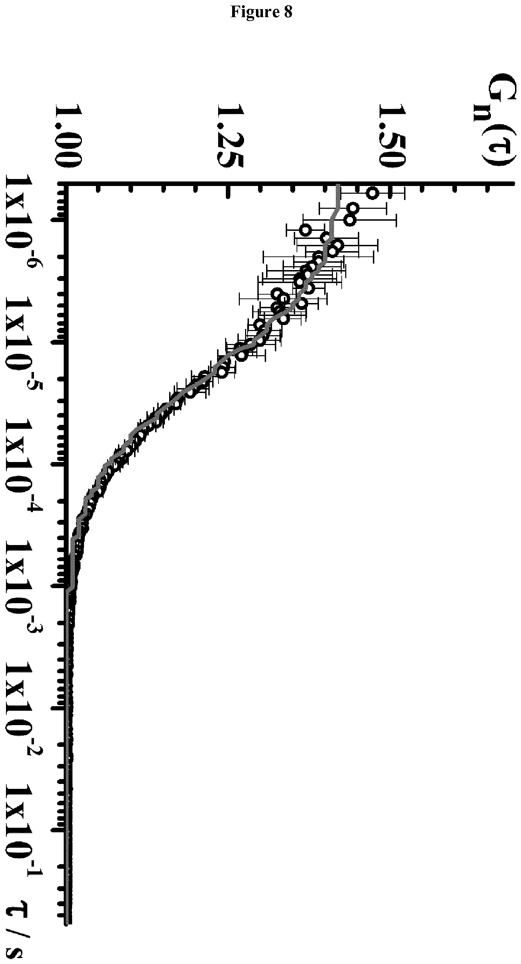

**Figure S1 (Figure 8). Instrument calibration.** Temporal autocorrelation curve recorded in a 25 nM water solution of Rh6G (black circles) using the same optical setting as for the ThT-assay. Autocorrelation function for free three dimensional (3D) diffusion (light grey), yielded by fitting the average number of molecules in the OVE, N = 2.5; translational diffusion time, $\tau$ = 24 $\mu$s; and structural parameter $S_p$ = 7. Under these conditions, the average counts per second per molecule and second for Rh6G was CPSM = (5.1 $\pm$ 0.6) kHz.

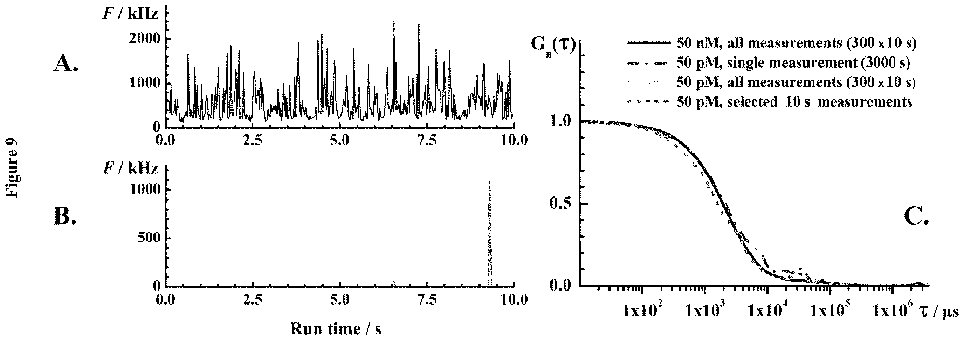

**Figure S2 (Figure 9). Fluospheres diffusion time determined from the analysis frequency of single of events occurrence agrees within the experimental error with the value obtained by temporal autocorrelation analysis. A.** Fluorescence intensity fluctuations recorded in a 50 nM aqueous suspension of fluospheres. **B.** Fluorescence intensity fluctuations recorded in a 50 pM aqueous suspension of fluospheres. **C.** tACCs normalized to the same amplitude, $G_n(\tau)$ = 1 at $\tau$ = 10 $\mu$s, obtained by analysing fluorescence intensity fluctuations recorded in 50 nM or 50 pM aqueous suspension of 100 nm fluospheres. *Solid line:* Average normalized tACC obtained from a series of 10 consecutively acquired measurements of fluorescence intensity fluctuations, with each individual measurement lasting 10 s, recorded in a 50 nM aqueous suspension of 100 nm fluospheres. *Dash-dot line:* Normalized tACC obtained by temporal autocorrelation analysis of fluorescence intensity fluctuations recorded for 3000 s in a 50 pM aqueous suspension of 100 nm fluospheres. *Dotted line:* Average normalized tACC obtained from 300 consecutively acquired measurements of fluorescence intensity fluctuations, with each individual measurement lasting 10 s, recorded in a 50 pM aqueous suspension of 100 nm fluospheres. *Dashed line:* Average normalized tACC obtained by analysing fluorescence intensity fluctuations, with each individual measurement lasting 10 s, recorded in a 50 pM aqueous suspension of 100 nm fluospheres where single events occurrence was identified as described in *Data analysis.* The dashed-dotted, dotted and dashed tACCs were obtained from measurements performed in the same sample, but using different data analysis procedures. The overlap of the normalized tACCs, indicates that all procedures tested yield, within the experimental error, the same results.

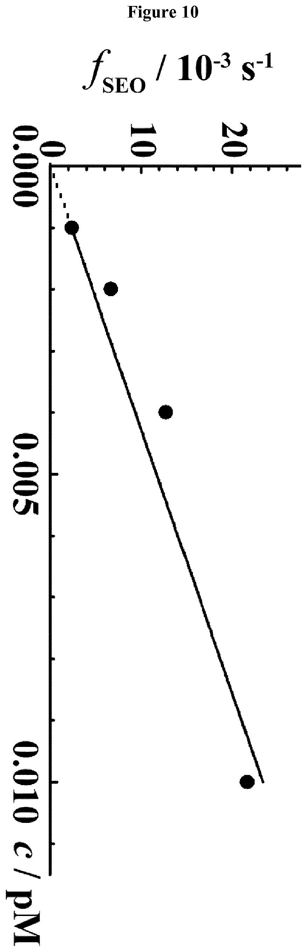

**Figure S3 (Figure 10). The frequency of single events occurrence $f_{SEO}$ is linearly proportional to the actual concentration of 100 nm fluospheres in dilute aqueous suspension at $c \leq$ 10 fM.** The slope of the regression line is (2.3 $\pm$ 0.3), and the coefficient of determination $R^2$ = 0.92.

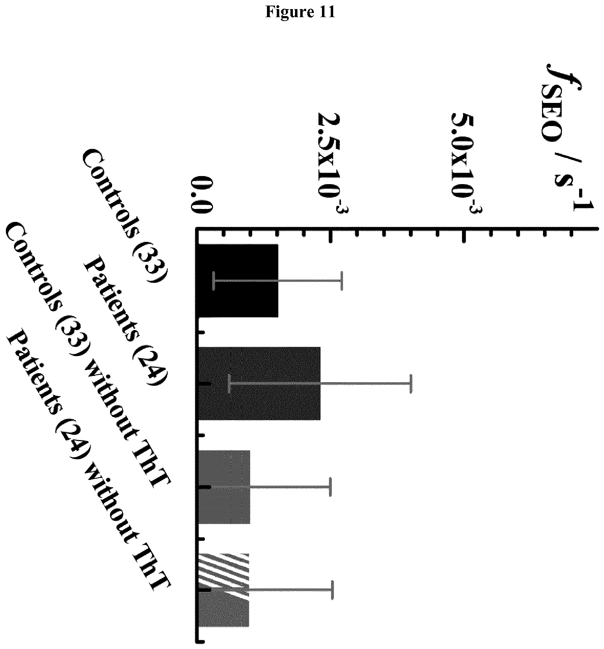

**Figure S4 (Figure 11).** Control experiments comparing the mean frequency of single event occurrence in serum with ThT for the control (black) and the patient (dark grey) groups with the measurement in serum without ThT for the control (light grey) and the patient (light grey striped) groups.

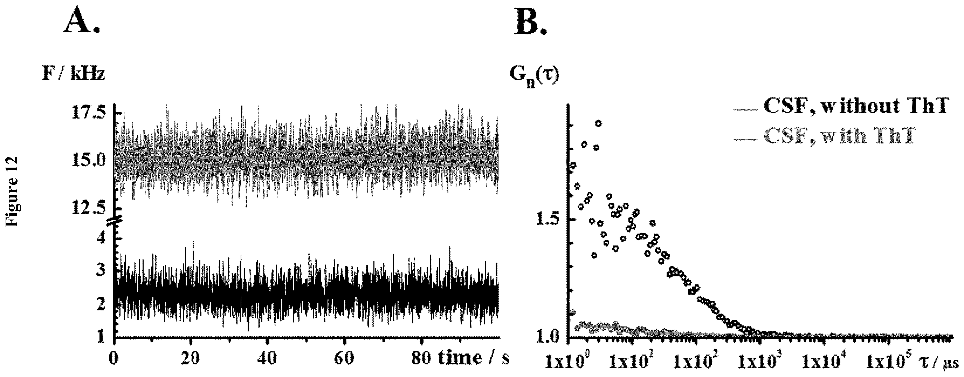

**Figure S5 (Figure 12). ThT assay in the CSF of an apparently healthy control subject. A.** Fluorescence intensity fluctuations over time recorded in the CSF before adding ThT (black) and after adding ThT (grey). **B.** Corresponding temporal autocorrelation curves.

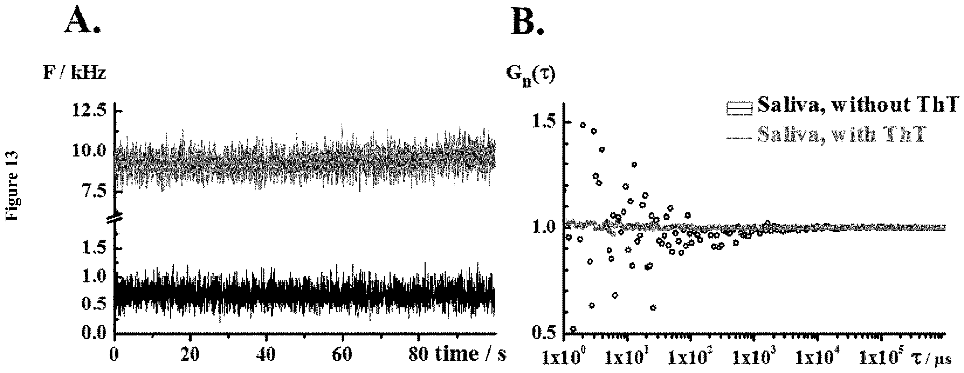

**Figure S6 (Figure 13). ThT assay in the saliva of an apparently healthy control subject.** A. Fluorescence intensity fluctuations over time recorded in saliva before adding ThT (black) and after adding ThT (grey). **B.** Corresponding temporal autocorrelation curves.

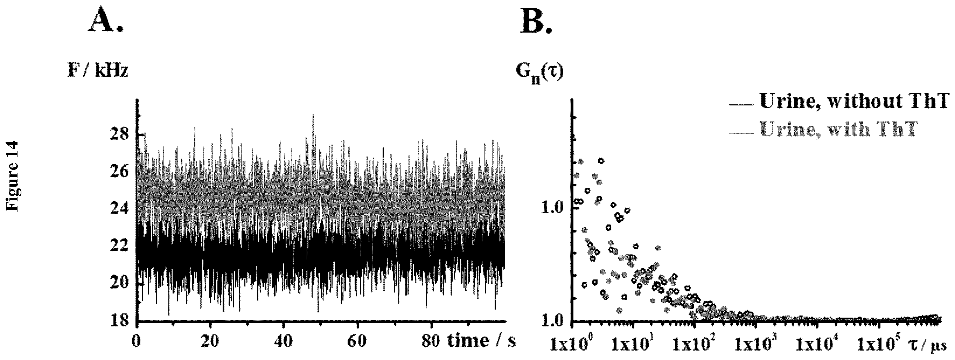

**Figure S7 (Figure 14). ThT assay in urine of an apparently healthy control subject. A.** Fluorescence intensity fluctuations over time recorded in urine before adding ThT (black) and after adding ThT (grey). **B.** Corresponding temporal autocorrelation curves.

**Figure 15. Schematic presentation of a single-molecule fluorescence imaging system with a zepto-litre observation wave-guide built on a transparent fused silica substrate using a metal film. One well in the metal film is magnified.** (M J Levene, J Korlach, S W Turner, M Foquet, H G Craighead and W W Webb, Science vol. 299, No. 5607 (Jan. 31, 2003) pp. 682-686).

**Figure 16. Diameter of the zepto-litre observation wave-guide. A.** Three dimensional finite-element time-domain simulation of the intensity distribution (log scale) for a zepto-litre wave-guide 50 nm in diameter and 100 nm deep. The metal material is Al (aluminium). **B.** S(z) curves for different diameters of the well diameter. **C.** Effective observation volume element size ($V_{eff}$) and the corresponding concentration for which there is, on average, one molecule in the volume (<N> = 1) (M J Levene, J Korlach, S W Turner, M Foquet, H G Craighead and W W Webb, Science vol. 299, No. 5607 (Jan. 31, 2003) pp. 682-686).

**Figure 17: Fused silica coverslip with zepro-litre waveguide arrays. A.** The coverslip with overlying gasket to isolate arrays for individual experiments. **B and C.** Successive increase in scale of waveguide. **D.** A scanning electron microscope image of an individual well. (M J Levene, J Korlach, S W Turner, M Foquet, H G Craighead and W W Webb, Science vol. 299, No. 5607 (Jan. 31, 2003) pp. 682-686).

**Figure 18. Schematic presentation of the optical arrangement in an instrument for massively parallel Fluorescence Correlation Spectroscopy (mpFCS).** The single laser beam, the intensity of which is regulated using neutral density filters with different transmission powers mounted on a filter wheel (NDFW), is expanded (L1 and L2), guided through a periscope assembly (PA) and focused by the Focusing Lens (L3) on the Diffractive Optical Element (DOE) designed to split the single laser beam into a rectangular array of 32×32 (1024) beams. The illumination matrix of 32×32 laser beam spots is formed at the image plane of the rear port of the microscope and guided by the relay optics of the microscope (RO) to the long pass dichroic mirror (LPDM) and through the objective (OBJ) into the sample. Fluorescence, collected by the objective, passes through the LPMD and is guided to the matching 2D array if single photon avalanche photodiodes (SPAD camera). TOP: Image of the illumination matrix generated in the focal plane of the microscope objective visualized using a thin layer of Rhodamine 6G (Rh6G) and an 18.0 megapixel digital single-lens reflex (DSLR) camera (Navigation camera). The zero-order diffraction peak is visible in the centre (Vital M, Bronzi D, Krmpot AJ, Nikolic SN, Schmitt F-J, Junghans C, Tisa S, Friedrich T, Vukojevic V, Terenius L, Zappa F, Rigler R, A single-photon avalanche camera for fluorescence lifetime imaging microscopy and correlation spectroscopty. IEEE J. Sel. Top. Quantum Electron. 2014 20:344-353).

**Figure 19. Determining the distribution of sizes of amyloidogenic aggregates in solution and in blood serum using massively parallel Fluorescent Correlation Spectroscopy (mpFCS). A.** Diffusion time map in 1024 observation volume elements. **B.** Diffusion histogram across all 1024 observation volume elements. **C.** Times series recorded in one observation volume in an aqueous solution of amyloid $\beta_{1\text{-}42}$ ($A\beta_{42}$) peptide (20 $\mu$M $A\beta_{42}$, 10 $\mu$M ThT, room temperature (RT ≈ 21°C)) 3 hours after initiation of the reaction. **D.** Time series recorded in one observation volume element, in a blood serum sample. The fluorescence intensity burst that is clearly visible above the background reflects the passage of a ThT reactive nanoplaque.

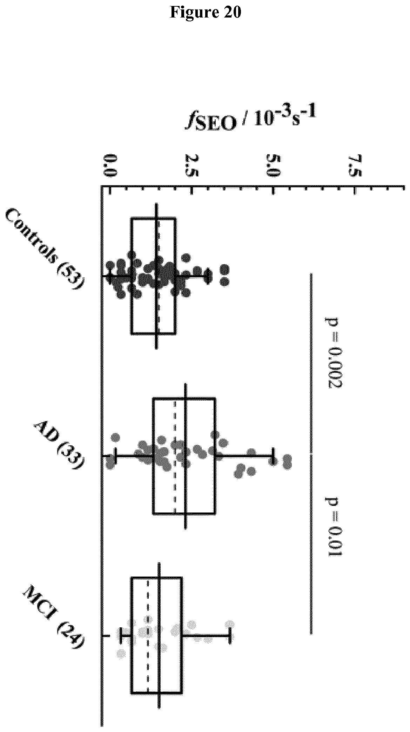

**Figure 20. Differences in the amount of structured Th-T reactive amyloidogenic aggregates in the blood serum of individual diagnosed with Alzheimer's disease (AD), mild cognitive impairment (MCI) and apparently healthy individuals (Controls) as reflected by the measured frequency of single events occurrence ($f_{SEO}$).** In the box-and-whisker plot, the box indicates one standard deviation above and below the mean of the data (solid line in the middle), the dashed horizontal line is the median, and the ends of the whiskers give the 5-95 % interval.

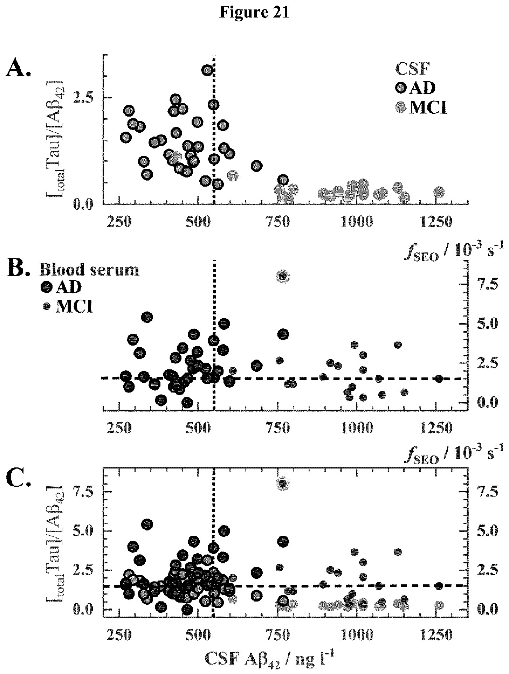

**Figure 21. Correlation between clinical CSF parameters and the frequency of single event occurrence ($f_{SEO}$) in blood serum. A.** The ratio of the total Tau level over $A\beta_{42}$ level in the CSF as a function of the $A\beta_{42}$ level in the CSF. Light grey dots with a dark grey rim designate data from individuals diagnosed with AD. Light grey dots designate data from individuals diagnosed with MCI. In all graphs, the dotted vertical line indicates the generally accepted limit for an AD diagnosis based on $A\beta_{42}$ levels in the CSF, $[A\beta_{42}] \leq 550$ ng l$^{-1}$. **B.** The frequency of single event occurrence in blood serum as a function of the $A\beta_{42}$ level in the CSF. Large dark grey dots with a black rim designate data from individuals diagnosed with AD. Small dark grey dots indicate data from individuals in the patient cohort that were diagnosed with MCI. The dashed horizontal line indicates the cut-off value for $f_{SEO}$ in the blood serum, $f_{SEO} \geq 1.5 \times 10\text{-}3$ s$^{-1}$ (5.4 h$^{-1}$). **C.** Comparison between CSF and blood serum measurements. Light grey axes and symbols relate to measurements in the CSF, showing the ratio of the total Tau level over $A\beta_{42}$ level in the CSF as a function of the $A\beta_{42}$ level in the CSF. Dark grey ordinate and symbols relate to measurements in the blood serum. Dark grey symbols present the frequency of single event occurrence in the blood serum as a function of the $A\beta_{42}$ level in the CSF. Data from individuals diagnosed with AD based on a multimodal clinical assessment are represented by the large dots. The point encircled by a light grey circle in B. and C. indicates the value from a patient with gastric bypass that was diagnosed with MCI.

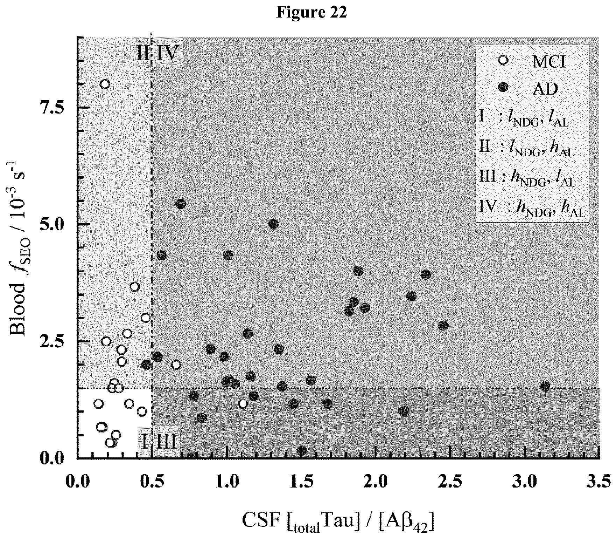

**Figure 22. Contribution of neurodegeneration *versus* the amyloidogenic load in individuals with MCI and AD.** By combining the CSF and blood analysis data, patients could be stratified in four groups according to the extent of neurodegeneration and amyloidogenic load as follows: **I** - low neurodegeneration ($l_{NDG}$) and low amyloidogenic load ($l_{AL}$); **II** - low neurodegeneration ($l_{NDG}$) and high amyloidogenic load ($h_{AL}$); **III** - high neurodegeneration ($h_{NDG}$) and high amyloidogenic load ($h_{AL}$); **IV** - high neurodegeneration ($h_{NDG}$) and low amyloidogenic load ($l_{AL}$). According to this classification, individuals with MCI that exhibit low neurodegeneration and high amyloidogenic load may be at risk to develop AD. In these individuals amyloid beta physiology is disturbed, while neurodegeneration is still low.

**Specific Examples**

**Example 1: Analysis in blood serum**

**Summary**

By using time-resolved detection of Thioflavin T (ThT) fluorescence intensity fluctuations in a sub-femtoliter sized observation volume element, we were able to detect with single-molecule sensitivity very low levels of small structured amyloidogenic nano-plaques in the blood serum of apparently healthy individuals and in patients diagnosed with Alzheimer's disease (AD). The frequency of encountering small structured amyloidogenic nano-plaques and their size were significantly higher in the blood serum of individuals diagnosed with AD than in control subjects. The results of our study show that unlike bulk fluorescence ThT spectroscopy, time-resolved ThT fluorescence intensity fluctuation analysis allows direct detection, quantification and size determination of small structured amyloidogenic nano-plaques in the blood serum. The method neither relies on the use of immune-based probes, nor on the use of radiotracers, signal-amplification or protein separation techniques, and provides a minimally invasive test for fast and cost-effective early determination of structurally modified proteins in bodily fluid, including the peripheral blood circulation (Figures 1-11 and 20-22), cerebrospinal fluid (Figure 12), saliva (Figure 13) and in urine (Figure 14).

**Abbreviations**

AD - Alzheimer's Disease; $A\beta$ - Amyloid $\beta$ peptide; CNS - Central Nervous System; CPSM - Counts per second per molecule; CSF - Cerebrospinal Fluid; FCS - Fluorescence Correlation Spectroscopy; FIFA - Fluorescence Intensity Fluctuation Analysis; Mild Cognitive Impairment - MCI; OVE - Observation Volume Element; Rh6G - Rhodamine 6G; Single event occurrence - SEO; Subjective Cognitive Impairment - SCI; tACC - Temporal Autocorrelation Curve; ThT - Thioflavin T.

**Introduction**

Alzheimer's disease (AD) is a devastating neurodegenerative ailment and the most common cause of dementia worldwide (1,2). AD progressively destroys vital mental abilities of the affected individuals and significantly reduces their cognitive capacity. The extent of damage induced by AD is such that the affected persons are eventually no longer able to take care of themselves, and are in need of constant care and assistance for the rest of their lives. Unfortunately, despite intensive research and a great societal need, methods for reliable and cost-effective early diagnosis of AD are still in their infancy. This also presents an impediment for the development of new drugs, and there is presently no cure for AD.

The main obstacle for further progress is the lack of important basic knowledge about complex dynamic processes that lie beneath the development of AD. While molecular mechanisms are not yet fully understood, it is well established that an imbalance in the production and clearance of amyloid beta ($A\beta$) peptides, accompanied by their self-assembly into structured aggregates and accumulation into so-called senile plaques in the central nervous system (CNS) of affected individuals, is a very early sign of AD. In view of that notion, the Amyloid Cascade Hypothesis proposes that misfolding, aggregation and deposition of $A\beta$ peptides is a major cause of AD (3). $A\beta$ peptides are 39-42 amino acids long peptides that are derived from the Amyloid Precursor Protein (APP) *via* proteolytic processing. The most common forms of $A\beta$ peptides are $A\beta40$ and $A\beta42$ (reviewed in (4)).

The aggregation mechanisms of $A\beta$ are complex. They have been investigated using a variety of different analytical techniques, including fluorescence spectroscopy and fluorescence correlation spectroscopy ((5) and references therein). Early on the benzothiazole salt Thioflavin T (ThT) fluorescence assay became a standard method for amyloid detection by fluorescence spectroscopy and microscopy (6,7). As more recent studies have shown, amyloidogenic aggregates enriched with pleated $\beta$-sheet secondary structure readily bind ThT and significantly alter its spectral properties, shifting the absorption spectrum towards longer wavelengths and significantly increasing the fluorescence quantum yield (Fig. 1, inset) (8-12).

Diagnostic methods for AD are still under heavy debate (13-16). For diagnosis before a potential patient's mental decline is noticeable, it is required that reliable biomarkers are available (17). Serum and CSF autoantibodies against $A\beta$ have shown promise and warrant further investigations (18,19), but the results have not been particularly encouraging so far. Brain imaging using PET and radiotracers will reflect reliably on fibrillar deposits of amyloid in the brain that are > 1 mm in size (20-22). Measuring the content of $A\beta$ peptides (and Tau protein) in the cerebrospinal fluid (CSF) using immunochemical techniques is also considered reliable (23-25). However, both PET and CSF methodologies are invasive and expensive. In topical reviews (13,14) the authors list drawbacks of simply measuring $A\beta$ in blood plasma as a diagnostic method, and show that attempts to correlate blood studies and PET or CSF results are not convincing

EP 3 775 884 B1

so far. The heterogeneous and different structural states of Aβ measured by the two technologies are one probable reason for the experimental difficulties: the immunochemical methods measure the soluble forms of the Aβ peptide(s), whereas the PET method measures the precipitated insoluble peptide aggregates in the brain. There is no clear direct connection between the two types of measurements, even if both relate back to the presence and behaviour of the Aβ peptide. There is obviously a strong need for a novel, non-invasive and reliable method for early AD diagnostics based on an additional biomarker methodology. The lack of such methods also presents an impediment for the development of new drugs - the development of novel therapies aiming to reduce the amyloid burden in the brain critically depends on the possibility to routinely identify and characterize the levels of structured amyloidogenic aggregates in the same individual repeatedly over time.

Here we present a fluorescence-based study of blood serum, simply labelled with the amyloid-sensitive dye ThT. The method is based on observations using a time-resolved and highly sensitive single-molecule fluorescence technology for Fluorescence Correlation Spectroscopy (FCS). We have earlier used the ThT based FCS assay (ThT-FCS), to study the heterogeneity and turnover of Aβ peptide aggregates during their fibril formation process *in vitro* (5). In the present study we demonstrate that there is a significant difference between the blood serum from diagnosed AD patients and control individuals, seen in the number and size of ThT-reactive structured amyloidogenic oligomeric aggregates, hereby named nano-plaques, visible in the serum of patients. The passages of these single nano-plaque particles through an extremely small (< femtoliter) observation volume can be observed in real time and counted. The number of passages, proportional to the concentration of nano-plaques, is significantly different in the blood serum from AD patients and controls.

**Materials and Methods**

*Patient cohort and ethics*

Blood samples were obtained upon informed consent from apparently healthy blood donors (control group) and from individuals who were, after examination in primary care settings, referred to the Memory Clinic, Karolinska University Hospital, Huddinge (patient cohort). Collection and handling of blood samples was approved by a regional ethics committee in Stockholm, permit nr. 2012/1019-31/1.

At the moment of admission AD diagnosis was not yet established and the patients did not take any pharmacological substances, such as cholinesterase inhibitors or N-methyl-D-aspartate (NMDA) receptor antagonists, typically used for the treatment of AD symptoms. However, due to their advanced age, many individuals in the patient cohort had age-related health problems and were on some prescribed medications. Detailed demographics and other relevant patient information are given in Table 1.

**Table 1. Demographics, pharmacotherapy and diagnosis of patients and control subjects.**

| Gender | Age | Count rate (kHz) | $f_{SEO}$ ($10^{-3}$ s$^{-1}$) | MMS | CSF A$\beta_{42}$ (ng/l) | CSF total Tau (ng/l) | CSF phospoTau (ng/l) | Diagnosis | Pharmacotherapy | Comorbidity |
|---|---|---|---|---|---|---|---|---|---|---|
| F | 58 | 92 | 8.0 | 26 | 766 | 140 | 24 | MCI* | Antidepressants, Sifrol, Paracetamol | Gastric bypass, Depression |
| M | 61 | 269 | 1.0 | 21 | 281 | 617 | 83 | AD | ASA, Statins | Transient Ischemic Attack (2002) |
| F | 61 | 255 | 5.4 | 24 | 338 | 234 | 33 | AD | Levaxin, Symbicort | Hypothyroidism |
| M | 84 | 324 | 3.1 | 27 | 315 | 574 | 64 | AD | Trombyl, Amlodipin, Losarstad | Renal insufficiency, Hypertension, Prostate cancer |
| F | 65 | 232 | 2.3 | 25 | 684 | 610 | 83 | AD | Losartan | Hypertension |
| F | 62 | 299 | 2.7 | 25 | 756 | 251 | 35 | MCI | Haldol, Venlafaxin, Propavan, Imovane | Depression, Generalized anxiety disorder, Smoker |
| M | 64 | 465 | 3.0 | 29 | 1020 | 466 | 60 | MCI | Vesicare, Pulmicort, Aerius | Asthma |
| F | 64 | 334 | 1.3 | 24 | 457 | 356 | 46 | early AD | Levaxin, Adalat, Alenat | Lung cancer (2000), Primary Biliary Cirrhosis, Hypothyroidism, Raynaud syndrome |
| M | 58 | 109 | 1.7 | 30 | 822 | 188 | 31 | SCI** | Tostrex, Cabergoline | Pituitary adenomas, Hyperprolactinemia |
| M | 77 | 175 | 0.9 | 24 | 441 | 367 | 47 | AD | Not on any medication | Lupus Erythematosus (2014), Psoriasis |
| M | 81 | 242 | 3.7 | 19 | 466 | 523 | 62 | AD and | Oral Antihyperglycemic | Hypertension, Renal insufficiency, |

| | | | | | | | | | | |
|---|---|---|---|---|---|---|---|---|---|---|
| | | | | | | | | Vascular Dementia | Agents, Antihypertensives | Obstructive Sleep Apnea, Diabetes Mellitus Type 2, Gout |
| M | 82 | 103 | 1.7 | 23 | 679 | 524 | 59 | AD and Vascular Dementia | Insulin, Statins | DM type 2 |
| F | 58 | 253 | 2.2 | 30 | 485 | 477 | 57 | AD | Not on any medication | Burnout |
| F | 66 | 196 | 1.6 | 30 | 328 | 326 | 37 | early AD | Citalopram, Naproxen, Statins, Antihypertensives | Hypertension, Transient Ischemic Attack, Depression |
| F | 77 | 169 | 1.4 | 24 | 543 | 450 | 60 | AD and Vascular Dementia | Pantoloc, Calcichew, Salures, Acetylcystein, Betmiga | Hypertension |
| F | 58 | 211 | 0.3 | 26 | 1100 | 235 | 60 | SCI | Omeprazol, Levaxin, ASA, Metformin, Omeprazxol, Cymbalta | Diabetes Mellitus Type 2, Depression, Obesity, Hypothyroidism |
| F | 60 | 182 | 1.2 | 25 | 800 | 276 | 35 | MCI | Omeprazol, Laktuloz | Depression, Epilepsy |
| M | 77 | 171 | 1.0 | 25 | 423 | 924 | 96 | AD | ASA, Seloken, Amlodipin, Lipitor | Hypertension, Angina Pectoris |
| F | 48 | 160.5 | 4.0 | 17 | 294 | 554 | 49 | AD | *Not established* | *Not established* |
| M | 75 | 135.5 | 2.3 | 30 | 474 | 491 | 61 | # | Omeprazol, ASA, Behepan | Transient Ischemic Attack |
| F | 61 | 119 | 1.7 | 28 | 332 | 545 | 53 | # | Lopid | Hypercholesterolemia |
| F | 56 | 111 | 2.8 | 23 | 428 | 1050 | 98 | AD | *Not established* | *Not established* |
| F | 68 | 109 | 1.5 | 30 | 450 | 541 | 58 | # | Omeprazol, Oral Antihyperglycemic Agents, Statins | Diabetes Mellitus Type 2, Hyperlipidemia |

| F | 78 | 135.5 | 0.2 | 22 | 252 | 373 | 44 | # | Calcichew, Alenat | Monoclonal Gammopathy of Undetermined Significance, Osteoporosis |
|---|---|---|---|---|---|---|---|---|---|---|
| **Patients** $n_p = 24$ **15F:9M** | **66 ± 10** | **202 ± 92** | **2.3 ± 1.7** | **25 ± 4** | **539 ± 236** | **462 ± 214** | **56 ± 20** | | | |
| **Controls** $n_c = 33$ **17F:16M** | **39 ± 15** | **155 ± 43** | **1.5 ± 1.2** | | | | | | | |

*MCI – Mild Cognitive Impairment
** SCI – Subjective Cognitive Impairment
#Diagnosis not established at the time of data analysis. The primary data were included in the overall analysis of patients versus control (Fig. 4A).

*Blood collection and serum, plasma and cell lysate sample preparation protocol*

Blood from superficial veins of the upper limbs is collected using the evacuated tube system for venepuncture (BD Vacutainer® tubes, Becton, Dickinson and Company) and treated as described below to yield blood serum. ThT was added to the sample before submission to time resolved Fluorescence Intensity Fluctuation Analysis (FIFA).

*Blood serum:* To extract blood serum, 5 ml of blood is collected in a BD Vacutainer® Plastic Serum tube with Red BD Hemogard™ Closure, which contains micronized silica particles with $SiO_2$ as the main clot activator component sprayed on the inner walls of the tube to accelerate the clotting process and does not contain thrombin. To activate blood clotting, the tube is gently inverted 180° and back 5-6 times and the blood is allowed to clot for 60 min. Thereafter, the tube is centrifuged for 10 min at $2500 \times g$ and the supernatant above the clot, *i.e.* the blood serum is visually inspected for turbidity and for evidence of hemolysis. Turbid blood serum samples and samples with grossly visible pink discoloration were not subjected to further analysis even though we have established in separate control experiments that moderate turbidity and hemolysis do not affect the outcome of the results (data not shown).

For immediate use, 200 $\mu$l of the blood serum is aliquoted into a well of an 8-well chambered coverglass (Lab-Tek Chambered Coverglass, Thermo Scientific, USA), mixed with 1.6 $\mu$l of 2.5 mM ThT to a final concentration of 20 $\mu$M ThT and subjected to FIFA as described below. All experiments are run in duplicates.

In order to assess the effect of short-term serum storage on the outcome of the analysis, the serum is suspended and 200 $\mu$l aliquots are transferred to 0.5 ml Eppendorf Safe-Lock Centrifuge Tubes and stored at - 20 °C for 2, 4 or 24 weeks. Only one freeze-and-thaw cycle was allowed.

*Blood plasma:* To extract blood plasma, 5 ml of blood is collected in a BD Vacutainer® Plastic K3EDTA tube with Lavender BD Hemogard™ Closure that contains 1.8 mg of the anticoagulant $K_3EDTA$ *per* 1 ml of blood, yielding an EDTA concentration of ~ 7 mM. This concentration of EDTA neither interferes with A$\beta$ aggregation, nor with the ThT assay, based on bulk ThT fluorescence spectroscopy measurements showing no effect of high EDTA concentrations (up to 10 mM) neither on the length of the lag phase nor on the slope of the fluorescence growth curve during the *in vitro* A$\beta$ peptide aggregation process (data not shown). The tube is gently inverted 180° and back 10 times and allowed to sediment for 60 min. Thereafter, the tube is centrifuged for 10 min at $2500 \times g$ and the supernatant above the cell layers, *i.e.* the blood plasma, is visually inspected for turbidity and for evidence of hemolysis. As described above, turbid blood plasma samples and blood plasma samples with grossly visible pink discoloration were discarded and not subjected to further analysis. For immediate use, 200 $\mu$l of blood plasma is aliquoted into a well of an 8-well chambered coverglass, mixed with 1.6 $\mu$l of 2.5 mM ThT to a final concentration of 20 $\mu$M ThT and subjected to FIFA following the same procedure as described for the blood serum.

In a limited set of experiments blood plasma is collected in BD Vacutainer® CTAD tube with Light Blue BD Hemogard™ Closure in order to assess the advantages of using citrate buffer. Differences between EDTA or citrate plasma samples were not observed.

*Cell lysate:* To prepare blood cell lysates, 0.1 ml of 1% Triton X-100 in 150 mM NaCl is added to the cell pellet remaining at the bottom of the BD Vacutainer® Plastic K3EDTA tube with Lavender BD Hemogard™ Closure after removal of the blood plasma; the content is centrifuged for 10 min at $2500 \times g$ and the cell lysate is removed for analysis. For immediate analysis, 200 $\mu$l of cell lysate is aliquoted into a well of an 8-well chambered coverglass, mixed with 1.6 $\mu$l of 2.5 mM ThT to a final concentration of 20 $\mu$M ThT and subjected to FIFA following the same procedure as described for the blood serum.

*Short background on Fluorescence Correlation Spectroscopy (FCS)*

Fluorescence Correlation Spectroscopy (FCS) is a quantitative analytical method with the ultimate, single-molecule sensitivity for the detection of bright fluorescent molecules in dilute aqueous solutions (26-32). FCS achieves single-molecule sensitivity by observing with high temporal resolution the time course of spontaneous fluorescence intensity fluctuations in a very small volume that is in conventional instrumental setups about $2 \times 10^{-16}$ l - $10 \times 10^{-16}$ l. In such a tiny volume the background noise originating from molecules present in large access, such as the solvent molecules, is significantly reduced by looking at a small number of molecules at a time. Hence, the passage of a bright fluorescent molecule through the small observation volume gives rise to a prominent change in fluorescence intensity that can be readily detected.

To generate the tiny observation volume the instrumentation for FCS takes advantage of the specific arrangement of optical elements in a confocal microscope to sharply focus incident laser light into the sample through a high numerical aperture (*NA*) objective and further reduce the volume from which fluorescence is detected by placing a pinhole in the optically conjugate plane in front of the detector to eliminate out-of-focus light (Fig. 1A). In this way, a miniature Observation Volume Element (OVE) is generated in the sample (Fig. 1B) that is sufficiently small to allow the observation of fluorescence intensity fluctuations that arise around a(n) (non)equilibrium steady state due to spontaneous, thermally driven microscopic fluctuations in the positions of molecules through the OVE (Fig. 1C). These fluctuations are recorded over time and analysed to extract information about the average number of molecules in the OVE, which is representative of the sample concentration, and the average transition time, *i.e.* the time needed for a molecule to pass through the OVE, the so-called translation diffusion time ($\tau_D$), which is defined by the diffusion coefficient (*D*) of the molecule of interest, *i.e.* by its size and shape in a medium of given viscosity (Fig. 1D).

Different methods can be used to analyse the florescence intensity fluctuations recorded in an FCS experiment (28,30). Typically, temporal autocorrelation analysis is applied to derive a temporal autocorrelation curve (tACC) and read out the average number of molecules in the OVE from the amplitude of the tACC and the diffusion time from the characteristic decay time of the tACC (26,27,30). Details of temporal autocorrelation analysis and how to derive the average number of molecules in the OVE and the translational diffusion coefficient of the investigated particles is given in "Temporal autocorrelation analysis of fluorescence intensity fluctuations". In this study, temporal autocorrelation analysis is used for instrument calibration, while the actual data analysis is performed by analysing the frequency of single events occurrence as described below, in *Data analysis.* This method is validated using standard series of dilute aqueous suspension of fluospheres (*d* = 100 nm), described in detail in "Validation of data analysis using dilute aqueous suspension of 100 nm fluospheres".

### Time-resolved ThT fluorescence intensity fluctuation measurements

Time-resolved fluorescence intensity fluctuation measurements are performed using a ConfoCor 2 system (Carl Zeiss, Jena, Germany), consisting of an inverted microscope equipped with a C-Apochromat 40 ×, *NA* = 1.2, water immersion UV-VIS-IR objective. ThT fluorescence is excited using the 458 nm line of the Argon laser. The incident laser light is tightly focused into the sample by passing through the objective lens; laser intensity measured at the objective is 6 $\mu$W. The elastically scattered light and the spectrally distinct fluorescence light are collected backward through the same objective lens and separated using the HFT 458 main dichroic beam splitter. The emitted light is spatially filtered by passing through a pinhole to the silicon avalanche photodiode detector (SPCM-AQR-1X; PerkinElmer, USA). The pinhole size in front of the detector was set to 70 $\mu$m (1 Airy). *Instrument calibration:* Standard aqueous solutions of Rhodamine 6G (Rh6G), 10-25 nM, are used for instrument calibration. For this purpose, temporal autocorrelation analysis was applied (as described in "Temporal autocorrelation analysis of fluorescence intensity fluctuations"). The same optical setting that is used for ThT, as described above, is used for Rh6G and the signal was recorded in a series of 10 consecutive measurements, each measurement lasting 10 s. Under these settings, the translation diffusion time of Rh6G was determined to be $\tau_{D,Rh6G}$ = (24 $\pm$ 2) $\mu$s (n = 460); the counts per molecule and second (CPMS) was CPMS = (5.1 $\pm$ 0.6) kHz, and the structural parameter was S = 7 (Fig. S1). The OVE size was determined to be V $\approx$ $2.0 \times 10^{-16}$ l. FCS measurements were validated using the individually modified ConforCor3 system that is available in our laboratory (33). Significant differences between measurements' outcomes obtained using one or the other system were not observed.

*Measurement on blood serum:* ThT serum fluorescence intensity fluctuations were recorded in a series of 300 measurements, each measurement lasting 10 s, yielding a total run time of 3000 s. All measurements were performed at 20 °C in an air conditioned room. The data were evaluated as described below.

### Data analysis

The frequency with which the structured amyloidogenic nano-plaques pass through the OVE is determined by performing an automated single event counting analysis to identify rare bursts in ThT fluorescence intensity that reflect the occasional passage of ThT-reactive structured amyloidogenic nano-plaques through the OVE. For this purpose a program was written using the Matlab software. A fluorescence peak, *i.e.* "a single event", is defined as an increase in fluorescence intensity, $\Delta F(t)$, that differs from the mean fluorescence intensity of the whole time series, $\langle F(t) \rangle$, by a value that is five times larger than the standard deviation (SD) of the whole time series, $\Delta F(t) = (F(t) - \langle F(t) \rangle) > 5 \times SD$. The number of observed fluorescence peaks is then normalized to the total measurement time (here 3000 s) and the thus obtained frequency of single events occurrence ($f_{SEO}$) is expressed in $s^{-1}$ and compared across samples.

The size of the ThT-reactive structured amyloidogenic oligomeric aggregates is determined in the following way. All recorded time traces were subjected to temporal autocorrelation analysis. Time traces where passage of ThT-reactive structured amyloidogenic oligomeric aggregates is observed gave rise to a tACCs, whereas time traces that only contained background signal did not show any tACC and were excluded from further analysis. All tACCs were then normalized to the same amplitude, $G(\tau) = 1$ at $\tau = 10$ $\mu$s, and all tACCs from the same group, patient or the control group, were combined to yield an average tACC for the patient and the control groups, respectively. Difference in size is then assessed from the difference in characteristic decay time of the tACCs - the longer the characteristic decay time of the tACC, the larger is the nano-plaque size.

The above described procedures for determining the $f_{SEO}$ and the size of the ThT-reactive structured amyloidogenic oligomeric aggregates were validated using dilute aqueous suspension of 100 nm fluospheres, as described in "Validation of data analysis using dilute aqueous suspension of 100 nm fluospheres", Figs S2 and S3. These control experiments confirm that the diffusion time is correctly determined using the proposed approach (Fig. S2) and that the concentration of ThT-reactive structured amyloidogenic oligomeric aggregates can be assessed from the $f_{SEO}$ by means of a calibration curve (Fig. S3). Researchers performing the time-resolved ThT serum FIFA were blinded as to the individual diagnoses until the data analysis was completed. Likewise, the diagnosis was established using standard clinical tests and results of the time-resolved ThT serum fluorescence intensity fluctuation analysis were not known by the clinicians until the study was completed and the code was revealed. In order to assess whether the mean $f_{SEO}$ values in the control and the patient cohorts are significantly different, unpaired t-test analysis was performed using the online available GraphPad Software tool (http://www.graphpad.com/quickcalcs/ttest1.cfm?Format=SD).

## *Chemicals*

All chemicals were used without further purification and purchased from commercial sources as follows: Rhodamine 6G (Sigma-Aldrich); Thioflavin T (Sigma-Aldrich); Carboxylate functionalized quantum dot nanocrystals, d = 20 nm, emission maxima at 525 nm (Qdot® 525 ITK™ Carboxyl Quantum Dots) and yellow-green fluorescent (Ex/Em: 505/515) carboxylate-modified polystyrene nano/microspheres of diameter d = 100 nm (FluoSpheres® Size Kit #2) from Molecular Probes. Fluosphere suspension was sonicated 30-60 min before use.

## *Temporal autocorrelation analysis of fluorescence intensity fluctuations*

In temporal autocorrelation analysis, signal self-similarity analysis is performed to detect non-randomness in data, and to identify an appropriate time series model if the data are not random. As a first step in this process, the so-called normalized intensity autocorrelation function $G(\tau)$ is calculated:

$$G(\tau) = 1 + \frac{\langle \delta F(t) \cdot \delta F(t+\tau) \rangle}{\langle F(t) \rangle^2} \quad , \qquad (1)$$

where $\langle F(t) \rangle$ is the mean fluorescence intensity over the whole time series; $\delta F(t) = F(t) - \langle F(t) \rangle$ is a fluctuation in fluorescence intensity recorded at the time point $t$; $\delta F(t + \tau) = F(t + \tau) - \langle F(t) \rangle$ is a fluctuation in fluorescence intensity in the same time series recorded at a later time point, $t + \tau$; and $\tau$ is the time lag between them.

The normalized autocorrelation function $G(t)$ is then plotted as a function of different time lags $\tau$, to yield an autocorrelation curve ACC. For processes that are random, $G(t) \approx 1$ for all lag time values. For processes that are not random, the ACC has a maximal limiting value of $G(\tau)$ as $\tau \to 0$, which decreases to the value of $G(\tau) = 1$ at long time lags, indicating that the correlation between the initial and the current property value of the fluorescence signal has been lost. For the simplest case of fluorescence intensity fluctuations generated by free three-dimensional (3D) translational diffusion of fluorescent molecules, the ACC has one characteristic decay time, which represents the average time that it takes for a molecule to pass through the OVE, *i.e.* it is equal to the translational diffusion time ($\tau_D$). The limiting value of $G(t)$ at t = 0, is inversely related to the average number of molecules ($N$) in the OVE and thus is representative of their concentration (26,27).

In order to extract $N$ and $\tau_D$ values, the experimentally obtained ACC is fitted using a theoretical autocorrelation function (AFt). For example, if one fluorescent species is present in the sample and transition between the singlet and the triplet state is the only intramolecular process affecting the fluorescence of the observed molecule, the ACC can be described using the following $AF_t$:

$$G(\tau) = 1 + \frac{1}{N} \cdot \frac{1}{\left(1 + \frac{\tau}{\tau_D}\right) \cdot \sqrt{1 + \frac{1}{S^2} \cdot \frac{\tau}{\tau_D}}} \cdot \left[1 + \frac{T}{1-T} \exp\left(-\frac{\tau}{\tau_D}\right)\right]. \qquad (2)$$

In equation (2) $N$ is the average number of molecules in the OVE; S is the spatial parameter, also called structural parameter, $S = \dfrac{\varpi_z}{\varpi_{xy}}$ , where $\varpi_{xy}$ and $\varpi_z$ are the $1/e^2$ radial and axial radius, respectively, of the OVE; $\tau$ is the time lag; $\tau_D$ is the translation diffusion time; $T$ is the average equilibrium fraction of molecules in the triplet state and $\tau_T$ is the triplet correlation time, related to the rate constants for intersystem crossing and the triplet decay. Spatial properties of the OVE are determined experimentally in instrument calibration measurements. The calibration is performed using a standard solution of a reference fluorescent dye for which the concentration and the translational diffusion coefficient are known. The experimentally derived ACC recorded for the standard solution is fitted by the autocorrelation function (2) allowing all parameters to freely vary and the values for $N$, $\tau_D$, $S^2$, $T$ and $\tau_T$ are determined by fitting. Using the known value of the translational diffusion coefficient $D$ for the reference dye and the determined values of $\tau_D$, the radial radius of the OVE can be determined from:

$$\tau_D = \frac{\varpi_{xy}^2}{4D} \, , \qquad\qquad (3)$$

and the axial radius $\varpi_z$ from the structure parameter $S^2$. For all further measurements, the structure parameter value is fixed to the value determined in the calibration measurements.

For our system, standard solutions of Rh6G in water were used for instrument calibration (Fig. S1).

### Validation of data analysis using dilute aqueous suspension of 100 nm fluospheres

Dilute aqueous suspension of fluospheres ($d$ = 100 nm) of known concentration, 1 fM $\leq$ c $\leq$ 50 nM, were used to validate the diffusion time determination procedure (Fig. S2) and the concentration by counting the single events, *i.e.* by determining the frequency of single events occurrence $f_{SEO}$ (Fig. S3).

### Autofluorescence measurements on blood serum

Serum without ThT was used as a control for assessing the effect of random disturbances on the obtained results (Fig. S4). As can be seen, while fluorescence intensity peaks are occasionally detected in the serum without ThT (light grey and light grey striped), the mean frequency of their occurrence is significantly lower than in the serum with ThT (black and dark grey); there is no difference in the frequency at which these peaks occur in the control and the patient group; and the standard deviation is larger than the mean, suggesting that the mean value of random disturbances is not significantly different from zero (Fig. S4).

### Results

### Serum autofluorescence and serum ThT fluorescence

Blood serum is a complex aqueous mixture of different classes of chemical compounds, of which lipids (in the form of triglycerides) and proteins (albumin and immunoglobulins) are most abundant. Many of the compounds that blood serum is comprised of are natively fluorescent and absorb light at 458 nm, such as flavin (FAD) and pyridinic (NADH) coenzymes and lipopigments. As a consequence, blood serum exhibits autofluorescence and gives also rise to a distinct tACC with an amplitude $A_{semm}$ = (0.003 $\pm$ 0.002) and a single characteristic decay time $\tau_{serum}$ = (55 $\pm$ 15) $\mu$s (Fig. 2A, black). The simplest theoretical model that could fit these experimental data was the autocorrelation function for free 3D diffusion without triplet (AF1 in (27)). Holding the structure parameter fixed to its value determined by instrument calibration with Rh6G, $S_p$ = 5, the parameter values derived by fitting are: $N$ = 300 and $\tau_D$ = 55 $\mu$s.

When ThT is added to blood serum, several important differences are observed. Most notably, the amplitude of the tACC decreases markedly ($A_{ThT}$ = (0.0014 $\pm$ 0.0007)) and a small but discernible contribution of one or more components with long characteristic decay time is observed (Fig. 2A, grey). Hence, the simplest theoretical model that could fit these experimental data was an autocorrelation function for free 3D diffusion of two components without triplet (AF3 in (27)). The parameter values derived from the best fitting autocorrelation function are: $N$ = 750, $\tau_{D1}$ = 40 $\mu$s, $\tau_{D2}$ = 100 ms, $y_1$ = 0.94 and $y_2$ = 0.06. The structure parameter was fixed at the value determined in calibration experiments, $S_p$ = 5. The characteristic decay time of the first component, $\tau_{D1}$ = 40 $\mu$s, derived by fitting is very similar to the characteristic decay time in the serum without ThT, $\tau_D$ = 55 $\mu$s; in agreement with experimental findings showing a close overlap of ACCs normalized to the same amplitude, G($\tau$) = 1 at $\tau$ = 10 $\mu$s, (Fig. 2B).

Decrease in the amplitude of the ACC, which indicates that the average number of observable molecules increases in the OVE upon ThT addition, is consistent with making otherwise invisible ThT-reactive entities visible. ThT fluorescence in blood serum was in detail characterized by bulk fluorescence spectroscopy by Chauhan *et al.* (34) and Griffin *et al.* (35). Using flotation fractionation, Griffin *et al.* were able to show that about 94 % of ThT reactivity in blood serum is associated with the non-apoB containing fraction of blood serum and identified glycated and other chemical adducts of water-soluble serum albumin as the main contributors to the baseline ThT serum fluorescence (35). The observed appearance of a second component with significantly longer diffusion time is consistent with making visible large ThT-reactive entities that are present at very low levels, hence the small relative amplitude of the second component.

As expected based on previously published results (34,35), the mean serum ThT fluorescence intensities showed considerable variation between individuals (Table 1). However, in contrast to previous observations by bulk ThT fluorescence spectroscopy, the mean count rate values between the patient, $CR_p$ = (202 ± 92) kHz, and the control groups, $CR_c$ = (155 ± 43) kHz, differed significantly, as evident from the two-tailed P value, P = 0.0126.

***Time-resolved detection of fluctuations in ThT fluorescence intensity reveals passage of ThT-reactive amyloidogenic structured nano-plaques through the OVE***

Time-resolved detection of ThT serum fluorescence reveals the infrequent occurrence of very well identifiable peaks in fluorescence intensity that differ from the mean fluorescence intensity by a value that is more than five times larger than the standard deviation (SD) of the time series, $(F(t) - \langle F(t) \rangle) > 5 \times SD$ (Fig. 3). As already indicated, we call these peaks "single events".

In order to ascertain that these outbursts do not reflect electrical disturbances or occasional passage of small air bubbles or dust particles through the OVE, but rather reflect the passage of ThT-reactive amyloidogenic structured nano-plaques, control measurements were performed in the serum without ThT. As can be seen (Fig. S4 in "Autofluorescence measurements on blood serum"), while fluorescence intensity peaks are occasionally detected in the serum before ThT is added, the mean frequency of their occurrence is significantly lower than in the serum with ThT; there is no difference in the frequency at which these peaks occur in the control and the patient's samples; and the standard deviation is larger than the mean, suggesting that the mean value of random disturbances is not significantly different from zero.

We therefore present the occurrence of fluorescence intensity bursts, *i.e.* the occurrence of single events and the frequency of their appearance, termed "frequency of single event occurrence" ($f_{SEO}$), as our direct measure of the concentration of structured nano-plaques in the blood serum.

***The frequency of single events occurrence in the blood serum of AD patients is higher than in the blood serum of apparently healthy controls***

The frequency of single event occurrence, calculated as described in the Materials and Methods: Data analysis subsection, differs between the patient group, $f_{SEOp}$ = (2.3 ± 1.7)×10$^{-3}$ s$^{-1}$, and the control group, $f_{SEOc}$ = (1.5 ± 1.2)×10$^{-3}$ s$^{-1}$. A two-tailed P value, P = 0.0418, determined by unpaired t-test analysis suggests that the difference in the frequency of single events occurrence between the patient group and the control group is statistically significant (Fig. 4A). After revealing the code and separating the patients into subgroups based on the clinical diagnosis, the difference between the AD patients, $f^{AD}_{SEOp}$ = (2.5 ± 1.5)×10$^{-3}$ s$^{-1}$, and the control group, $f_{SEOc}$ = (1.5 ± 1.2)×10$^{-3}$ s$^{-1}$, remains statistically significant, P = 0.0420 (Fig. 4B).

Our analysis also shows that the serum concentration of structured amyloidogenic nano-plaques increases somewhat with aging, as evident from the frequency of single event occurrence that increases with a slope of (1.6 ± 1.0)×10$^{-5}$ s$^{-1}$ year$^{-1}$ and an intercept of (9.4 ± 0.6)×10$^{-4}$ s$^{-1}$ (Fig. 5, solid line). However, the adjusted R-squared value,

$$R^2_{adj} = 0.032,$$

indicates that this correlation is very weak. As a matter of fact, the same data set could be fitted with a zero-slope linear regression, with an intercept of (1.3 ± 0.2)×10$^{-3}$ s$^{-1}$, and a residual sum of squares (RSS) value that is lower for the zero-slope linear regression, $RSS_{z-slr}$ = 30.2, than for the best fit linear regression, $RSS_{lr}$ = 66.7.

***The size of the ThT-reactive amyloidogenic structured nano-plaques in the blood serum of AD patients is larger than in the blood serum of apparently healthy controls***

Temporal autocorrelation analysis, performed as described in Materials and Methods: Data analysis, shows an obvious difference in the characteristic decay time between the tACCs recorded for the two groups, with a markedly longer decay time for the patient group (Fig. 6). Evaluation of the normalized tACCs shows that the analysed system is complex and does not contain particles that are uniform in size, but rather contains a mixture of particles of very different sizes. The average diffusion time for the ThT-active particles in the control group is about 5 ms, and the average diffusion time for particles in the patient group is about 15 ms, corresponding, for globular molecules, to a molecular weight difference of about 27 times between the two groups. Obviously, the patient group carries larger ThT-active structured aggregates in the serum in addition to the larger number of nano-plaques.

*Clinical output in CSF versus time resolved ThT fluorescence intensity fluctuation analysis in blood serum*

We have compared the results obtained by time-resolved ThT FIFA in blood serum with the results obtained using established biochemical biomarkers in the cerebrospinal fluid (CSF), including CSF levels of the 42 amino acid form of the Amyloid beta peptide ($A\beta_{42}$) and the total CSF levels of the tau protein (t-Tau). The comparison shows that the results obtained by time-resolved ThT blood serum FIFA obviously match the results obtained by CSF biochemical analysis (Fig. 7). The generally accepted limit for an AD diagnosis based on $A\beta_{42}$ level in CSF is < 550 ng l$^{-1}$ (36). Based on the data presented in Fig. 7, we suggest that a limit of $f_{SEO} > 1.5 \times 10^{-3}$ s$^{-1}$ in blood serum is indicative of AD.

**Discussion**

In the presence of amyloid fibrils and structured amyloidogenic oligomers ThT acquires fluorescence excitation and emission spectra that are markedly distinct from those of the free dye (Fig. 1, inset) (8-12). This change in fluorescence is generally considered to be specific for ThT interactions with amyloidogenic structures, and is not induced upon binding to native protein monomers or amorphous aggregates (6,7). Due to this property, ThT has been widely used to visualize tissue amyloid deposits by fluorescence microscopy (37-39); ThT congeners are the bases for *in vivo* imaging using Positron Emission Tomography (PET) and Single Photon Emission Computed Tomography (SPECT) (40-42); and ThT is indispensable in basic studies of protein aggregation by classical, bulk fluorescence spectroscopy to monitor the time course of amyloid fibril formation from purified proteins and peptides *in vitro* (5,43,44).

While classical bulk ThT fluorescence spectroscopy is very important for basic studies of $A\beta$ aggregation kinetics (43), this method also has serious limitations. Most notably, classical bulk ThT fluorescence spectroscopy involves ThT fluorescence signal averaging over all $A\beta$ aggregation states that are present in the sample, and temporal averaging of ThT fluorescence intensity with a signal integration time that is typically of the order of 1 - 10 s (43). Under such measurement conditions, the contribution of the signal that originates from rare amyloidogenic structured aggregates is too small to be discernible from the background. Hence, the usefulness of bulk ThT fluorescence spectroscopy in biomedical diagnostics of amyloid-featuring diseases has been shown to be limited (34,35). In contrast to bulk ThT fluorescence spectroscopy, time-resolved detection of fluctuations in ThT fluorescence intensity in a sub-femtoliter OVE does not involve such averaging (5), allowing us to identify with fidelity the passage of ThT-active structured amyloidogenic aggregates through the OVE (Fig. 3). Thus, we report, for the first time, detection of structured amyloidogenic aggregates in the blood serum using time-resolved detection of ThT serum fluorescence intensity fluctuations. Moreover, we demonstrate that there is a positive correlation between AD diagnosis and the mean frequency at which ThT-reactive structured amyloidogenic aggregates occur in the blood serum AD (Figs 4 and 7). While the capacity of this method for early identification of individuals at risk to develop AD remains to be established, our results clearly show that this new method with single-molecule sensitivity, which neither relies on the use of immune-based probes, nor on the use of radiotracers, signal-amplification or protein separation techniques, may provide a minimally invasive test for fast and cost-effective early determination of structurally modified proteins in the peripheral blood circulation, CSF (Fig. S5), but also in other, more easily accessible biological fluids, such as saliva and urine (Figs S6 and S7, respectively). Immune-based assays can be used to confirm the results of the method, such as the most frequently used Enzyme-Linked Immunosorbent Assay (ELISA), digital ELISA, sandwich ELISA (45,46) sandwich ELISA (47) and associated methods recently reviewed by Andreasson *et al.* (48), and immunoprecipitation-mass spectrometry (IP-MS) (49) all have in common inherent limitations that are related to the specificity and the sensitivity of the antibody-antigen reaction (50-52). In the case of $A\beta$ immune-based assays, the following difficulties, to name but a few, are typically encountered: monomeric $A\beta$ that is present in excess can occupy binding sites on the antibody, thereby interfering with the detection of $A\beta$ oligomers (53); interference from the p3 peptide and $A\beta$-catabolic peptides derived from the non-amyloidogenic pathway of APP processing my introduce uncertainty in the interpretation of results (54); substrates for signal amplification and detection are inherently unstable and may produce signal even in the absence of enzyme (48). Proteomic approaches using Sodium Dodecyl Sulfate- PolyAcrylamide Gel Electrophoresis (SDS-PAGE), 2D-PAGE, Liquid Chromatography (LC), Mass Spectrometry (MS), Matrix Assisted Laser Desorption/Ionization-Time of Flight (MALDI-TOF) mass spectrometry, Electrospray ionization (ESI) mass spectrometry, Liquid Chromatography-Tandem Mass Spectrometry (LC-MS/MS) and related techniques, characterize directly the molecule of interest with high sensitivity and specificity, thereby providing invaluable information (55). However, these techniques are complicated by the difficulty to detect low-abundance proteins in complex mixtures such as biological fluids due to limitations in the dynamic range; inability to measure intact larger proteins and involved sample preparation procedures (56). As a consequence, there is to date no CSF biomarker discovered in proteomic studies that has reached the clinic (57).

In summary, we would like to underline that currently available immune-based methods clinically used for CSF and blood plasma biochemical analysis show that the concentration of $A\beta42$ decreases in the CSF and blood plasma of AD patients (23,36,49,58-60). This is very likely due to the fact that the antibodies largely recognize monomeric $A\beta$ peptides, whereas aggregation renders $A\beta$ peptides into states that are not easily recognized by immunochemistry as discussed by Klaver *et al.* (53) and references cited therein. In contrast, our method measures the ThT active states in the blood serum that corresponds to the actual amyloid states present in the fibrils seen in the brain. Obviously, the two types of observations both correlate with the AD pathology of the patient, as can be seen from the data presented in Fig. 7, but our method relies on a simple blood test to derive the information. The limit of quantification of the proposed method is unparalleled, reaching the ultimate single-molecule sensitivity for structured aggregates comprising more than 40 A$\beta$ monomers. Moreover, the proposed method allows us also to determine the distribution of structured aggregates size, which may be a valuable indicator of disease stage (early vs late) and a reliable predictor of disease progression.

**Example 2: Analysis in CSF**

0.5 ml Cerebral Spinal Fluid (CSF) aliquot stored at -80°C in a polypropylene tube without any additives was used. The sample was allowed to taw for 60 min at room temperature. Thereafter, 200 $\mu$l aliquots were transferred to an 8-well chambered coverslide containing 10 $\mu$l of aqueous ThT solution in each well. The sample was gently mixed by dispensing/drawing several times and subjected to analysis. Analysis was performed in duplicates.

**Example 3: Analysis in urine**

A midstream first morning urine specimen from apparently healthy donors was collected into a sterile container without any urine preservatives. The specimen was allowed to sediment for 60 min at room temperature. Thereafter, 200 $\mu$l aliquots were transferred to an 8-well chambered coverslide containing 10 $\mu$l of aqueous ThT solution in each well. The sample was gently mixed by dispensing/drawing several times and subjected to analysis. Analysis was performed in duplicates.

**Example 4: : Analysis in saliva**

Saliva samples from apparently healthy donors were taken in the morning, 2 hours after breakfast and after morning hygienic procedures. During this time the donor was allowed to take water only. After cursory cleaning the mouth by flushing with deionized water just before sample collection, 2 ml of naturally flowing saliva were passively sampled (without aspiration) and collected into a sterile container immersed in an ice-bath. After collection, 200 $\mu$l aliquots were transferred to an 8-well chambered coverslide containing 10 $\mu$l of aqueous ThT solution in each well. The sample was gently mixed by dispensing/drawing several times, allowed to temperate for 15 min and subjected to analysis. Analysis was performed in duplicates.

**References**

1. Prince, M., Comas-Herrera, A., Knapp, M., Guerchet, M., and Karagiannidou, M. (2016) World Alzheimer Report 2016. London, UK

2. (2017) Alzheimer's Disease Facts and Figures. in Alzheimer's & dementia, Alzheimer's Association

3. Selkoe, D. J., and Hardy, J. (2016) EMBO molecular medicine 8, 595-608

4. Wallin, C., Luo, J., Jarvet, J., Wärmländer, K. T. S., and Gräslund, A. (2017 in press) Israel J. Chem. 57

5. Tiiman, A., Jarvet, J., Graslund, A., and Vukojevic, V. (2015) Biochemistry 54, 7203-7211

6. Naiki, H., Higuchi, K., Hosokawa, M., and Takeda, T. (1989) Analytical biochemistry 177, 244-249

7. LeVine, H., 3rd. (1999) Methods in enzymology 309, 274-284

8. Biancalana, M., and Koide, S. (2010) Biochimica et biophysica acta 1804, 1405-1412

9. Wolfe, L. S., Calabrese, M. F., Nath, A., Blaho, D. V., Miranker, A. D., and Xiong, Y. (2010) Proceedings of the National Academy of Sciences of the United States of America 107, 16863-16868

10. Freire, S., de Araujo, M. H., Al-Soufi, W., and Novo, M. (2014) Dyes Pigm. 110, 97-105

11. Singh, P. K., Mora, A. K., and Nath, S. (2015) Chemical communications 51, 14042-14045

12. Kuznetsova, I. M., Sulatskaya, A. I., Maskevich, A. A., Uversky, V. N., and Turoverov, K. K. (2016) Analytical chemistry 88, 718-724

13. Oh, E. S., Troncoso, J. C., and Fangmark Tucker, S. M. (2008) Neuromolecular medicine 10, 195-207

14. Toledo, J. B., Shaw, L. M., and Trojanowski, J. Q. (2013) Alzheimer's research & therapy 5, 8

15. O'Bryant, S. E., Mielke, M. M., Rissman, R. A., Lista, S., Vanderstichele, H., Zetterberg, H., Lewczuk, P., Posner, H., Hall, J., Johnson, L., Fong, Y. L., Luthman, J., Jeromin, A., Batrla-Utermann, R., Villarreal, A., Britton, G., Snyder, P. J., Henriksen, K., Grammas, P., Gupta, V., Martins, R., Hampel, H., and Biofluid Based Biomarker Professional Interest, A. (2017) Alzheimer's & dementia : the journal of the Alzheimer's Association 13, 45-58

16. Blennow, K. (2017) Neurology and therapy 6, 15-24

17. Counts, S. E., Ikonomovic, M. D., Mercado, N., Vega, I. E., and Mufson, E. J. (2017) Neurotherapeutics : the journal of the American Society for Experimental NeuroTherapeutics 14, 35-53

18. DeMarshall, C. A., Nagele, E. P., Sarkar, A., Acharya, N. K., Godsey, G., Goldwaser, E. L., Kosciuk, M., Thayasivam, U., Han, M., Belinka, B., Nagele, R. G., and Alzheimer's Disease Neuroimaging, I. (2016) Alzheimer's & dementia 3, 51-62

19. Wu, J., and Li, L. (2016) Journal of biomedical research 30, 361-372

20. Saint-Aubert, L., Lemoine, L., Chiotis, K., Leuzy, A., Rodriguez-Vieitez, E., and Nordberg, A. (2017) Molecular neurodegeneration 12, 19

21. Garibotto, V., Herholz, K., Boccardi, M., Picco, A., Varrone, A., Nordberg, A., Nobili, F., Ratib, O., and Geneva Task Force for the Roadmap of Alzheimer's, B. (2017) Neurobiology of aging 52, 183-195

22. Chiotis, K., Saint-Aubert, L., Boccardi, M., Gietl, A., Picco, A., Varrone, A., Garibotto, V., Herholz, K., Nobili, F., Nordberg, A., and Geneva Task Force for the Roadmap of Alzheimer's, B. (2017) Neurobiology of aging 52, 214-227

23. Andreasen, N., Sjogren, M., and Blennow, K. (2003) The world journal of biological psychiatry : the official journal of the World Federation of Societies of Biological Psychiatry 4, 147-155

24. Hu, W. T., Watts, K. D., Shaw, L. M., Howell, J. C., Trojanowski, J. Q., Basra, S., Glass, J. D., Lah, J. J., and Levey, A. I. (2015) Annals of clinical and translational neurology 2, 131-139

25. Lewczuk, P., Lelental, N., Spitzer, P., Maler, J. M., and Kornhuber, J. (2015) Journal of Alzheimer's disease : JAD 43, 183-191

26. Elson, E. L. (2001) Traffic 2, 789-796

27. Vukojevic, V., Pramanik, A., Yakovleva, T., Rigler, R., Terenius, L., and Bakalkin, G. (2005) Cellular and molecular life sciences : CMLS 62, 535-550

28. Rigler, R. (2010) Biochemical and biophysical research communications 396, 170-175

29. Elson, E. L. (2011) Biophysical journal 101, 2855-2870

30. Elson, E. L. (2013) Methods in enzymology 518, 11-41

31. Rigler, R., and Widengren, J. (2017) European biophysics journal: EBJ

32. Elson, E. L. (2017) Methods

33. Vukojevic, V., Heidkamp, M., Ming, Y., Johansson, B., Terenius, L., and Rigler, R. (2008) Proceedings of the National Academy of Sciences of the United States of America 105, 18176-18181

34. Chauhan, A., Pirttila, T., Chauhan, V. P., Mehta, P., and Wisniewski, H. M. (1998) Journal of the neurological sciences 154, 159-163

35. Griffin, M. D., Wilson, L. M., Mok, Y. F., Januszewski, A. S., Wilson, A. M., Karschimkus, C. S., Romas, E., Lee, A. B., Godfrey, T., Wong, M., Clemens, L., Jenkins, A. J., and Howlett, G. J. (2010) Clinical biochemistry 43, 278-286

36. Tapiola, T., Alafuzoff, I., Herukka, S. K., Parkkinen, L., Hartikainen, P., Soininen, H., and Pirttila, T. (2009) Archives of neurology 66, 382-389

37. Sole-Domenech, S., Sjovall, P., Vukojevic, V., Fernando, R., Codita, A., Salve, S., Bogdanovic, N., Mohammed, A. H., Hammarstrom, P., Nilsson, K. P., LaFerla, F. M., Jacob, S., Berggren, P. O., Gimenez-Llort, L., Schalling, M., Terenius, L., and Johansson, B. (2013) Acta neuropathologica 125, 145-157

38. Horrocks, M. H., Lee, S. F., Gandhi, S., Magdalinou, N. K., Chen, S. W., Devine, M. J., Tosatto, L., Kjaergaard, M., Beckwith, J. S., Zetterberg, H., Iljina, M., Cremades, N., Dobson, C. M., Wood, N. W., and Klenerman, D. (2016) ACS chemical neuroscience 7, 399-406

39. Groenning, M. (2010) Journal of chemical biology 3, 1-18

40. Mathis, C. A., Mason, N. S., Lopresti, B. J., and Klunk, W. E. (2012) Seminars in nuclear medicine 42, 423-432

41. Svedberg, M. M., Rahman, O., and Hall, H. (2012) Nuclear medicine and biology 39, 484-501

42. Svedberg, M. M., Hellström-Lindahl, E., Rahman, O., and Hall, H. (2012) Amyloid Imaging PET Ligands as Biomarkers for Alzheimer's Disease, Preclinical Evaluation. in Positron Emission Tomography - Current Clinical and Research Aspects (Hsieh, C.-H. ed., InTech, Rijeka, Croatia

43. Cohen, S. I., Linse, S., Luheshi, L. M., Hellstrand, E., White, D. A., Rajah, L., Otzen, D. E., Vendruscolo, M., Dobson, C. M., and Knowles, T. P. (2013) Proceedings of the National Academy of Sciences of the United States of America 110, 9758-9763

44. Lindgren, M., and Hammarstrom, P. (2010) The FEBS journal 277, 1380-1388

45. Chang, L., Rissin, D. M., Fournier, D. R., Piech, T., Patel, P. P., Wilson, D. H., and Duffy, D. C. (2012) Journal of immunological methods 378, 102-115

46. Rissin, D. M., Kan, C. W., Campbell, T. G., Howes, S. C., Fournier, D. R., Song, L., Piech, T., Patel, P. P., Chang, L., Rivnak, A. J., Ferrell, E. P., Randall, J. D., Provuncher, G. K., Walt, D. R., and Duffy, D. C. (2010) Nature biotechnology 28, 595-599

47. Holtta, M., Hansson, O., Andreasson, U., Hertze, J., Minthon, L., Nagga, K., Andreasen, N., Zetterberg, H., and Blennow, K. (2013) PloS one 8, e66381

# EP 3 775 884 B1

48. Andreasson, U., Blennow, K., and Zetterberg, H. (2016) Alzheimer's & dementia 3, 98-102

49. Nakamura, A., Kaneko, N., Villemagne, V. L., Kato, T., Doecke, J., Dore, V., Fowler, C., Li, Q. X., Martins, R., Rowe, C., Tomita, T., Matsuzaki, K., Ishii, K., Ishii, K., Arahata, Y., Iwamoto, S., Ito, K., Tanaka, K., Masters, C. L., and Yanagisawa, K. (2018) Nature 554, 249-254

50. Reverberi, R., and Reverberi, L. (2007) Blood transfusion = Trasfusione del sangue 5, 227-240

51. Schiettecatte, J., Anckaert, E., and Smitz, J. (2012) Interferences in Immunoassays. in Advances in Immunoassay Technology (Chiu, N. H. L., and Christopoulos, T. K. eds.), InTech, Rijeka, Croatia

52. Saiki, H. (2012) Detection Curb. in Trends in Immunolabelled and Related Techniques (Abuelzein, E. ed., InTech, Rijeka, Croatia

53. Klaver, A. C., Patrias, L. M., Finke, J. M., and Loeffler, D. A. (2011) Journal of neuroscience methods 195, 249-254

54. Hunter, S., and Brayne, C. (2017) Journal of negative results in biomedicine 16, 1

55. Lundstrom, S. L., Zhang, B., Rutishauser, D., Aarsland, D., and Zubarev, R. A. (2017) Scientific reports 7, 41929

56. Hirsch, J., Hansen, K. C., Burlingame, A. L., and Matthay, M. A. (2004) American journal of physiology. Lung cellular and molecular physiology 287, L1-23

57. Portelius, E., Brinkmalm, G., Pannee, J., Zetterberg, H., Blennow, K., Dahlen, R., Brinkmalm, A., and Gobom, J. (2017) Expert review of proteomics 14, 1007-1020

58. Lin, Y. T., Cheng, J. T., Yao, Y. C., Juo, Lo, Y. K., Lin, C. H., Ger, L. P., and Lu, P. J. (2009) Journal of Alzheimer's disease : JAD 18, 907-918

59. Janelidze, S., Stomrud, E., Palmqvist, S., Zetterberg, H., van Westen, D., Jeromin, A., Song, L., Hanlon, D., Tan Hehir, C. A., Baker, D., Blennow, K., and Hansson, O. (2016) Scientific reports 6, 26801

60. Nitsch, R. M., Rebeck, G. W., Deng, M., Richardson, U. I., Tennis, M., Schenk, D. B., Vigo-Pelfrey, C., Lieberburg, I., Wurtman, R. J., Hyman, B. T., and et al. (1995) Annals of neurology 37, 512-518

61. Vital M, Bronzi D, Krmpot AJ, Nikolic SN, Schmitt F-J, Junghans C, Tisa S, Friedrich T, Vukojevic V, Terenius L, Zappa F, Rigler R, A single-photon avalanche camera for fluorescence lifetime imaging microscopy and correlation spectroscopty. IEEE J. Sel. Top. Quantum Electron. 2014 20:344-353

62 M J Levene, J Korlach, S W Turner, M Foquet, H G Craighead and W W Webb, Science vol. 299, No. 5607 (Jan. 31, 2003) pp. 682-686).

## Claims

1. A method for the diagnosis and/or prognosis of an amyloid-associated disease in a subject, the amyloid-associated disease being associated with individual amyloid aggregates,

   wherein the method comprises the steps of:

   • adding a detection agent to a sample of bodily fluid from the subject, the detection agent binding to and/or associating with and/or reacting with individual amyloid aggregates;
   • determining the presence and/or amount and/or concentration and/or size of individual amyloid aggregates in the sample;

   wherein determining the presence and/or amount and/or concentration and/or size of individual amyloid aggregates in the sample comprises an analysis based on time-resolved detection comprising fluorescence correlation spectroscopy (FCS) and fluorescence intensity fluctuation analysis (FIFA), and wherein the method does not comprise amplification of individual amyloid aggregates.

2. The method according to claim 1, wherein the method does not necessitate the use of non-target amyloid molecules/peptides/proteins/aggregates (amyloid entities) or the inclusion of a step comprising mixing target amyloid molecules/peptides/proteins/aggregates and non-target amyloid molecules/aggregates.

3. The method according to claim 1 or 2, wherein the bodily fluid is one or more from the group consisting of: blood and/or cerebrospinal fluid (CSF) and/or saliva and/or urine and/or synovial fluid and/or faeces, wherein the blood sample is preferably one or more from the group consisting of: whole blood and/or blood serum and/or blood plasma and/or cell lysates and/or erythrocytes, leukocytes and/or thrombocytes, wherein the bodily fluid is more preferably selected from the group consisting of blood plasma and/or blood serum, and wherein the bodily fluid is even more preferably selected from blood serum.

4. The method according to any one of the preceding claims, wherein the size profile of amyloid molecules/particles/aggregates can be determined for each subject.

5. The method according to any one of claims 3 to 4, wherein the blood sample does not comprise one or more from the group consisting of: flavin adenine dinucleotide (FAD) enzymes and/or FAD lipopigments; and/or pyridinic (NADH) enzymes and/or NADH lipopigments.

6. The method according to any one of the preceding claims, wherein

    (i) the detection agent comprises a fluorescent moiety which emits fluorescence when it binds to and/or associates with and/or reacts with individual amyloid aggregates, and/or wherein
    (ii) the detection agent is one or more from the group consisting of: Thioflavin T (ThT) and/or ARCAM1 and/or pentameric formyl thiophene acetic acid (pFTAA) and/or 4-(dicyanovinyl)- julolidine (DCVJ) and/or 1-amino-8-naphtalene sulphonate (ANS) and/or bis-ANS and/or 2-[N-bis-(3-dimethylaminopropyl)-amino]-4-[2,3-dihydro-3-methyl-(benzo-1, 3-thiazol-2-yl)-methylidene]-1-phenyl-quinolinium (PicoGreen).

7. The method according to any one of the preceding claims, wherein

    (i) the individual amyloid aggregate is selected from the group comprising: an amyloid oligomer; an amyloid nano-aggregate; and/or an amyloid senile aggregate; and/or an amyloid fibril; and/or an amyloid protofibril, wherein the senile amyloid aggregate preferably comprises more than 1,000,000 proteins or peptides, and/or wherein
    (ii) the individual amyloid aggregate comprises about 20 or more peptides; for example: about 30 or more; about 40 or more; about 50 or more; about 60 or more; about 70 or more; about 80 or more; about 90 or more; or about 100 or more peptides, preferably about 40 or more peptides.

8. The method according to claim 7, wherein the amyloid nano-aggregate comprises about 20 proteins or peptides to 100,000 proteins or peptides.

9. The method according to any one of the preceding claims, wherein the individual amyloid aggregates comprise one or more beta ($\beta$)-sheets and that the detection agent binds to and/or associates with and/or reacts with individual amyloid aggregates comprising one or more beta ($\beta$)-sheet.

10. The method according to any one of the preceding claims, wherein the amyloid-associated disease is one or more selected from the group comprising: Alzheimer's disease; and/or Parkinson's disease; and/or Huntington's disease; and/or amyotrophic lateral sclerosis (ALS); and/or type 2 diabetes; and/or systemic amyloidosis; and/or a prion disease (such as Creutzfeldt-Jakob disease (CJD)); and/or amyloid light-chain amyloidosis; and/or multiple myeloma; and/or inflammation; and/or kidney failure; and/or Leukocyte Chemotactic Factor 2 (LECT2) amyloidosis; and/or transthyretin-related hereditary amyloidosis with polyneuropathy (also known as Familial Amyloid Polyneuropathy (FAP) or Skelleftesjukan); and/or haemodialysis-associated amyloidosis; and/or Cerebral amyloid angiopathy; and/or familial visceral amyloidosis; and/or primary cutaneous amyloidosis; and/or cerebral amyloid angiopathy; and/or prolactinoma; and/or familial corneal amyloidosis; and/or senile amyloid of the atria of heart; and/or medullary carcinoma of the thyroid; and/or pharmaceutical insulin-derived amyloidosis, wherein the amyloid-associated disease preferably is Alzheimer's disease, and the individual amyloid aggregates preferably comprise an amyloid beta ($\beta$) peptide ($\beta$ peptide), for example $\beta40$ and/or $\beta42$ and/or $\beta43$, and wherein the Alzheimer's disease preferably is selected from the group comprising: Subjective Cognitive Impairment; Mild Cognitive impairment; Alzheimer's disease; and Alzheimer's disease with vascular dementia.

11. The method according to any one of the preceding claims, wherein determining the presence and/or amount and/or concentration and/or size of individual amyloid aggregates is at an individual amyloid aggregate resolution.

12. The method according to any one of the preceding claims, wherein a diagnosis and/or prognosis of the amyloid-associated disease is provided if the presence and/or amount and/or concentration of the individual amyloid aggregates in the sample is higher than the presence and/or amount and/or concentration of the individual amyloid aggregates in a control sample; and/or the size of the individual amyloid aggregates in the sample is larger than the size of the individual amyloid aggregates in a control sample.

13. A method for identifying an agent for the treatment of an amyloid-associated disease, wherein the method comprises the steps of:

• determining the presence and/or amount and/or concentration and/or size of individual amyloid aggregates in a first sample of bodily fluid that has been obtained from a test subject;
• determining the presence and/or amount and/or concentration and/or size of individual amyloid aggregates in a second sample of bodily fluid that has been obtained from the test subject after having administered the agent to the subject;
• comparing the determination of the presence and/or amount and/or concentration and/or size of individual amyloid aggregates in the first sample with the determination of presence and/or amount and/or concentration and/or size of individual amyloid aggregates in the second sample;
• identifying the agent as being for the treatment of the amyloid-associated disease based on the comparison;

wherein the amyloid-associated disease is associated with individual amyloid aggregates, the method further comprises the addition of a detection agent to the sample, the detection agent binding to and/or associating with and/or reacting with individual amyloid aggregates, the determination of the presence and/or amount and/or concentration and/or size of individual amyloid aggregates in the sample comprises an analysis based on time-resolved detection comprising fluorescence correlation spectroscopy (FCS) and fluorescence intensity fluctuation analysis (FIFA), and wherein the method does not comprise amplification of individual amyloid aggregates.

14. A method for identifying an agent for the treatment of an amyloid-associated disease, wherein the method comprises the steps of:

• determining the presence and/or amount and/or concentration and/or size of individual amyloid aggregates in a first sample that has been taken from a test fluid;
• determining the presence and/or amount and/or concentration and/or size of individual amyloid aggregates in a second sample that has been taken from the test fluid after having administered the agent to the fluid;
• comparing the determination of the presence and/or amount and/or concentration and/or size of individual amyloid aggregates in the first sample with the determination of presence and/or amount and/or concentration and/or size of individual amyloid aggregates in the second sample;
• identifying the agent as being for the treatment of the amyloid-associated disease based on the comparison;

wherein the amyloid-associated disease is associated with individual amyloid aggregates, the method further comprises the addition of a detection agent to the sample, the detection agent binding to and/or associating with and/or reacting with individual amyloid aggregates, the determination of the presence and/or amount and/or concentration and/or size of individual amyloid aggregates in the sample comprises an analysis based on fluorescence correlation spectroscopy (FCS) and fluorescence intensity fluctuation analysis (FIFA), and wherein the method does not comprise amplification of individual amyloid aggregate.

**Patentansprüche**

1. Verfahren zur Diagnose und/oder Prognose einer Amyloid-assoziierten Krankheit in einem Subjekt, wobei die Amyloid-assoziierte Krankheit mit einzelnen Amyloidaggregaten assoziiert ist,

wobei das Verfahren die folgenden Schritte umfasst:

• Zugeben eines Detektionsmittels zu einer Probe aus Körperflüssigkeit von dem Subjekt, wobei das Detektionsmittel an einzelne Amyloidaggregate bindet und/oder mit ihnen assoziiert und/oder mit ihnen reagiert;
• Bestimmen des Vorhandenseins und/oder der Menge und/oder der Konzentration und/oder der Größe einzelner Amyloid-Aggregate in der Probe;

wobei das Bestimmen des Vorhandenseins und/oder der Menge und/oder der Konzentration und/oder der Größe einzelner Amyloidaggregate in der Probe eine Analyse auf der Grundlage einer zeitaufgelösten Detektion umfasst, die Fluoreszenz-Korrelationsspektroskopie (FCS) und Fluoreszenz-Intensitätsfluktuationsanalyse (FIFA) umfasst, und wobei das Verfahren nicht eine Amplifikation einzelner Amyloidaggregate umfasst.

2. Verfahren nach Anspruch 1, wobei das Verfahren nicht die Verwendung von Nicht-Ziel-Amyloid-Molekülen/Peptiden/Proteinen/Aggregaten (Amyloid-Einheiten) oder die Einbeziehung eines Schritts erfordert, der ein Mischen von Ziel-Amyloid-Molekülen/Peptiden/Proteinen/Aggregaten und Nicht-Ziel Amyloidmolekülen/Aggregaten umfasst.

3. Verfahren nach Anspruch 1 oder 2, wobei die Körperflüssigkeit eine oder mehrere aus der Gruppe bestehend aus den Folgenden ist: Blut und/oder zerebrospinale Flüssigkeit (CSF) und/oder Speichel und/oder Urin und/oder Synovialflüssigkeit und/oder Fäkalien, wobei die Blutprobe vorzugsweise eine oder mehrere aus der Gruppe bestehend aus den Folgenden ist: Vollblut und/oder Blutserum und/oder Blutplasma und/oder Zelllysaten und/oder Erythrozyten, Leukozyten und/oder Thrombozyten, wobei die Körperflüssigkeit stärker bevorzugt ausgewählt ist aus der Gruppe bestehend aus Blutplasma und/oder Blutserum, und wobei die Körperflüssigkeit noch stärker bevorzugt aus Blutserum ausgewählt ist.

4. Verfahren nach einem der vorhergehenden Ansprüche, wobei das Größenprofil der Amyloid-Moleküle/Partikel/Aggregate für jedes Subjekt bestimmt werden kann.

5. Verfahren nach einem der Ansprüche 3 bis 4, wobei die Blutprobe nicht eines oder mehrere aus der Gruppe bestehend aus den Folgenden umfasst: Flavin-Adenin-Dinukleotid (FAD)-Enzyme und/oder FAD-Lipopigmenten; und/oder Pyridin-(NADH)-Enzyme und/oder NADH-Lipopigmenten.

6. Verfahren nach einem der vorhergehenden Ansprüchen, wobei

(i) das Detektionsmittel eine fluoreszierende Komponente umfasst, die Fluoreszenz emittiert, wenn sie an einzelne Amyloidaggregate bindet und/oder mit ihnen assoziiert und/oder mit ihnen reagiert, und/oder wobei
(ii) das Detektionsmittel eines oder mehrere aus der Gruppe bestehend aus Folgenden ist: Thioflavin T (ThT) und/oder ARCAM1 und/oder pentamerische Formylthiophenessigsäure (pFTAA) und/oder 4-(Dicyanvinyl)-julolidin (DCVJ) und/oder 1-Amino-8-naphtalensulfonat (ANS) und/oder Bis-ANS und/oder 2-[N-Bis-(3-dimethylaminpropyl)-amin]-4-[2,3-dihydro-3-methyl-(benzo-1,3-thiazol-2- yl)-methyliden]-1-phenyl-quinolinium (Pico-Green).

7. Verfahren nach einem der vorhergehenden Ansprüche, wobei

(i) das einzelne Amyloidaggregat ausgewählt ist aus der Gruppe, umfassend: ein Amyloid-Oligomer; ein Amyloid-Nanoaggregat; und/oder ein seniles Amyloidaggregat; und/oder eine Amyloid-Fibrille; und/oder eine Amyloid-Protofibrille, wobei das senile Amyloidaggregat vorzugsweise mehr als 1.000.000 Proteine oder Peptide umfasst, und/oder wobei
(ii) das einzelne Amyloidaggregat etwa 20 oder mehr Peptide umfasst, beispielsweise: etwa 30 oder mehr; etwa 40 oder mehr; etwa 50 oder mehr; etwa 60 oder mehr; etwa 70 oder mehr; etwa 80 oder mehr; etwa 90 oder mehr; oder etwa 100 oder mehr Peptide, vorzugsweise etwa 40 oder mehr Peptide.

8. Verfahren nach Anspruch 7, wobei das Amyloid-Nanoaggregat etwa 20 Proteine oder Peptide bis 100.000 Proteine oder Peptide umfasst.

9. Verfahren nach einem der vorhergehenden Ansprüche, wobei die einzelnen Amyloidaggregate ein oder mehrere beta-($\beta$)-Blätter umfassen und dass das Detektionsmittel an einzelne Amyloidaggregate, die ein oder mehrere beta-($\beta$)-Blätter umfassen, bindet und/oder mit ihnen assoziiert und/oder mit ihnen reagiert.

10. Verfahren nach einem der vorhergehenden Ansprüche, wobei die Amyloid-assoziierte Krankheit eine oder mehrere ausgewählt ist aus der Gruppe umfassend: Alzheimer-Krankheit; und/oder Parkinson-Krankheit; und/oder Huntington-Krankheit; und/oder amyotrophe Lateralsklerose (ALS); und/oder Typ-2-Diabetes; und/oder systemische Amyloidose; und/oder eine Prionenkrankheit (wie Creutzfeldt-Jakob-Krankheit (CJD)); und/oder Amyloid-Leichtketten-Amyloidose; und/oder multiples Myelom; und/oder Entzündung; und/oder Nierenversagen; und/oder Leukozyten-Chemotaktischer Faktor 2-(LECT2)-Amyloidose; und/oder Transthyretin-bezogene hereditäre Amyloidose mit Polyneuropathie (auch bekannt als Familiäre Amyloid-Polyneuropathie (FAP) oder Skelleftesjukan); und/oder Hämodialyse-assoziierte Amyloidose; und/oder zerebrale Amyloidangiopathie; und/oder familiäre viszerale Amyloidose; und/oder primäre kutane Amyloidose; und/oder zerebrale Amyloidangiopathie; und/oder Prolaktinom; und/oder familiäre Homhautamyloidose; und/oder seniles Amyloid der Vorhöfe des Herzens; und/oder medulläres Karzinom der Schilddrüse; und/oder pharmazeutische Insulinabgeleitete Amyloidose, wobei die Amyloid-assoziierte Krankheit vorzugsweise eine Alzheimer-Krankheit ist und die einzelnen Amyloid-Aggregate vorzugsweise ein Amyloid-beta-(β)-Peptid (β-Peptid), beispielsweise β40 und/oder β42 und/oder β43, umfassen, und wobei die Alzheimer-Krankheit vorzugsweise ausgewählt ist aus der Gruppe umfassend: Subjektive kognitive Beeinträchtigung; leichte kognitive Beeinträchtigung; Alzheimer-Krankheit; und Alzheimer-Krankheit mit vaskulärer Demenz.

**11.** Verfahren nach einem der vorhergehenden Ansprüche, wobei das Bestimmen des Vorhandenseins und/oder der Menge und/oder der Konzentration und/oder der Größe einzelner Amyloidaggregate bei einer individuellen Amyloidaggregatauflösung erfolgt.

**12.** Verfahren nach einem der vorhergehenden Ansprüche, wobei eine Diagnose und/oder Prognose der Amyloid-assoziierten Krankheit bereitgestellt wird, wenn das Vorhandensein und/oder die Menge und/oder die Konzentration der einzelnen Amyloidaggregate in der Probe höher ist als das Vorhandensein und/oder die Menge und/oder die Konzentration der einzelnen Amyloidaggregate in einer Kontrollprobe; und/oder die Größe der einzelnen Amyloidaggregate in der Probe größer ist als die Größe der einzelnen Amyloidaggregate in einer Kontrollprobe.

**13.** Verfahren zum Identifizieren eines Mittels für die Behandlung einer Amyloid-assoziierten Krankheit, wobei das Verfahren die folgenden Schritte umfasst:

• Bestimmen des Vorhandenseins und/oder der Menge und/oder der Konzentration und/oder der Größe einzelner Amyloid-Aggregate in einer ersten Probe aus Körperflüssigkeit, die von einem Subjekt erhalten wurde;
• Bestimmen des Vorhandenseins und/oder der Menge und/oder der Konzentration und/oder der Größe einzelner Amyloidaggregate in einer zweiten Probe aus Körperflüssigkeit, die von dem Subjekt erhalten wurde, nachdem das Mittel an das Subjekt verabreicht wurde;
• Vergleichen der Bestimmung des Vorhandenseins und/oder der Menge und/oder der Konzentration und/oder der Größe einzelner Amyloidaggregate in der ersten Probe mit der Bestimmung eines Vorhandenseins und/oder einer Menge und/oder einer Konzentration und/oder einer Größe einzelner Amyloidaggregate in der zweiten Probe;
• Identifizieren des Mittels als Mittel zur Behandlung der Amyloid-assoziierten Krankheit auf der Grundlage des Vergleichs;

wobei die Amyloid-assoziierte Krankheit mit einzelnen Amyloid-Aggregaten assoziiert ist, das Verfahren ferner die Zugabe eines Detektionsmittels zu der Probe umfasst, wobei das Detektionsmittel an einzelne Amyloid-Aggregate bindet und/oder mit ihnen assoziiert und/oder mit ihnen reagiert, die Bestimmung des Vorhandenseins und/oder der Menge und/oder der Konzentration und/oder der Größe einzelner Amyloid-Aggregate in der Probe eine Analyse auf der Grundlage einer zeitaufgelösten Detektion umfasst, die Fluoreszenz-Korrelationsspektroskopie (FCS) und Fluoreszenz-Intensitätsfluktuationsanalyse (FIFA) umfasst, und wobei das Verfahren nicht eine Amplifikation einzelner Amyloidaggregate umfasst.

**14.** Verfahren zum Identifizieren eines Mittels für die Behandlung einer Amyloid-assoziierten Krankheit, wobei das Verfahren die folgenden Schritte umfasst:

• Bestimmen des Vorhandenseins und/oder der Menge und/oder der Konzentration und/oder der Größe einzelner Amyloid-Aggregate in einer ersten Probe, die aus einer Testflüssigkeit entnommen wurde;
• Bestimmen des Vorhandenseins und/oder der Menge und/oder der Konzentration und/oder der Größe einzelner Amyloid-Aggregate in einer zweiten Probe, die der Testflüssigkeit entnommen wurde, nachdem das Mittel der Flüssigkeit verabreicht wurde;
• Vergleichen der Bestimmung des Vorhandenseins und/oder der Menge und/oder der Konzentration und/oder der Größe einzelner Amyloidaggregate in der ersten Probe mit der Bestimmung eines Vorhandenseins und/oder einer Menge und/oder einer Konzentration und/oder einer Größe einzelner Amyloidaggregate in der zweiten Probe;
• Identifizieren des Mittels als Mittel zur Behandlung der Amyloid-assoziierten Krankheit auf der Grundlage des Vergleichs;

wobei die Amyloid-assoziierte Krankheit mit einzelnen Amyloid-Aggregaten assoziiert ist, das Verfahren ferner die Zugabe eines Detektionsmittels zu der Probe umfasst, wobei das Detektionsmittel an einzelne Amyloid-Aggregate bindet und/oder mit ihnen assoziiert und/oder mit ihnen reagiert, die Bestimmung des Vorhandenseins und/oder der Menge und/oder der Konzentration und/oder der Größe einzelner Amyloid-Aggregate in der Probe eine Analyse auf der Grundlage von Fluoreszenz-Korrelationsspektroskopie (FCS) und Fluoreszenz-Intensitätsfluktuationsanalyse (FIFA) umfasst, und wobei das Verfahren nicht eine Amplifikation einzelner Amyloid-Aggregate umfasst.

**Revendications**

1. Procédé pour le diagnostic et/ou le pronostic d'une maladie associée à l'amyloïde chez un sujet, la maladie associée à l'amyloïde étant associée à des agrégats amyloïdes individuels,

   où le procédé comprend les étapes de:

   • addition d'un agent de détection à un échantillon de liquide corporel provenant du sujet, l'agent de détection se liant à et/ou s'associant avec et/ou réagissant avec des agrégats amyloïdes individuels;
   • détermination de la présence et/ou la quantité et/ou la concentration et/ou la taille d'agrégats amyloïdes individuels dans l'échantillon;

   où la détermination de la présence et/ou la quantité et/ou la concentration et/ou la taille d'agrégats amyloïdes individuels dans l'échantillon comprend une analyse basée sur la détection résolue dans le temps comprenant la spectroscopie de corrélation de fluorescence (SCF) et l'analyse des fluctuations de l'intensité de fluorescence (AFIF), et où le procédé ne comprend pas l'amplification d'agrégats amyloïdes individuels.

2. Procédé selon la revendication 1, où le procédé ne nécessite pas l'utilisation de molécules/peptides/protéines/agrégats amyloïdes (entités amyloïdes) non cibles ou l'inclusion d'une étape comprenant le mélange de molécules/peptides/protéines/agrégats amyloïdes cibles et de molécules/agrégats amyloïdes non cibles.

3. Procédé selon la revendication 1 ou 2, où le liquide corporel est un ou plusieurs du groupe consistant en: le sang et/ou le liquide céphalo-rachidien (LCR) et/ou la salive et/ou l'urine et/ou le liquide synovial et/ou les selles, où l'échantillon de sang est de préférence un ou plusieurs du groupe consistant en: le sang total et/ou le sérum sanguin et/ou le plasma sanguin et/ou les lysats cellulaires et/ou les érythrocytes, les leucocytes et/ou les thrombocytes, où le liquide corporel est de préférence encore choisi dans le groupe consistant en le plasma sanguin et/ou le sérum sanguin, et où le liquide corporel est choisi de manière encore préférable parmi le sérum sanguin.

4. Procédé selon l'une quelconque des revendications précédentes, où le profil de taille des molécules/particules/agrégats amyloïdes peut être déterminé pour chaque sujet.

5. Procédé selon l'une quelconque des revendications 3 à 4, où l'échantillon de sang ne comprend pas un ou plusieurs du groupe consistant en: les enzymes flavine adénine dinucléotide (FAD) et/ou les lipopigments FAD; et/ou les enzymes pyridiniques (NADH) et/ou les lipopigments NADH.

6. Procédé selon l'une quelconque des revendications précédentes, où

   (i) l'agent de détection comprend un fragment fluorescent qui émet une fluorescence lorsqu'il se lie à et/ou s'associe avec et/ou réagit avec des agrégats amyloïdes individuels,
   et/ou où
   (ii) l'agent de détection est un ou plusieurs du groupe consistant en: la thioflavine T (ThT) et/ou l'ARCAM1 et/ou l'acide formyl thiophène acétique pentamérique (pFTAA) et/ou la 4-(dicyanovinyl)-julolidine (DCVJ) et/ou le 1-amino-8-naphtalène sulfonate (ANS) et/ou le bis-ANS et/ou le 2-[N-bis-(3-diméthylaminopropyl)-amino]-4-[2,3-dihydro-3-méthyl-(benzo-1,3-thiazol-2-yl)-méthylidène]-1-phényl-quinolinium (PicoGreen).

7. Procédé selon l'une quelconque des revendications précédentes, où

   (i) l'agrégat amyloïde individuel est choisi dans le groupe comprenant: un oligomère amyloïde; un nano-agrégat amyloïde; et/ou un agrégat sénile amyloïde; et/ou une fibrille amyloïde; et/ou une protofibrille amyloïde, où l'agrégat sénile amyloïde comprend de préférence plus de 1 000 000 de protéines ou de peptides,
   et/ou où
   (ii) l'agrégat amyloïde individuel comprend environ 20 peptides ou plus; par exemple: environ 30 ou plus; environ 40 ou plus; environ 50 ou plus; environ 60 ou plus; environ 70 ou plus; environ 80 ou plus; environ 90 ou plus; ou environ 100 peptides ou plus, de préférence environ 40 peptides ou plus.

8. Procédé selon la revendication 7, où le nano-agrégat amyloïde comprend environ 20 protéines ou peptides à 100 000 protéines ou peptides.

9. Procédé selon l'une quelconque des revendications précédentes, où les agrégats amyloïdes individuels comprennent un ou plusieurs feuillets bêta (β) et en ce que l'agent de détection se lie à et/ou s'associe avec et/ou réagit avec des agrégats amyloïdes individuels comprenant un ou plusieurs feuillets bêta (β).

10. Procédé selon l'une quelconque des revendications précédentes, où la maladie associée à l'amyloïde est une ou plusieurs choisies dans le groupe comprenant: la maladie d'Alzheimer; et/ou la maladie de Parkinson; et/ou la maladie de Huntington: et/ou la sclérose latérale amyotrophique (SLA); et/ou le diabète de type 2; et/ou l'amylose systémique; et/ou une maladie à prion (telle que la maladie de Creutzfeldt-Jakob (MCJ)); et/ou l'amylose à chaîne légère amyloïde; et/ou le myélome multiple; et/ou l'inflammation; et/ou l'insuffisance rénale; et/ou l'amylose à facteur chimiotactique leucocytaire 2 (LECT2); et/ou l'amylose héréditaire liée à la transthyrétine avec polyneuropathie (également connue sous le nom de polyneuropathie amyloïde familiale (PAF) ou Skelleftesjukan); et/ou l'amylose associée à l'hémodialyse; et/ou l'angiopathie amyloïde cérébrale; et/ou l'amylose viscérale familiale; et/ou l'amylose cutanée primitive; et/ou l'angiopathie amyloïde cérébrale; et/ou le prolactinome; et/ou l'amylose cornéenne familiale; et/ou l'amyloïde sénile des oreillettes du coeur; et/ou le carcinome médullaire de la thyroïde; et/ou une amylose pharmaceutique dérivée de l'insuline, où la maladie associée à l'amyloïde est de préférence la maladie d'Alzheimer, et les agrégats amyloïdes individuels comprennent de préférence un peptide bêta (β) amyloïde (peptide β), par exemple β40 et/ou β42 et/ou P43, et où la maladie d'Alzheimer est choisie de préférence dans le groupe comprenant: la déficience cognitive subjective; la déficience cognitive légère; la maladie d'Alzheimer; et la maladie d'Alzheimer avec démence vasculaire.

11. Procédé selon l'une quelconque des revendications précédentes, où la détermination de la présence et/ou la quantité et/ou la concentration et/ou la taille d'agrégats amyloïdes individuels est à une résolution d'agrégat amyloïde individuel.

12. Procédé selon l'une quelconque des revendications précédentes, où un diagnostic et/ou pronostic de la maladie associée à l'amyloïde est fourni si la présence et/ou la quantité et/ou la concentration des agrégats amyloïdes individuels dans l'échantillon est supérieure à la présence et/ou la quantité et/ou la concentration des agrégats amyloïdes individuels dans un échantillon témoin; et/ou la taille des agrégats amyloïdes individuels dans l'échantillon est plus grande que la taille des agrégats amyloïdes individuels dans un échantillon témoin.

13. Procédé pour identifier un agent pour le traitement d'une maladie associée à l'amyloïde, où le procédé comprend les étapes de:

• détermination de la présence et/ou la quantité et/ou la concentration et/ou la taille d'agrégats amyloïdes individuels dans un premier échantillon de liquide corporel qui a été obtenu auprès d'un sujet test;
• détermination de la présence et/ou la quantité et/ou la concentration et/ou la taille d'agrégats amyloïdes individuels dans un second échantillon de liquide corporel qui ont été obtenu auprès du sujet test après avoir administré l'agent au sujet;
• comparaison de la détermination de la présence et/ou la quantité et/ou la concentration et/ou la taille d'agrégats amyloïdes individuels dans le premier échantillon avec la détermination de la présence et/ou la quantité et/ou la concentration et/ou la taille d'agrégats amyloïdes individuels dans le second échantillon;
• identification de l'agent comme étant pour le traitement de la maladie associée à l'amyloïde sur la base de la comparaison;

où la maladie associée à l'amyloïde est associée à des agrégats amyloïdes individuels, le procédé comprend en outre l'addition d'un agent de détection à l'échantillon, l'agent de détection se liant à et/ou s'associant avec et/ou réagissant avec des agrégats amyloïdes individuels, la détermination de la présence et/ou la quantité et/ou la concentration et/ou la taille d'agrégats amyloïdes individuels dans l'échantillon comprend une analyse basée sur la détection résolue dans le temps comprenant la spectroscopie de corrélation de fluorescence (SCF) et l'analyse des fluctuations de l'intensité de fluorescence (AFIF), et où le procédé ne comprend pas l'amplification d'agrégats amyloïdes individuels.

14. Procédé pour identifier un agent pour le traitement d'une maladie associée à l'amyloïde, où le procédé comprend les étapes de:

• détermination de la présence et/ou la quantité et/ou la concentration et/ou la taille d'agrégats amyloïdes individuels dans un premier échantillon qui a été prélevé sur un liquide test;
• détermination de la présence et/ou la quantité et/ou la concentration et/ou la taille d'agrégats amyloïdes

**EP 3 775 884 B1**

individuels dans un second échantillon qui a été prélevé sur le liquide test après avoir administré l'agent au liquide;
• comparaison de la détermination de la présence et/ou la quantité et/ou la concentration et/ou la taille d'agrégats amyloïdes individuels dans le premier échantillon avec la détermination de la présence et/ou la quantité et/ou la concentration et/ou la taille d'agrégats amyloïdes individuels dans le second échantillon;
• identification de l'agent comme étant pour le traitement de la maladie associée à l'amyloïde sur la base de la comparaison;

où la maladie associée à l'amyloïde est associée à des agrégats amyloïdes individuels, le procédé comprend en outre l'addition d'un agent de détection à l'échantillon, l'agent de détection se liant à et/ou s'associant avec et/ou réagissant avec des agrégats amyloïdes individuels, la détermination de la présence et/ou la quantité et/ou la concentration et/ou la taille d'agrégats amyloïdes individuels dans l'échantillon comprend une analyse basée sur la spectroscopie de corrélation de fluorescence (SCF) et l'analyse des fluctuations de l'intensité de fluorescence (AFIF), et où le procédé ne comprend pas l'amplification d'agrégat amyloïde individuel.

Figure 1

A.

Laser
458 nm

Main Dichroic
Beamsplitter

Pinhole

Fluorescence

APD

B.

$\omega_z$

$\omega_{xy}$

C.

Fluorescence / kHz

150

100

50

0                    60

Run time / s

D.

$G(\tau)$

2.5

1.0

0.01  0.1   1    10   100

$\tau$ / ms

Figure 2

**Figure 3**

Figure 4

Figure 5

Figure 6

**Figure 7**

Figure 8

Figure 9

Figure 10

EP 3 775 884 B1

Figure 11

Figure 12

**Figure 13**

A.

B.

63

**Figure 14**

**A.**

F / kHz

**B.**

$G_n(\tau)$

Urine, without ThT
Urine, with ThT

**Figure 15**

## Figure 16

## Figure 17

EP 3 775 884 B1

Figure 18

68

## Figure 19

Figure 20

**Figure 21**

**Figure 22**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6498017 B
- WO 201604090 A1
- WO 2017004560 A

**Non-patent literature cited in the description**

- **PRINCE et al.** *World Alzheimer Report,* 2016
- Alzheimer's Disease Facts and Figures. Alzheimer's Association, 2017
- **TIIMAN et al.** *Biochemistry,* 2015, vol. 54, 7203-7211
- **GUAN et al.** *ACS Chem. Neurosci.,* 2015, vol. 6, 1503-1508
- **WENNMALM et al.** *Anal. Chem.,* 2015, vol. 87, 11700-11705
- **OH et al.** *Neuromolecular medicine.,* 2008, vol. 10 (3), 195-207
- **TOLEDO et al.** *Alzheimer's research & therapy.,* 2013, vol. 5 (2), 8
- **BURCH HB ; BESSEY OA ; LOWRY OH.** Fluorometric Measurements of Riboflavin and its Natural Derivatives in Small Quantities of Blood Serum and Cells. *J Biol Chem.,* 1948, vol. 175 (1), 457-470
- **NICHOLS et al.** *Biochemistry,* 2015, vol. 54 (13), 2193-2204
- **BYRON ; VESTERGAARD.** *Curr. Opin. Struct. Biol.,* 2015, vol. 35, 76-86
- **NICHOLS MR ; COLVIN BA ; HOOD EA ; PARANJAPE GS ; OSBORN DC ; TERRILL-USERY SE.** Biophysical comparison of soluble amyloid-β(1-42) protofibrils, oligomers, and protofilaments. *Biochemistry,* 2015, vol. 54 (13), 2193-2204
- **BYRON O ; VESTERGAARD B.** Protein-protein interactions: a supra-structural phenomenon demanding trans-disciplinary biophysical approaches. *Curr. Opin. Struct. Biol.,* 2015, vol. 35, 76-86
- Amyloid like protofibrils with different physical properties. **RELINI A.** Bio-nanoimaging: Protein Misfolding and Aggregation. 2013
- **GROENNING, M.** Binding mode of Thioflavin T and other molecular probes in the context of amyloid fibrils-current status. *Journal of Chemical Biology,* 2010, vol. 3, 1-18
- **GUAN et al.** *ACS Chemical Neuroscience,* 2015, vol. 6, 1503-1508
- **HEI-NGA CHAN ; DI XU ; SEE-LOK HO ; MAN SHING WONG.** ORCID logo* and Hung-Wing Li, Ultra-sensitive detection of protein biomarkers for diagnosis of Alzheimer's disease. *Chem. Sci.,* 2017, vol. 8, 4012-4018
- **BRELSTAFF et al.** *Front Neurosci.,* 29 May 2015, vol. 9, 184
- **MUELLER et al.** *Biophys. J.,* 2011, vol. 101 (7), 1651-1660
- **SEVIGNY et al.** *Nature,* 2016, vol. 537, 50-56
- **M J LEVENE ; J KORLACH ; S W TURNER ; M FOQUET ; H G CRAIGHEAD ; W W WEBB.** *Science,* 31 January 2003, vol. 299 (5607), 682-686
- **VITAL M ; BRONZI D ; KRMPOT AJ ; NIKOLIC SN ; SCHMITT F-J ; JUNGHANS C ; TISA S ; FRIEDRICH T ; VUKOJEVIC V ; TERENIUS L.** A single-photon avalanche camera for fluorescence lifetime imaging microscopy and correlation spectroscopty. *IEEE J. Sel. Top. Quantum Electron.,* 2014, vol. 20, 344-353
- **PRINCE, M. ; COMAS-HERRERA, A. ; KNAPP, M. ; GUERCHET, M. ; KARAGIANNIDOU, M.** *World Alzheimer Report 2016. London, UK,* 2016
- Alzheimer's Disease Facts and Figures. Alzheimer's & dementia. Alzheimer's Association, 2017
- **SELKOE, D. J. ; HARDY, J.** *EMBO molecular medicine,* 2016, vol. 8, 595-608
- **WALLIN, C. ; LUO, J. ; JARVET, J. ; WÄRMLÄNDER, K. T. S. ; GRÄSLUND, A.** *Israel J. Chem.,* 2017, vol. 57
- **TIIMAN, A. ; JARVET, J. ; GRASLUND, A. ; VUKOJEVIC, V.** *Biochemistry,* 2015, vol. 54, 7203-7211
- **NAIKI, H. ; HIGUCHI, K. ; HOSOKAWA, M. ; TAKEDA, T.** *Analytical biochemistry,* 1989, vol. 177, 244-249
- **LEVINE, H., 3RD.** *Methods in enzymology,* 1999, vol. 309, 274-284
- **BIANCALANA, M. ; KOIDE, S.** *Biochimica et biophysica acta,* 2010, vol. 1804, 1405-1412

- WOLFE, L. S. ; CALABRESE, M. F. ; NATH, A. ; BLAHO, D. V. ; MIRANKER, A. D. ; XIONG, Y. *Proceedings of the National Academy of Sciences of the United States of America,* 2010, vol. 107, 16863-16868
- FREIRE, S. ; DE ARAUJO, M. H. ; AL-SOUFI, W. ; NOVO, M. *Dyes Pigm.,* 2014, vol. 110, 97-105
- SINGH, P. K. ; MORA, A. K. ; NATH, S. *Chemical communications,* 2015, vol. 51, 14042-14045
- KUZNETSOVA, I. M. ; SULATSKAYA, A. I. ; MASKEVICH, A. A. ; UVERSKY, V. N. ; TUROVEROV, K. K. *Analytical chemistry,* 2016, vol. 88, 718-724
- OH, E. S. ; TRONCOSO, J. C. ; FANGMARK TUCKER, S. M. *Neuromolecular medicine,* 2008, vol. 10, 195-207
- TOLEDO, J. B. ; SHAW, L. M. ; TROJANOWSKI, J. Q. *Alzheimer's research & therapy,* 2013, vol. 5, 8
- O'BRYANT, S. E. ; MIELKE, M. M. ; RISSMAN, R. A. ; LISTA, S. ; VANDERSTICHELE, H. ; ZETTERBERG, H. ; LEWCZUK, P. ; POSNER, H. ; HALL, J. ; JOHNSON, L. *Alzheimer's & dementia : the journal of the Alzheimer's Association,* 2017, vol. 13, 45-58
- BLENNOW, K. *Neurology and therapy,* 2017, vol. 6, 15-24
- COUNTS, S. E. ; IKONOMOVIC, M. D. ; MERCADO, N. ; VEGA, I. E. ; MUFSON, E. J. *Neurotherapeutics : the journal of the American Society for Experimental NeuroTherapeutics,* 2017, vol. 14, 35-53
- DEMARSHALL, C. A. ; NAGELE, E. P. ; SARKAR, A. ; ACHARYA, N. K. ; GODSEY, G. ; GOLDWASER, E. L. ; KOSCIUK, M. ; THAYASIVAM, U. ; HAN, M. ; BELINKA, B. *Alzheimer's & dementia,* 2016, vol. 3, 51-62
- WU, J. ; LI, L. *Journal of biomedical research,* 2016, vol. 30, 361-372
- SAINT-AUBERT, L. ; LEMOINE, L. ; CHIOTIS, K. ; LEUZY, A. ; RODRIGUEZ-VIEITEZ, E. ; NORDBERG, A. *Molecular neurodegeneration,* 2017, vol. 12, 19
- GARIBOTTO, V. ; HERHOLZ, K. ; BOCCARDI, M. ; PICCO, A. ; VARRONE, A. ; NORDBERG, A. ; NOBILI, F. ; RATIB, O. *Neurobiology of aging,* 2017, vol. 52, 183-195
- CHIOTIS, K. ; SAINT-AUBERT, L. ; BOCCARDI, M. ; GIETL, A. ; PICCO, A. ; VARRONE, A. ; GARIBOTTO, V. ; HERHOLZ, K. ; NOBILI, F. ; NORDBERG, A. *Neurobiology of aging,* 2017, vol. 52, 214-227
- ANDREASEN, N. ; SJOGREN, M. ; BLENNOW, K. *The world journal of biological psychiatry : the official journal of the World Federation of Societies of Biological Psychiatry,* 2003, vol. 4, 147-155
- HU, W. T. ; WATTS, K. D. ; SHAW, L. M. ; HOWELL, J. C. ; TROJANOWSKI, J. Q. ; BASRA, S. ; GLASS, J. D. ; LAH, J. J. ; LEVEY, A. I. *Annals of clinical and translational neurology,* 2015, vol. 2, 131-139
- LEWCZUK, P. ; LELENTAL, N. ; SPITZER, P. ; MALER, J. M. ; KORNHUBER, J. *Journal of Alzheimer's disease : JAD,* 2015, vol. 43, 183-191
- ELSON, E. L. *Traffic,* 2001, vol. 2, 789-796
- VUKOJEVIC, V. ; PRAMANIK, A. ; YAKOVLEVA, T. ; RIGLER, R. ; TERENIUS, L. ; BAKALKIN, G. *Cellular and molecular life sciences : CMLS,* 2005, vol. 62, 535-550
- RIGLER, R. *Biochemical and biophysical research communications,* 2010, vol. 396, 170-175
- ELSON, E. L. *Biophysical journal,* 2011, vol. 101, 2855-2870
- ELSON, E. L. *Methods in enzymology,* 2013, vol. 518, 11-41
- RIGLER, R. ; WIDENGREN, J. *European biophysics journal: EBJ,* 2017
- ELSON, E. L. *Methods,* 2017
- VUKOJEVIC, V. ; HEIDKAMP, M. ; MING, Y. ; JOHANSSON, B. ; TERENIUS, L. ; RIGLER, R. *Proceedings of the National Academy of Sciences of the United States of America,* 2008, vol. 105, 18176-18181
- CHAUHAN, A. ; PIRTTILA, T. ; CHAUHAN, V. P. ; MEHTA, P. ; WISNIEWSKI, H. M. *Journal of the neurological sciences,* 1998, vol. 154, 159-163
- GRIFFIN, M. D. ; WILSON, L. M. ; MOK, Y. F. ; JANUSZEWSKI, A. S. ; WILSON, A. M. ; KARSCHIMKUS, C. S. ; ROMAS, E. ; LEE, A. B. ; GODFREY, T. ; WONG, M. *Clinical biochemistry,* 2010, vol. 43, 278-286
- TAPIOLA, T. ; ALAFUZOFF, I. ; HERUKKA, S. K. ; PARKKINEN, L. ; HARTIKAINEN, P. ; SOININEN, H. ; PIRTTILA, T. *Archives of neurology,* 2009, vol. 66, 382-389
- SOLE-DOMENECH, S. ; SJOVALL, P. ; VUKOJEVIC, V. ; FERNANDO, R. ; CODITA, A. ; SALVE, S. ; BOGDANOVIC, N. ; MOHAMMED, A. H. ; HAMMARSTROM, P. ; NILSSON, K. P. *Acta neuropathologica,* 2013, vol. 125, 145-157
- HORROCKS, M. H. ; LEE, S. F. ; GANDHI, S. ; MAGDALINOU, N. K. ; CHEN, S. W. ; DEVINE, M. J. ; TOSATTO, L. ; KJAERGAARD, M. ; BECKWITH, J. S. ; ZETTERBERG, H. *ACS chemical neuroscience,* 2016, vol. 7, 399-406
- GROENNING, M. *Journal of chemical biology,* 2010, vol. 3, 1-18
- MATHIS, C. A. ; MASON, N. S. ; LOPRESTI, B. J. ; KLUNK, W. E. *Seminars in nuclear medicine,* 2012, vol. 42, 423-432
- SVEDBERG, M. M. ; RAHMAN, O. ; HALL, H. *Nuclear medicine and biology,* 2012, vol. 39, 484-501

- Amyloid Imaging PET Ligands as Biomarkers for Alzheimer's Disease, Preclinical Evaluation. **SVEDBERG, M. M. ; HELLSTRÖM-LINDAHL, E. ; RAHMAN, O. ; HALL, H.** Positron Emission Tomography - Current Clinical and Research Aspects. InTech, 2012
- **COHEN, S. I. ; LINSE, S. ; LUHESHI, L. M. ; HELLSTRAND, E. ; WHITE, D. A. ; RAJAH, L. ; OTZEN, D. E. ; VENDRUSCOLO, M. ; DOBSON, C. M. ; KNOWLES, T. P.** *Proceedings of the National Academy of Sciences of the United States of America,* 2013, vol. 110, 9758-9763
- **LINDGREN, M. ; HAMMARSTROM, P.** *The FEBS journal,* 2010, vol. 277, 1380-1388
- **CHANG, L. ; RISSIN, D. M. ; FOURNIER, D. R. ; PIECH, T. ; PATEL, P. P. ; WILSON, D. H. ; DUFFY, D. C.** *Journal of immunological methods,* 2012, vol. 378, 102-115
- **RISSIN, D. M. ; KAN, C. W. ; CAMPBELL, T. G. ; HOWES, S. C. ; FOURNIER, D. R. ; SONG, L. ; PIECH, T. ; PATEL, P. P. ; CHANG, L. ; RIVNAK, A. J.** *Nature biotechnology,* 2010, vol. 28, 595-599
- **HOLTTA, M. ; HANSSON, O. ; ANDREASSON, U. ; HERTZE, J. ; MINTHON, L. ; NAGGA, K. ; ANDREASEN, N. ; ZETTERBERG, H. ; BLENNOW, K.** *PloS one,* 2013, vol. 8, e66381
- **ANDREASSON, U. ; BLENNOW, K. ; ZETTERBERG, H.** *Alzheimer's & dementia,* 2016, vol. 3, 98-102
- **NAKAMURA, A. ; KANEKO, N. ; VILLEMAGNE, V. L. ; KATO, T. ; DOECKE, J. ; DORE, V. ; FOWLER, C. ; LI, Q. X. ; MARTINS, R. ; ROWE, C.** *Nature,* 2018, vol. 554, 249-254
- **REVERBERI, R. ; REVERBERI, L.** *Blood transfusion = Trasfusione del sangue,* 2007, vol. 5, 227-240
- Interferences in Immunoassays. **SCHIETTECATTE, J. ; ANCKAERT, E. ; SMITZ, J.** Advances in Immunoassay Technology. InTech, 2012
- Detection Curb. **SAIKI, H.** Trends in Immunolabelled and Related Techniques. InTech, 2012
- **KLAVER, A. C. ; PATRIAS, L. M. ; FINKE, J. M. ; LOEFFLER, D. A.** *Journal of neuroscience methods,* 2011, vol. 195, 249-254
- **HUNTER, S. ; BRAYNE, C.** *Journal of negative results in biomedicine,* 2017, vol. 16, 1
- **LUNDSTROM, S. L. ; ZHANG, B. ; RUTISHAUSER, D. ; AARSLAND, D. ; ZUBAREV, R. A.** *Scientific reports,* 2017, vol. 7, 41929
- **HIRSCH, J. ; HANSEN, K. C. ; BURLINGAME, A. L. ; MATTHAY, M. A.** *American journal of physiology. Lung cellular and molecular physiology,* 2004, vol. 287, L1-23
- **PORTELIUS, E. ; BRINKMALM, G. ; PANNEE, J. ; ZETTERBERG, H. ; BLENNOW, K. ; DAHLEN, R. ; BRINKMALM, A. ; GOBOM, J.** *Expert review of proteomics,* 2017, vol. 14, 1007-1020
- **LIN, Y. T. ; CHENG, J. T. ; YAO, Y. C. ; JUO, LO, Y. K. ; LIN, C. H. ; GER, L. P. ; LU, P. J.** *Journal of Alzheimer's disease : JAD,* 2009, vol. 18, 907-918
- **JANELIDZE, S. ; STOMRUD, E. ; PALMQVIST, S. ; ZETTERBERG, H. ; VAN WESTEN, D. ; JEROMIN, A. ; SONG, L. ; HANLON, D. ; TAN HEHIR, C. A. ; BAKER, D.** *Scientific reports,* 2016, vol. 6, 26801
- **NITSCH, R. M. ; REBECK, G. W. ; DENG, M. ; RICHARDSON, U. I. ; TENNIS, M. ; SCHENK, D. B. ; VIGO-PELFREY, C. ; LIEBERBURG, I. ; WURTMAN, R. J. ; HYMAN, B. T. et al.** *Annals of neurology,* 1995, vol. 37, 512-518